# EUROPEAN PATENT SPECIFICATION

(11) **EP 3 930 676 B1**
(45) Date of publication and mention of the grant of the patent: **03.08.2022**
(21) Application number: 20715240.6
(22) Date of filing: 01.03.2020
(51) Int. Cl.: A61K 8/60, A61Q 19/08

(54) **POLYRIBONUCLEOTIDES AND COSMETIC USES THEREOF**
POLYRIBONUKLEOTIDE UND KOSMETISCHE VERWENDUNGEN DAVON
POLYRIBONUCLÉOTIDES ET LEURS UTILISATIONS COSMÉTIQUES

(30) Priority: 01.03.2019 US 201962812772 P
(43) Date of publication of application: 05.01.2022
(73) Proprietor: Flagship Pioneering Innovations VI, LLC, Cambridge, MA 02142 (US)
(72) Inventor: KAHVEJIAN, Avak, Cambridge, Massachusetts 02142 (US); PLUGIS, Nicholas McCartney, Cambridge, Massachusetts 02142 (US); DE BOER, Alexandra Sophie, Cambridge, Massachusetts 02142 (US); CARMONA, Ellese Marie, Cambridge, Massachusetts 02142 (US); STEWART, Morag Helen, Cambridge, Massachusetts 02142 (US)
(74) Representative: J A Kemp LLP
(86) International application number: PCT/US2020/020561
(87) International publication number: WO 2020/180752

(56) References cited:
- EP-A2- 1 598 421
- EP-A2- 1 723 958
- WO-A1-2013/053481
- DE-A1-102005 001 784
- US-A- 5 877 160
- US-A1- 2014 134 636
- US-A1- 2017 042 782
- US-A1- 2018 037 894

## Description

### BACKGROUND

Polyribonucleotides are critical biomolecules for biological activities such as gene expression, gene regulation, and cellular signaling transduction. In the field of cosmetics a range of polyribonucleotides have been disclosed for obtaining a variety of beneficial effects, in particular on skin. These polyribonucleotides are of linear nature. See for example US 2014/134636 A1, WO 2013/053481 A1, EP 1 598 421 A2, EP 1 723 958 A2, US 5 877 160 A, DE 10 2005 001784 A1, US 2018/037894 A1 and US 2017/042782 A1.

### SUMMARY

The present invention described herein includes compositions, cosmetic compositions, and methods for delivery of circular polyribonucleotides (e.g., cosmetic circular polyribonucleotides). In some embodiments, the compositions and cosmetic compositions include ethanol and the circular polyribonucleotides. In other embodiments, the compositions and cosmetic compositions include alcohol and the circular polyribonucleotides. In other embodiments, the compositions and cosmetic compositions include a cell-penetrating agent and the circular polyribonucleotides. The methods comprise applying these compositions and cosmetic compositions to a surface area (e.g., an integumentary member) of a subject.

The present invention described herein includes cosmetic compositions free of any a carrier comprising a cosmetic circular polyribonucleotide and diluent. The cosmetic compositions can be applied to epithelial cells for delivery of the cosmetic circular polyribonucleotide. The cosmetic compositions can be applied to a surface area (e.g., an integumentary member) after application of a sterilizing agent to that area.

In some aspects, a cosmetic composition comprises a mixture of a circular polyribonucleotide and ethanol, wherein the ethanol constitutes 0.3% v/v to 75% v/v of the mixture. In some embodiments, the ethanol constitutes 0.3% v/v to 70% v/v, 0.3% v/v to 60% v/v, 0.3% v/v to 50% v/v, 0.3% v/v to 40% v/v, 30% v/v to 20% v/v, 0.3% v/v to 15% v/v, 0.3% v/v to 10% v/v, 0.3% v/v to 5% v/v, 0.3% v/v to 1% v/v, or 0.3% v/v to 0.5% v/v of the mixture. In some embodiments, the ethanol constitutes 0.5% v/v to 75% v/v, 1% v/v to 75% v/v, 5% v/v to 75% v/v, 10% v/v to 75% v/v, 15% v/v to 75% v/v, 20% v/v to 75% v/v, 30% v/v to 75% v/v, 40% v/v to 75% v/v, 50% v/v to 75% v/v, 60% v/v to 75% v/v, or 70% v/v to 75% v/v of the mixture.

In some aspects, a cosmetic composition comprises a mixture of a circular polyribonucleotide and an alcohol, wherein the alcohol constitutes 0.3% v/v to 75% v/v of the mixture. In some embodiments, the alcohol constitutes 0.3% v/v to 70% v/v, 0.3% v/v to 60% v/v, 0.3% v/v to 50% v/v, 0.3% v/v to 40% v/v, 30% v/v to 20% v/v, 0.3% v/v to 15% v/v, 0.3% v/v to 10% v/v, 0.3% v/v to 5% v/v, 0.3% v/v to 1% v/v, or 0.3% v/v to 0.5% v/v of the mixture. In some embodiments, the alcohol constitutes 0.5% v/v to 75% v/v, 1% v/v to 75% v/v, 5% v/v to 75% v/v, 10% v/v to 75% v/v, 15% v/v to 75% v/v, 20% v/v to 75% v/v, 30% v/v to 75% v/v, 40% v/v to 75% v/v, 50% v/v to 75% v/v, 60% v/v to 75% v/v, or 70% v/v to 75% v/v of the mixture. In some embodiments, the alcohol is selected from the group consisting of: methanol, ethanol, isopropanol, butanol, pentanol, cetyl alcohol, ethylene glycol, propylene glycol, denatured alcohol, benzyl alcohol, specially denatured alcohol, glycol, stearyl alcohol, cetearyl alcohol, menthol, polyethylene glycols (PEG)-400, isopropyl alcohol, dimethylaminoethanol, retinol, d-alpha-tocopherol, SD alcohol, SD alcohol 40, c12-16, lauryl alcohol, myristyl alcohol, butylene glycol, and propanediol.

In some aspects, a cosmetic composition comprises a mixture of a circular polyribonucleotide and a cell-penetrating agent, wherein the cell-penetrating agent constitutes 0.3% v/v to 75% v/v of the mixture. In some embodiments, the cell-penetrating agent constitutes 0.3% v/v to 70% v/v, 0.3% v/v to 60% v/v, 0.3% v/v to 50% v/v, 0.3% v/v to 40% v/v, 30% v/v to 20% v/v, 0.3% v/v to 15% v/v, 0.3% v/v to 10% v/v, 0.3% v/v to 5% v/v, 0.3% v/v to 1% v/v, or 0.3% v/v to 0.5% v/v of the mixture. In some embodiments, the cell-penetrating agent constitutes 0.5% v/v to 75% v/v, 1% v/v to 75% v/v, 5% v/v to 75% v/v, 10% v/v to 75% v/v, 15% v/v to 75% v/v, 20% v/v to 75% v/v, 30% v/v to 75% v/v, 40% v/v to 75% v/v, 50% v/v to 75% v/v, 60% v/v to 75% v/v, or 70% v/v to 75% v/v of the mixture. In some embodiments, the cell-penetrating agent is an alcohol. In some embodiments, the alcohol is selected from the group consisting of: methanol, ethanol, isopropanol, butanol, pentanol, cetyl alcohol, ethylene glycol, propylene glycol, denatured alcohol, benzyl alcohol, specially denatured alcohol, glycol, stearyl alcohol, cetearyl alcohol, menthol, polyethylene glycols (PEG)-400, isopropyl alcohol, dimethylaminoethanol, retinol, d-alpha-tocopherol, SD alcohol, SD alcohol 40, c12-16, lauryl alcohol, myristyl alcohol, butylene glycol, and propanediol. The circular polyribonucleotide may encode a protein. The protein may be a cosmetic protein. The protein may be a hair or eyelash (e.g., keratin fiber), nail, or skin protein. In some embodiments, the protein is collagen, keratin, elastin, botulinum toxin, or a fragment thereof. In some embodiments, the cosmetic composition is a liquid, gel, lotion, paste, cream, foam, serum, ointment, or stick. In some embodiments, the circular polyribonucleotide is a cosmetic circular polyribonucleotide. The polyribonucleotide may be an mRNA. In some embodiments, the polyribonucleotide lacks a cap or poly-A tail. The polyribonucleotide is a circular polyribonucleotide. In some embodiments, the polyribonucleotide comprises a modified ribonucleotide. In some embodiments, the cosmetic composition has a pH of about 7. In some embodiments, the cosmetic composition has a viscosity that is about the same as water. In some embodiments, the cosmetic composition is substantially free of hydrophobic or lipophilic groups. In some embodiments, the cosmetic composition is substantially free of hydrocarbons. In some embodiments, the cosmetic composition is substantially free of cationic liposomes. In some embodiments, the cosmetic composition is substantially free of fatty acids, lipids, liposomes, cholesterol, or any combination thereof. In some embodiments, the cosmetic composition is substantially free form glycerin, propylene glycol, or a combination thereof. In some embodiments, the cosmetic composition is an anti-wrinkle composition. In some embodiments, the cell penetrating agent is soluble in polar solvents. In some embodiments, the cell penetrating agent is insoluble in polar solvents.

The cosmetic composition may comprise a circular polyribonucleotide and an alcohol, wherein the alcohol is configured for topical administration. The cosmetic composition may comprise a circular polyribonucleotide and a cell-penetrating agent, wherein the cell-penetrating agent is configured for topical administration.

The cosmetic composition may comprise a circular polyribonucleotide and an alcohol, wherein the circular polyribonucleotide comprises a payload or a sequence encoding a payload and wherein the payload has a cosmetic effect on a cell or a tissue. The cosmetic composition may comprise a circular polyribonucleotide and a cell-penetrating agent, wherein the circular polyribonucleotide comprises a payload or a sequence encoding a payload and wherein the payload has a cosmetic effect on a cell or a tissue. In some aspects, the payload is a cosmetic protein or peptide selected from the group consisting of collagen, keratin, elastin, and botulinum toxin, or any a fragment thereof.

The cosmetic composition may comprise a circular polyribonucleotide and an alcohol, wherein the circular polyribonucleotide is in an amount effective to have a cosmetic effect on a cell or tissue and wherein the alcohol is in an amount effective to have a cosmetic effect on a cell or tissue. The cosmetic composition may comprise a circular polyribonucleotide and a cell-penetrating agent, wherein the circular polyribonucleotide is in an amount effective to have a cosmetic effect on a cell or tissue and wherein the cell-penetrating agent is in an amount effective to have a cosmetic effect on a cell or tissue.

The cosmetic composition may comprise a circular polyribonucleotide, an alcohol, and a topical delivery excipient, wherein the topical delivery excipient comprises a stabilizer. The cosmetic composition may comprise a circular polyribonucleotide, a cell-penetrating agent, and a topical delivery excipient, wherein the topical delivery excipient comprises a stabilizer. The stabilizer may comprise glucose (4.5g/L).

The cosmetic composition may comprise a biodegradable scaffold loaded with circular polyribonucleotide and an alcohol. The cosmetic composition may comprise a biodegradable scaffold loaded with circular polyribonucleotide and a cell-penetrating agent. In some embodiments, the cell-penetrating agent comprises an alcohol. In some embodiments, the alcohol is selected from the group consisting of: methanol, ethanol, isopropanol, butanol, pentanol, cetyl alcohol, ethylene glycol, propylene glycol, denatured alcohol, benzyl alcohol, specially denatured alcohol, glycol, stearyl alcohol, cetearyl alcohol, menthol, polyethylene glycols (PEG)-400, isopropyl alcohol, dimethylaminoethanol, retinol, d-alpha-tocopherol, SD alcohol, SD alcohol 40, c12-16, lauryl alcohol, myristyl alcohol, butylene glycol, and propanediol. In some embodiments, the alcohol is ethanol.

In some aspects, a method of delivering a cosmetic polyribonucleotide to a subject comprises: a) applying a sterilizing agent to an integumentary member of the subject; b) applying a cosmetic composition free of any carrier comprising the cosmetic polyribonucleotide and diluent to the integumentary member, wherein the cosmetic polyribonucleotide is a circular polyribonucleotide. In some embodiments, the sterilizing agent is an alcohol, UV light, laser light, or heat.

In some aspects, a method of delivering a cosmetic polyribonucleotide to a subject comprises a) applying an alcohol to an integumentary member of the subject; b) applying a cosmetic composition free of any carrier comprising the cosmetic polyribonucleotide and diluent to the integumentary member, wherein the cosmetic polyribonucleotide is a circular polyribonucleotide.

In some aspects, a method of delivering a polyribonucleotide to an epithelial cell comprises applying a cosmetic composition free of any carrier comprising a diluent and a cosmetic polyribonucleotide that is not modified to the epithelial cell, wherein the cosmetic polyribonucleotide is a circular polyribonucleotide.

In some aspects, a method of delivering a polyribonucleotide to a subject comprises applying a cosmetic composition comprising a mixture of a cosmetic polyribonucleotide and ethanol to an integumentary member of the subject, wherein the ethanol constitutes 0.3% v/v to 75% v/v of the mixture, and wherein the cosmetic polyribonucleotide is a circular polyribonucleotide. In aspects, a method of delivering a circular polyribonucleotide to a subject comprises applying a cosmetic composition comprising a mixture of a cosmetic polyribonucleotide and an alcohol to an integumentary member of the subject, wherein the alcohol constitutes 0.3% v/v to 75% v/v of the mixture. In some aspects, a method of delivering a circular polyribonucleotide to a subject comprises applying a cosmetic composition comprising a mixture of a cosmetic circular polyribonucleotide and a cell-penetrating agent to an integumentary member of the subject, wherein the cell-penetrating agent constitutes 0.3% v/v to 75% v/v of the mixture. In some embodiments, the cosmetic composition delivers the cosmetic circular polyribonucleotide to a dermal or epidermal tissue of the subject. In some embodiments, the cosmetic composition delivers the cosmetic circular polyribonucleotide to the dermal or epidermal tissue of the subject without iontophoresis.

A method of delivering a cosmetic circular polyribonucleotide to a cell or tissue comprising contacting the cell or tissue to a mixture comprising the cosmetic circular polyribonucleotide and an alcohol, wherein the alcohol constitutes 0.3% v/v to 75% v/v of the mixture is disclosed, along with a method of delivering a cosmetic circular polyribonucleotide to a cell or tissue comprising contacting the cell or tissue to a mixture comprising the cosmetic polyribonucleotide and a cell-penetrating agent, wherein the cell-penetrating agent constitutes 0.3% v/v to 75% v/v of the mixture.

A method of delivering a cosmetic composition to a cell or tissue comprising contacting the cell or tissue to the cosmetic composition comprising a cosmetic circular polyribonucleotide and an alcohol, wherein the alcohol is configured for topical administration is disclosed, along with a method of delivering a cosmetic composition to a cell or tissue comprising contacting the cell or tissue to the cosmetic composition comprising a cosmetic circular polyribonucleotide and a cell-penetrating agent, wherein the cell-penetrating agent is configured for topical administration.

In some aspects, a method of delivery of a cosmetic circular polyribonucleotide comprises applying a mixture comprising the cosmetic circular polyribonucleotide and an alcohol onto an integumentary member of a subject. In some aspects, a method of delivery of a cosmetic circular polyribonucleotide comprises applying a mixture comprising the cosmetic polyribonucleotide and a cell-penetrating agent onto an integumentary member of a subject. In some embodiments, the cell-penetrating agent comprises an alcohol. In some embodiments, the alcohol is selected from the group consisting of: methanol, ethanol, isopropanol, butanol, pentanol, cetyl alcohol, ethylene glycol, propylene glycol, denatured alcohol, benzyl alcohol, specially denatured alcohol, glycol, stearyl alcohol, cetearyl alcohol, menthol, polyethylene glycols (PEG)-400, isopropyl alcohol, dimethylaminoethanol, retinol, d-alpha-tocopherol, SD alcohol, SD alcohol 40, c12-16, lauryl alcohol, myristyl alcohol, butylene glycol, and propanediol. In some embodiments, the alcohol comprises ethanol. In some embodiments, the ethanol, alcohol, or cell-penetrating agent constitutes 0.3% v/v to 70% v/v, 0.3% v/v to 60% v/v, 0.3% v/v to 50% v/v, 0.3% v/v to 40% v/v, 30% v/v to 20% v/v, 0.3% v/v to 15% v/v, 0.3% v/v to 10% v/v, 0.3% v/v to 5% v/v, 0.3% v/v to 1% v/v, or 0.3% v/v to 0.5% v/v of the mixture. In some embodiments, the ethanol, alcohol, or cell-penetrating agent 0.5% v/v to 75% v/v, 1% v/v to 75% v/v, 5% v/v to 75% v/v, 10% v/v to 75% v/v, 15% v/v to 75% v/v, 20% v/v to 75% v/v, 30% v/v to 75% v/v, 40% v/v to 75% v/v, 50% v/v to 75% v/v, 60% v/v to 75% v/v, or 70% v/v to 75% v/v of the mixture. In some embodiments, the method further comprises mixing the cosmetic circular polyribonucleotide with the alcohol. In some embodiments, the method further comprises mixing the cosmetic circular polyribonucleotide with the cell-penetrating agent. The cosmetic circular polyribonucleotide may be in a solid form before the mixing. The cosmetic circular polyribonucleotide may be lyophilized before the mixing. The cosmetic circular polyribonucleotide may be in a liquid form before the mixing. The cosmetic circular polyribonucleotide may be dissolved in a solvent before the mixing. The cosmetic circular polyribonucleotide may comprise a payload or a sequence encoding a payload and wherein the payload has a cosmetic effect on a cell or a tissue. In some aspects, the payload is a cosmetic protein or peptide selected from the group consisting of collagen, keratin, elastin, and botulinum toxin, or any a fragment thereof. The cosmetic circular polyribonucleotide may be in an amount effective to have a cosmetic effect on a cell and the cell-penetrating agent is in an amount effective to have a cosmetic effect on a cell or a tissue. The cosmetic circular polyribonucleotide may encode a protein. In some embodiments, the protein is a cosmetic protein. The protein may be a hair, skin, or nail protein. In some embodiments, the protein is collagen, keratin, elastin, botulinum toxin, or a fragment thereof. In some embodiments, the composition is a liquid, serum, gel, lotion, paste, cream, foam, or stick. The cosmetic circular polyribonucleotide may be an mRNA. In some embodiments, the cosmetic circular polyribonucleotide lacks a cap or poly-A tail. The cosmetic circular polyribonucleotide may be immunogenic. The cosmetic circular polyribonucleotide may be non-immunogenic. The cosmetic circular polyribonucleotide is a circular polyribonucleotide. In some embodiments, the cosmetic polyribonucleotide comprises a modified ribonucleotide. In some embodiments, the cosmetic composition has a pH of about 7. In some embodiments, the cosmetic composition has a viscosity that is about the same as water. In some embodiments, the cosmetic composition is substantially free of hydrophobic or lipophilic groups. In some embodiments, the cosmetic composition is substantially free of hydrocarbons. In some embodiments, the cosmetic composition is substantially free of cationic liposomes. In some embodiments, the cosmetic composition is substantially free of fatty acids, lipids, liposomes, cholesterol, or any combination thereof. In some embodiments, the cosmetic composition is free from glycerin, propylene glycol, or a combination thereof. In some embodiments, the cell penetrating agent is soluble in polar solvents. In some embodiments, the cell penetrating agent is insoluble in polar solvents. The cosmetic composition may further comprise a cosmetically acceptable excipient. The delivery may be localized. In some embodiments, the integumentary member comprises skin, hair, or nails. Applying may comprise depositing a drop of the mixture directly onto the integumentary member. Applying my comprise wiping the integumentary member with a patch, a gel, or a film embedded with the mixture. In some embodiments, applying comprises spraying the mixture onto the integumentary member. Applying may comprise administering the mixture to the subject via aerosolization. The cell may comprise an epithelial cell. The circular polyribonucleotide may have a translation efficiency at least 5%, at least 10%, at least 15%, at least 20%, at least 30%, at least 40%, at least 50%, at least 60%, at least 70%, at least 80%, at least 90%, at least 100%, at least 150%, at least 2 fold, at least 3 fold, at least 4 fold, at least 5 fold, at least 6 fold, at least 7 fold, at least 8 fold, at least 9 fold, at least 10 fold, at least 20 fold, at least 50 fold, or at least 100 fold greater than a linear counterpart. The circular polyribonucleotide may have a translation efficiency at least 5 fold greater than a linear counterpart. The cosmetic circular polyribonucleotide may have a short term cosmetic effect. The cosmetic circular polyribonucleotide may have a long term cosmetic effect. A concentration of the cosmetic circular polyribonucleotide in the mixture may be at least about 50 ng/mL, at least about 100 ng/mL, at least about 500 ng/mL, at least about 1 µg/mL, at least about 2 µg/mL, at least about 3 µg/mL, at least about 4 µg/mL, at least about 5 µg/mL, at least about 10 µg/mL, at least about 20 µg/mL, at least about 50 µg/mL, at least about 100 µg/mL, at least about 200 µg/mL, at least about 500 µg/mL, at least about 1 mg/mL, at least about 2 mg/mL, at least about 5 mg/mL, at least about 10 mg/mL, at least about 20 mg/mL, at least about 50 mg/mL, or at least about 100 mg/mL.

In some aspects, a kit comprises an application tool and the cosmetic composition of any one of the preceding embodiments, wherein the application tool is configured to apply the cosmetic composition to an integumentary member of a subject.

In some aspects, a kit comprises a first application tool, a second application tool, a sterilizing agent, and a cosmetic composition free of any carrier comprising the cosmetic polyribonucleotide and diluent, wherein the first application tool is configured to apply a sterilizing agent to an integumentary member of a subject and the second application tool is configured to apply the composition to the integumentary member of the subject, and wherein the cosmetic polyribonucleotide is a circular polyribonucleotide. In some embodiments, the sterilizing agent is an alcohol, UV light, laser light, or heat. In some embodiments, the alcohol is selected from the group consisting of: methanol, ethanol, isopropanol, butanol, pentanol, cetyl alcohol, ethylene glycol, propylene glycol, denatured alcohol, benzyl alcohol, specially denatured alcohol, glycol, stearyl alcohol, cetearyl alcohol, menthol, polyethylene glycols (PEG)-400, isopropyl alcohol, dimethylaminoethanol, retinol, d-alpha-tocopherol, SD alcohol, SD alcohol 40, c12-16, lauryl alcohol, myristyl alcohol, butylene glycol, and propanediol. In some embodiments, the first application tool is a wipe. In some embodiments, the wipe comprises the sterilizing agent. In some embodiments, first application tool is a device that applies UV light or laser light. In some embodiments, the first application tool is a device that applies heat.

In some aspects, a kit comprises an application tool and a mixture comprising a circular polyribonucleotide and a cell-penetrating agent or alcohol, wherein the application tool is configured to apply the mixture to an integumentary member of a subject. In some embodiments, the application tool or second application tool comprises a pipette. In some embodiments, the application tool or second application tool comprises a substrate, and wherein the substrate is embedded with the mixture. In some embodiments, the substrate is made of natural or artificial fibers. In some embodiments, the application tool or second application tool comprises a patch. In some embodiments, the application tool or second application tool comprises a sprayer. In some embodiments, the application tool or second application tool comprises a peel pad. In some embodiments, the application tool or second application tool is configured to release the mixture in a controlled manner. In some embodiments, the integumentary member comprises skin, nails, eyelashes, or hair.

In another aspect, a kit comprises a cosmetic composition described herein and an alcohol wipe. The kit may comprise a cosmetic composition described herein and a vial containing an alcohol for application to the surface area of a subject.

### DEFINITIONS

The present invention will be described with respect to particular embodiments and with reference to certain figures but the invention is not limited thereto but only by the claims. Terms as set forth hereinafter are generally to be understood in their common sense unless indicated otherwise.

The term "polynucleotide" as used herein means a molecule comprising one or more nucleic acid subunits, or nucleotides, and can be used interchangeably with "nucleic acid" or "oligonucleotide". A polynucleotide can include one or more nucleotides selected from adenosine (A), cytosine (C), guanine (G), thymine (T) and uracil (U), or variants thereof. A nucleotide can include a nucleoside and at least 1, 2, 3, 4, 5, 6, 7, 8, 9, 10, or more phosphate (PO₃) groups. A nucleotide can include a nucleobase, a five-carbon sugar (either ribose or deoxyribose), and one or more phosphate groups. Ribonucleotides are nucleotides in which the sugar is ribose. Polyribonucleotides or ribonucleic acids, or RNA, can refer to macromolecules that include multiple ribonucleotides that are polymerized via phosphodiester bonds. Deoxyribonucleotides are nucleotides in which the sugar is deoxyribose. Polydeoxyribonucleotides or deoxyribonucleic acids, or DNA, can refer to macromolecules that include multiple deoxyribonucleotides that are polymerized via phosphodiester bonds. A nucleotide can be a nucleoside monophosphate or a nucleoside polyphosphate. A nucleotide can be a deoxyribonucleoside polyphosphate, such as, e.g., a deoxyribonucleoside triphosphate (dNTP), which can be selected from deoxyadenosine triphosphate (dATP), deoxycytidine triphosphate (dCTP), deoxyguanosine triphosphate (dGTP), uridine triphosphate (dUTP) and deoxythymidine triphosphate (dTTP) dNTPs, that include detectable tags, such as luminescent tags or markers (e.g., fluorophores). A nucleotide can include any subunit that can be incorporated into a growing nucleic acid strand. Such subunit can be an A, C, G, T, or U, or any other subunit that is specific to one or more complementary A, C, G, T or U, or complementary to a purine (i.e., A or G, or variant thereof) or a pyrimidine (i.e., C, T or U, or variant thereof). In some examples, a polynucleotide is deoxyribonucleic acid (DNA), ribonucleic acid (RNA), or derivatives or variants thereof. In some cases, a polynucleotide is a short interfering RNA (siRNA), a microRNA (miRNA), a plasmid DNA (pDNA), a short hairpin RNA (shRNA), small nuclear RNA (snRNA), messenger RNA (mRNA), precursor mRNA (pre-mRNA), antisense RNA (asRNA), to name a few, and encompasses both the nucleotide sequence and any structural embodiments thereof, such as single-stranded, double-stranded, triple-stranded, helical, hairpin, etc. In some cases, a polynucleotide molecule is circular. A polynucleotide can have various lengths. A nucleic acid molecule can have a length of at least about 10 bases, 20 bases, 30 bases, 40 bases, 50 bases, 100 bases, 200 bases, 300 bases, 400 bases, 500 bases, 1 kilobase (kb), 2 kb, 3, kb, 4 kb, 5 kb, 10 kb, 50 kb, or more. A polynucleotide can be isolated from a cell or a tissue. As embodied herein, the polynucleotide sequences may include isolated and purified DNA/RNA molecules, synthetic DNA/RNA molecules, and synthetic DNA/RNA analogs.

Polynucleotides, e.g., polyribonucleotides or polydeoxyribonucleotides, may include one or more nucleotide variants, including nonstandard nucleotide(s), non-natural nucleotide(s), nucleotide analog(s) and/or modified nucleotides. Examples of modified nucleotides include, but are not limited to diaminopurine, 5-fluorouracil, 5-bromouracil, 5-chlorouracil, 5-iodouracil, hypoxanthine, xantine, 4-acetylcytosine, 5-(carboxyhydroxylmethyl)uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D- mannosylqueosine, 5'-methoxycarboxymethyluracil, 5-methoxyuracil, 2-methylthio-D46- isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5- oxyacetic acid methylester, uracil-5-oxyacetic acid(v), 5-methyl-2-thiouracil, 3-(3-amino- 3-N-2-carboxypropyl) uracil, (acp3)w, 2,6-diaminopurine and the like. In some cases, nucleotides may include modifications in their phosphate moieties, including modifications to a triphosphate moiety. Non-limiting examples of such modifications include phosphate chains of greater length (e.g., a phosphate chain having, 4, 5, 6, 7, 8, 9, 10 or more phosphate moieties) and modifications with thiol moieties (e.g., alpha-thiotriphosphate and beta-thiotriphosphates). Nucleic acid molecules may also be modified at the base moiety (e.g., at one or more atoms that typically are available to form a hydrogen bond with a complementary nucleotide and/or at one or more atoms that are not typically capable of forming a hydrogen bond with a complementary nucleotide), sugar moiety or phosphate backbone. Nucleic acid molecules may also contain amine -modified groups, such as amino ally 1-dUTP (aa-dUTP) and aminohexhylacrylamide-dCTP (aha-dCTP) to allow covalent attachment of amine reactive moieties, such as N-hydroxysuccinimide esters (NHS). Alternatives to standard DNA base pairs or RNA base pairs in the oligonucleotides of the present disclosure can provide higher density in bits per cubic mm, higher safety (resistant to accidental or purposeful synthesis of natural toxins), easier discrimination in photo-programmed polymerases, or lower secondary structure. Such alternative base pairs compatible with natural and mutant polymerases for de novo and/or amplification synthesis are described in Betz K, Malyshev DA, Lavergne T, Welte W, Diederichs K, Dwyer TJ, Ordoukhanian P, Romesberg FE, Marx A. Nat. Chem. Biol. 2012 Jul;8(7):612-4.

A polyribonucleotide can be present in circular form, or for reference linear form. As used herein, the terms "linear RNA" or "linear polyribonucleotide' are used interchangeably, and mean a polyribonucleotide having free 5' and 3' ends. Linear RNA may have a free 5' end or 3' end. As used herein, the terms "circRNA" or "circular polyribonucleotide" or "circular RNA" are used interchangeably and mean a polyribonucleotide that forms a circular structure through covalent or non-covalent bonds. In some cases, circular polyribonucleotide has a continuous loop in which typical free 5' and 3' ends of a corresponding linear polyribonucleotide are joined together via either covalent or non-covalent bond, or via a non-nucleic acid linker (e.g., a non-nucleic acid polymer or a protein). In some cases, circular polyribonucleotide provided herein can be formed by two or more linear polyribonucleotides, which are joined together to form a continuous loop structure, via covalent or non-covalent bonds. While not being bound by theory, it is possible that multiple segments of an RNA can be produced from a DNA and their 5' and 3' free ends annealed to produce a "string" of RNA, which ultimately can be circularized when only one 5' and one 3' free end remains.

As used herein, the term "cosmetic polypeptides of interest" means any polypeptide which is selected to be encoded in the polyribonucleotide of the cosmetic composition of the present disclosure. As used herein, "polypeptide" means a polymer of amino acid residues (natural or unnatural) linked together most often by peptide bonds. The term, as used herein, means proteins, polypeptides, and peptides of any size, structure, or function. Polypeptides can include gene products, naturally occurring polypeptides, synthetic polypeptides, homologs, orthologs, paralogs, fragments and other equivalents, variants, and analogs of the foregoing. A polypeptide can be a single molecule or may be a multi- molecular complex such as a dimer, trimer or tetramer. They can also comprise single chain or multichain polypeptides such as antibodies or insulin and can be associated or linked. Most commonly disulfide linkages are found in multichain polypeptides. The term polypeptide can also apply to amino acid polymers in which one or more amino acid residues are an artificial chemical analogue of a corresponding naturally occurring amino acid.

As used herein, the term "mixture" means a material made of two or more different substances that are mixed. In some cases, a mixture described herein can be a homogenous mixture of the two or more different substances, e.g., the mixture can have the same proportions of its components (e.g., the two or more substances) throughout any given sample of the mixture. In some cases, a mixture as provided herein can be a heterogeneous mixture of the two or more different substances, e.g., the proportions of the components of the mixture (e.g., the two or more substances) can vary throughout the mixture. In some cases, a mixture is a liquid solution, e.g., the mixture is present in liquid phase. In some instances, a liquid solution can be regarded as comprising a liquid solvent and a solute. Mixing a solute in a liquid solvent can be termed as "dissolution" process. In some cases, a liquid solution is a liquid-in-liquid solution (e.g., a liquid solute dissolved in a liquid solvent), a solid-in-liquid solution (e.g., a solid solute dissolved in a liquid solvent), or a gas-in-liquid solution (e.g., a solid solute dissolved in a liquid solvent). In some cases, there is more than one solvent and/or more than one solute. In some cases, a mixture is a colloid, liquid suspension, or emulsion. In some cases, a mixture is a solid mixture, e.g., the mixture is present in solid phase.

As used herein, the term "cell-penetrating agent" means an agent that, when contacted to a cell, facilitates entry into the cell. In some cases, a cell-penetrating agent facilitates direct penetration of the cell membrane, for instance, via direct electrostatic interaction with negatively charged phospholipids of the cell membrane, or transient pore formation by inducing configurational changes in membrane proteins or the phospholipid bilayer. In some cases, a cell-penetrating agent facilitates endocytosis-mediated translocation into the cell. For example, under certain situation, the cell-penetrating agent can stimulate the cell to undergo the endocytosis process, by which the cell membrane can fold inward into the cell. In certain embodiments, a cell-penetrating agent helps form a transitory structure that transports across the cell membrane. Without wishing to be bound to a particular theory, a cell-penetrating agent as provided herein can increase the permeability of the cell membrane or increase internalization of a molecule into the cell membrane, as a result of which, delivery into the cell can be more efficient when the cell is contacted with the cell-penetrating agent simultaneously as compared to otherwise identical delivery without the cell-penetrating agent.

As used herein, the term "payload" means any molecule delivered by the polyribonucleotide as disclosed herein. In some cases, a payload is a nucleic acid, a protein, a chemical, a ribonucleoprotein, or any combination thereof. In some cases, a payload is a nucleic acid sequence directly contained within the polyribonucleotide as disclosed herein. In some cases, a payload is attached to or associated with the polyribonucleotide as disclosed herein, for instance via complementary hybridization, or via protein-nucleic acid interactions. In certain cases, the payload is a protein encoded by a nucleic acid sequence contained within, attached to, or associated with the polyribonucleotide. In some cases, the "attachment" meanscovalent bond or non-covalent interaction between two molecules. In some cases, the "association" when used in the context of the interaction between a payload and a polyribonucleotide can mean that the payload is indirectly linked to the polyribonucleotide via one or more other molecules in between. In some cases, the attachment or association can be transient. In some cases, a payload is attached to or associated with the polyribonucleotide under one condition but not under another condition, for instance, depending on the ambient pH condition or the presence or absence of a stimulus or a binding partner.

As used herein, the term "alcohol" means any organic compound in which the hydroxyl functional group (-OH) is bound to a carbon. An alcohol as discussed herein can include, but is not limited to, monohydric alcohols, polyhydric alcohols, unsaturated aliphatic alcohols, and alicyclic alcohols. In some cases, an alcohol can refer to ethanol. In some cases, an alcohol can include, but is not limited to, methanol, ethanol, isopropanol, butanol, pentanol, cetyl alcohol, ethylene glycol, propylene glycol, denatured alcohol, benzyl alcohol, specially denatured alcohol, glycol, stearyl alcohol, cetearyl alcohol, menthol, polyethylene glycols (PEG)-400, isopropyl alcohol, dimethylaminoethanol, retinol, d-alpha-tocopherol, SD alcohol, SD alcohol 40, c12-16, lauryl alcohol, myristyl alcohol, butylene glycol, and propanediol.

As used herein, the term "surface area" of a subject body, means any area of a subject that is or has a potential to be exposed to an exterior environment. A surface area of a subject body, e.g., a mammal body, e.g., a human body, includes skin, nail, hair, eyelashes, and surface areas of ear cavity and eye. In some cases, a surface area of a subject body can often refer to the outer area under which epithelial cells are lined up. Skin, for example, can be one type of surface area as discussed herein and can be composed of epidermis and dermis, the former of which forms the outermost layers of kin and can include organized assembly of epithelial cells among many other types of cells.

As used herein, the term "topical delivery" means delivery of a substance to the skin or an epithelial layer accessible though non-invasive means. Typically, topical delivery of a cosmetic composition has a local effect on the subject, e.g., the topically delivered cosmetic composition has a pharmacodynamic effect at or proximate to the particular part of the body (e.g. skin) where the cosmetic composition is delivered.

As used herein, the term "cosmetic effect" means a change in the appearance of an integumentary member of a subject. An integumentary member of a subject can include, but is not limited to, skin, nails, hair, eyelashes, and other tissue. Cosmetic effects described herein can include changes in the appearance of an integumentary member of a subject, such as, but not limited to, improving skin appearance, enhancing skin rejuvenation, increasing moisturization and/or elasticity, preventing or reducing wrinkles, lightening or darking skin or hair, changing hair color or density, and/or providing other anti-aging care.

As used herein, the term "expression sequence" means a nucleic acid sequence that encodes a product, e.g., a peptide or polypeptide, or a regulatory nucleic acid. An exemplary expression sequence that codes for a peptide or polypeptide can include a plurality of nucleotide triads, each of which can code for an amino acid and is termed as a "codon".

As used herein, the term "modified ribonucleotide" means to a nucleotide with at least one modification to the sugar, the nucleobase, or the internucleoside linkage.

As used herein, the term "substantially resistant" can refer to one that has at least 50%, 55%, 60%, 65%, 70%, 75%, 80%, 85%, 90%, 95%, 96%, 97%, 98% or 99% resistance as compared to a reference.

"Polypeptide" and "protein" are used interchangeably and means a polymer of two or more amino acids joined by a covalent bond (e.g., an amide bond). Polypeptides as described herein can include full length proteins (e.g., fully processed proteins) as well as shorter amino acid sequences (e.g., fragments of naturally-occurring proteins or synthetic polypeptide fragments). Polypeptides can include naturally occurring amino acids (e.g., one of the twenty amino acids commonly found in peptides synthesized in nature, and known by the one letter abbreviations A, R, N, C, D, Q, E, G, H, I, L, K, M, F, P, S, T, W, Y and V) and non-naturally occurring amino acids (e.g., amino acids which is not one of the twenty amino acids commonly found in peptides synthesized in nature, including synthetic amino acids, amino acid analogs, modified amino acids, and amino acid mimetics).

As used herein, the term "carrier" means a compound, composition, reagent, or molecule that facilitates the transport or delivery of a composition (e.g., a circular polyribonucleotide) into a cell by a covalent modification of the circular polyribonucleotide, via a partially or completely encapsulating agent, or a combination thereof. Non-limiting examples of carriers include carbohydrate carriers (e.g., an anhydride- modified phytoglycogen or glycogen-type material), nanoparticles (e.g., a nanoparticle that encapsulates or is covalently linked binds to the circular polyribonucleotide), liposomes, fusosomes, ex vivo differentiated reticulocytes, exosomes, protein carriers (e.g., a protein covalently linked to the circular polyribonucleotide), or cationic carriers (e.g., a cationic lipopolymer or transfection reagent).

As used herein, the term "naked delivery" or "naked RNA" means a formulation of RNA for delivery to a cell without the aid of a carrier and without covalent modification to a moiety that aids in delivery to a cell. A naked delivery formulation is free from any transfection reagents, cationic carriers, carbohydrate carriers, nanoparticle carriers, or protein carriers. For example, naked delivery formulation of a circular polyribonucleotide is a formulation that comprises a circular polyribonucleotide without covalent modification and is free from a carrier. As used herein, the term "diluent" means a cosmetically acceptable vehicle comprising an inactive solvent in which a composition described herein (e.g., a composition comprising a circular polyribonucleotide) may be diluted or dissolved. A diluent can be an RNA solubilizing agent, a buffer, an isotonic agent, or a mixture thereof. A cosmetically acceptable diluent can be a liquid diluent or a solid diluent. Non-limiting examples of liquid diluents include water or other solvents, solubilizing agents and emulsifiers such as ethyl alcohol, isopropyl alcohol, ethyl carbonate, ethyl acetate, benzyl alcohol, benzyl benzoate, propylene glycol, 1,3- butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and 1,3-butanediol. Non-limiting examples of solid diluents include calcium carbonate, sodium carbonate, calcium phosphate, dicalcium phosphate, calcium sulfate, calcium hydrogen phosphate, sodium phosphate lactose, sucrose, cellulose, microcrystalline cellulose, kaolin, mannitol, sorbitol, inositol, sodium chloride, dry starch, cornstarch, or powdered sugar.

As used herein, the term "sterilizing agent" means any agent that is bacteriostatic, bactericidal, and/or actively kills microorganisms, inactivates microorganisms, or prevents microorganisms from growing. A sterilizing agent that kills microorganisms can be antimicrobial and/or antiseptic. The sterilizing agent may be a liquid, such as an alcohol, iodine, or hydrogen peroxide. In some embodiments, the sterilizing agent, is UV light or a laser light. The sterilizing agent may be heat delivered electrically or through other means (e.g., vapor, contact).

### BRIEF DESCRIPTION OF THE DRAWINGS

The following detailed description of the embodiments of the invention will be better understood when read in conjunction with the appended drawings. For the purpose of illustrating the invention, there are shown in the drawings embodiments, which are presently exemplified. It should be understood, however, that the invention is not limited to the precise arrangement and instrumentalities of the embodiments shown in the drawings.

**FIG. 1** illustrates an example in which both exemplary linear and circular RNA were delivered topically to the ear skin of mice and their RNA levels in the ear tissue were examined over the 6 hour to 3 days after delivery.

**FIGS. 2A and 2B** are plots summarizing qPCR results from ear punches of mice 6 hours, 1 day, 3 days, or 12 days after topical delivery of linear or circular RNAs with the aid of Boost (ethanol). **FIG. 2A** shows results from qPCR assays using primers for detection of both the linear and circular RNAs. **FIG. 2B** shows results from qPCR assays using primers for detection of the circular RNA, but not the linear RNA.

**FIGS. 3A and 3B** are plots summarizing qPCR results from ear punches of mice 6 hours, 1 day, 3 days, or 12 days after topical delivery of linear or circular RNAs with the aid of both Boost (ethanol) and TransIT mRNA agent (Mirus Bio, LLC), a cationic, non-liposomal polymer/lipid transit agent. **FIG. 3A** shows results from qPCR assays using primers for detection of both the linear and circular RNAs. **FIG. 3B** shows results from qPCR assays using primers for detection of the circular RNA, but not the linear RNA.

**FIG. 4** illustrates the protein expression from topically administered RNA using DMSO gel (RNA) or DMSO gel alone (vehicle).

**FIG. 5** illustrates the protein expression from topically administered RNA formulated with Johnson & Johnson baby lotion (RNA) or Johnson & Johnson baby lotion alone (vehicle).

**FIG. 6** illustrates protein expression from topically administered RNA with ethanol (RNA) or ethanol alone (vehicle).

**FIG. 7** shows fluorescent images (B/W) from topical administration of circRNA-Cy5 results in RNA delivery to tissue.

**FIG. 8** shows quantification of fluorescent images from topical administration of circRNA-Cy5 results in RNA delivery to tissue.

**FIG. 9** shows fluorescent images (B/W) from topical administration of mRNA-Cy5 results in RNA delivery to tissue.

**FIG. 10** shows quantification of fluorescent images from topical administration of mRNA-Cy5 results in RNA delivery to tissue.

**FIG. 11** shows topical administration of mRNA results in RNA delivery to tissue at day 1 and day 4 after administration when the tissue is wiped with an ethanol wipe prior to application.

**FIG. 12** shows topical administration of mRNA results in RNA delivery to tissue at day 1 and day 4 after administration when tissue is wiped with an isopropyl alcohol wipe prior to application.

**FIG. 13** shows topical administration of circular RNA results in RNA delivery to tissue at day 1 and day 4 after administration when tissue is wiped with an ethanol wipe prior to application.

**FIG. 14** shows topical administration of circular RNA mixed with 10% ethanol results in RNA delivery to tissue at day 1 and day 4 after administration.

**FIG. 15** shows topical administration of circular RNA mixed with 10% isopropyl alcohol results in RNA delivery to tissue at day 1 and day 4 after administration.

**FIG. 16** shows topical administration of circular RNA results in RNA delivery to tissue at day 1 and day 4 after administration when the tissue is wiped with an isopropyl alcohol wipe prior to application.

**FIG. 17** shows topical administration of linear mRNA mixed with PBS, PBS and 10% ethanol, or PBS and 10% isopropyl alcohol results in RNA delivery to tissue at day 1 after administration, and topical administration of linear mRNA mixed with PBS and 10% ethanol or PBS and 10% isopropyl alcohol results in RNA delivery to tissue at day 4 after administration.

**FIG. 18** shows topical administration of circRNA results in expression of functional protein in tissue when tissue is wiped with an ethanol wipe prior to application.

**FIG. 19** shows topical administration of circRNA results in RNA delivery to tissue when circRNA is administered with 10% ethanol.

**FIG. 20** shows topical administration of circRNA results in RNA delivery to tissue when circRNA is administered with 10% isopropyl alcohol.

**FIG. 21** shows topical administration of mRNA results in RNA delivery to tissue when the skin is wiped with an ethanol wipe before application.

**FIG. 22** shows topical administration of mRNA results in RNA delivery to tissue when the mRNA is administered with PBS only.

**FIG. 23** shows topical administration of mRNA results in RNA delivery to tissue when the mRNA is administered with 10% isopropyl alcohol.

### DETAILED DESCRIPTION

This disclosure relates generally to compositions, preparations, and delivery of polyribonucleotides and applications thereof for cosmetic uses. The polyribonucleotides can be circular polyribonucleotides (circRNAs), or for reference linear polyribonucleotides, or for reference a combination thereof. The polyribonucleotide can comprise a payload. The payload can be a cosmetic payload, such as a sequence encoding any protein or peptide that has a cosmetic effect. In some aspects, the payload is a cosmetic protein or peptide selected from the group consisting of collagen, keratin, elastin, and botulinum toxin, or any a fragment thereof.

The present disclosure provides compositions and methods for delivery of circular polyribonucleotide to a cell. The compositions and methods provided herein may deliver circular polyribonucleotides into a cell to modify the appearance of a surface area of a subject, e.g., a member of the intergumentary system of a subject. The compositions and methods provided herein are particularly useful for topical delivery of polyribonucleotides into a cell of a subject.

The compositions disclosed herein can include a mixture of a cosmetic polyribonucleotide and an alcohol (e.g., ethanol). The methods disclosed herein can include delivering a polyribonucleotide in a composition comprising a mixture of the cosmetic polyribonucleotide and an alcohol. The present disclosure provides a kit comprising a cosmetic circular polyribonucleotide and an alcohol for delivery of the circular polyribonucleotide into a cell. In some embodiments, the kit comprises a sterilizing agent.

The compositions disclosed herein can include a mixture of a cosmetic circular polyribonucleotide and a cell-penetrating agent. The methods disclosed herein can include delivering a circular polyribonucleotide in a composition comprising a mixture of the cosmetic circular polyribonucleotide and a cell-penetrating agent. In Disclosed is a kit comprising a cosmetic circular polyribonucleotide and a cell-penetrating agent for delivery of the circular polyribonucleotide into a cell. In some embodiments, the kit comprises a sterilizing agent.

The cosmetic compositions described herein may be directly administered to a surface area (e.g., a topical surface area or integumentary member). The cosmetic compositions described herein may be applied to a surface area of a subject after application of a sterilizing agent.

The compositions, methods, and kits provided herein can offer a simple and effective solution in which to delivery polyribonucleotides into cells. A polyribonucleotide can be delivered into a cell more efficiently in the presence of the alcohol or the cell-penetrating agent than in the absence of the alcohol or cell-penetrating agent. In some cases, the alcohol or cell-penetrating agent described herein can increase the efficiency of delivery of the polynucleotide by at least about 10%, 15%, 20%, 30%, 40%, 50%, 60%, 70%, 80%, 90%, 100%, 120%, 150%, 200%, 250%, 300%, 400%, 500%, 600%, 700%, 800%, 900%, 1000%, 2000%, 5000%, 8000%, 10,000%, 20,000%, or 50,000% as compared to the efficiency of delivery of the polynucleotide in the absence of the alcohol or cell-penetrating agent.

### COMPOSITIONS FOR POLYRIBONUCLEOTIDE DELIVERY

In some aspects, the present disclosure provides compositions or cosmetic compositions for delivery of a circular polyribonucleotide. The polyribonucleotide can comprise a payload, such as a cosmetic payload. A cosmetic payload can be any protein or peptide that has a cosmetic effect. In some aspects, the cosmetic payload is a cosmetic protein or peptide selected from the group consisting of collagen, keratin, elastin, and botulinum toxin, or any a fragment thereof. The compositions or cosmetic compositions can comprise an alcohol (e.g., ethanol) for delivery of the polyribonucleotide to a cell. The compositions or cosmetic compositions can comprise a cell-penetrating agent. The cell-penetrating agent is configured to enhance delivery of the polyribonucleotide into a cell. The polyribonucleotide are present in circular form.

In some cases, the compositions or cosmetic compositions provided herein are suitable for cosmetic applications, e.g., the circular polyribonucleotide is a cosmetic circular polyribonucleotide that has cosmetic effects when the composition is administered to a subject. The circular polyribonucleotide may encode a cosmetic protein for use in the treatment or care of skin, hair, nails, or eyelashes.

In aspects, the present disclosure provides cosmetic compositions and methods of administering the compositions or cosmetic compositions described herein. The compositions or cosmetic compositions described herein may be directly administered to a surface area (e.g., a topical surface area or integumentary member). The compositions or cosmetic compositions described herein may be applied to a surface area (e.g., integumentary member) of a subject after application of a sterilizing agent.

The compositions or cosmetic compositions can comprise a cell-penetrating agent and a polyribonucleotide. The cell-penetrating agent is configured to enhance delivery of the polyribonucleotide into a cell. In some cases, the compositions provided herein are suitable for cosmetic applications, e.g., the polyribonucleotide is a cosmetic polyribonucleotide that has cosmetic effects when the composition is administered to a subject. In aspects, the present disclosure provides cosmetic compositions and methods of administering the cosmetic compositions described herein. The polyribonucleotide is present in or circular form.

### Alcohol

Alcohol as described herein can be used for the delivery of a polyribonucleotide (e.g., a cosmetic polyribonucleotide) into a cell. An alcohol can be in a mixture with a polyribonucleotide as described herein for delivery of the polyribonucleotide into a cell. The mixture can comprise the alcohol in 0.3% v/v alcohol to 75% v/v. The alcohol can be ethanol. In some embodiments, the mixture is applied to a surface area of a subject. In some embodiments, the mixture is a cosmetic composition.

An alcohol can be any alcohol that comprises one or more hydroxyl function groups. In some cases, the alcohol is, but is not limited to, a monohydric alcohol, a polyhydric alcohol, an unsaturated aliphatic alcohol, or an alicyclic alcohol. The alcohol can include one or more of methanol, ethanol, isopropanol, butanol, pentanol, cetyl alcohol, ethylene glycol, propylene glycol, denatured alcohol, benzyl alcohol, specially denatured alcohol, glycol, stearyl alcohol, cetearyl alcohol, menthol, or polyethylene glycols (PEG)-400. In certain embodiments, the alcohol is ethanol.

In other cases, the compositions, cosmetic compositions, and methods provided herein only include an alcohol and do not have or use any other agent to enhance the delivery of the polyribonucleotide into a cell. In some cases, the alcohol is ethanol and the composition, cosmetic composition, and methods do not have or use any other agent to enhance delivery of polyribonucleotide into a cell. In some cases, the alcohol is a cell-penetrating agent. In some cases the alcohol is not a cell-penetrating agent.

The composition disclosed herein can include a mixture of an alcohol and a polyribonucleotide. In some cases, the polyribonucleotide is present in a pre-mixed mixture with the alcohol. In some cases, the polyribonucleotides is provided separately from the alcohol prior to contact to a cell. In these instances, the polyribonucleotide is contacted with the alcohol when being applied to a cell, and becomes mixed together for delivery of the polyribonucleotide into the cell. Without being bound to a certain theory, the concentration of the alcohol in the mixture can contribute to the efficiency of delivery. Therefore, in some cases, the alcohol is provided at a predetermined concentration in the mixture. In some other cases, when the alcohol and the polyribonucleotide are separate initially but mixed together when being applied for delivery, the alcohol is provided at a sufficient amount relative to the polyribonucleotide that would ensure it reach a minimum predetermined concentration in the mixture.

In some cases, the alcohol constitutes at least about 0.01%, at least about 0.02%, at least about 0.03%, at least about 0.04%, at least about 0.05%, at least about 0.06%, at least about 0.07%, at least about 0.08%, at least about 0.09%, at least about 0.1%, at least about 0.2%, at least about 0.3%, at least about 0.4%, at least about 0.5%, at least about 0.6%, at least about 0.7%, at least about 0.9%, at least about 1%, at least about 2%, at least about 3%, at least about 4%, at least about 5%, at least about 6%, at least about 7%, at least about 8%, at least about 9%, at least about 10%, at least about 20%, at least about 30%, at least about 40%, at least about 50%, at least about 60%, at least about 70%, at least about 80%, at least about 90%, at least about 95%, or at least about 98% volume per volume (v/v) of the mixture. In some cases, the alcohol constitutes at most about 0.01%, at most about 0.02%, at most about 0.03%, at most about 0.04%, at most about 0.05%, at most about 0.06%, at most about 0.07%, at most about 0.08%, at most about 0.09%, at most about 0.1%, 0.2%, 0.3%, 0.4%, 0.5%, 0.6%, 0.7%, 0.9%, 1%, 2%, 3%, 4%, 5%, 6%, 7%, 8%, 9%, 10%, 20%, 30%, 40%, 50%, 60%, 70%, 80%, or 90% v/v of the mixture. In some cases, the alcohol constitutes about 10%, about 20%, about 30%, about 40%, about 50%, about 60%, about 70%, about 80%, about 90%, about 95%, about 98%, or about 100% v/v of the mixture.

In some cases, the alcohol constitutes at least about 0.01%, at least about 0.02%, at least about 0.03%, at least about 0.04%, at least about 0.05%, at least about 0.06%, at least about 0.07%, at least about 0.08%, at least about 0.09%, at least about 0.1%, at least about 0.2%, at least about 0.3%, at least about 0.4%, at least about 0.5%, at least about 0.6%, at least about 0.7%, at least about 0.9%, at least about 1%, at least about 2%, at least about 3%, at least about 4%, at least about 5%, at least about 6%, at least about 7%, at least about 8%, at least about 9%, at least about 10%, at least about 20%, at least about 30%, at least about 40%, at least about 50%, at least about 60%, at least about 70%, at least about 80%, at least about 90%, at least about 95%, or at least about 98% weight per weight (w/w) of the mixture. In some cases, the alcohol constitutes at most about 0.01%, at most about 0.02%, at most about 0.03%, at most about 0.04%, at most about 0.05%, at most about 0.06%, at most about 0.07%, at most about 0.08%, at most about 0.09%, at most about 0.1%, 0.2%, 0.3%, 0.4%, 0.5%, 0.6%, 0.7%, 0.9%, 1%, 2%, 3%, 4%, 5%, 6%, 7%, 8%, 9%, 10%, 20%, 30%, 40%, 50%, 60%, 70%, 80%, or 90% w/w of the mixture. In some cases, the cell-penetrating agent constitutes about 10%, about 20%, about 30%, about 40%, about 50%, about 60%, about 70%, about 80%, about 90%, about 95%, or about 98% w/w of the mixture. In some cases, the alcohol constitutes about 10% v/v of the mixture.

In some embodiments, the alcohol constitutes 0.5% v/v to 75% v/v, 1% v/v to 75% v/v, 5% v/v to 75% v/v, 10% v/v to 75% v/v, 15% v/v to 75% v/v, 20% v/v to 75% v/v, 30% v/v to 75% v/v, 40% v/v to 75% v/v, 50% v/v to 75% v/v, 60% v/v to 75% v/v, or 70% v/v to 75% v/v of the mixture, or any percentage v/v therebetween. In some embodiments, the alcohol constitutes 0.3% v/v to 70% v/v, 0.3% v/v to 60% v/v, 0.3% v/v to 50% v/v, 0.3% v/v to 40% v/v, 30% v/v to 20% v/v, 0.3% v/v to 15% v/v, 0.3% v/v to 10% v/v, 0.3% v/v to 5% v/v, 0.3% v/v to 1% v/v, or 0.3% v/v to 0.5% v/v of the mixture, or any percentage v/v therebetween.

In some cases, the mixture described herein is a liquid solution. For instance, the alcohol is a liquid substance itself. In these cases, the polyribonucleotide can also be dissolved in the liquid solution.

In some cases, ethanol constitutes at least about 0.1%, at least about 0.2%, at least about 0.3%, at least about 0.4%, at least about 0.5%, at least about 0.6%, at least about 0.7%, at least about 0.9%, at least about 1%, at least about 2%, at least about 3%, at least about 4%, at least about 5%, at least about 6%, at least about 7%, at least about 8%, at least about 9%, at least about 10%, at least about 20%, at least about 30%, at least about 40%, at least about 50%, at least about 60%, at least about 70%, at least about 80%, at least about 90%, at least about 95%, or at least about 98% volume per volume (v/v) of the mixture. In some cases, ethanol constitutes at most about 0.1%, 0.2%, 0.3%, 0.4%, 0.5%, 0.6%, 0.7%, 0.9%, 1%, 2%, 3%, 4%, 5%, 6%, 7%, 8%, 9%, 10%, 20%, 30%, 40%, 50%, 60%, 70%, 80%, or 90% v/v of the mixture. In some cases, ethanol constitutes about 10%, about 20%, about 30%, about 40%, about 50%, about 60%, about 70%, about 80%, about 90%, about 95%, about 98%, or about 100% v/v of the mixture. In some cases, ethanol constitutes about 10% v/v of the mixture.

In some embodiments, the ethanol constitutes 0.5% v/v to 75% v/v, 1% v/v to 75% v/v, 5% v/v to 75% v/v, 10% v/v to 75% v/v, 15% v/v to 75% v/v, 20% v/v to 75% v/v, 30% v/v to 75% v/v, 40% v/v to 75% v/v, 50% v/v to 75% v/v, 60% v/v to 75% v/v, or 70% v/v to 75% v/v of the mixture, or any percentage v/v therebetween. In some embodiments, the ethanol constitutes 0.3% v/v to 70% v/v, 0.3% v/v to 60% v/v, 0.3% v/v to 50% v/v, 0.3% v/v to 40% v/v, 30% v/v to 20% v/v, 0.3% v/v to 15% v/v, 0.3% v/v to 10% v/v, 0.3% v/v to 5% v/v, 0.3% v/v to 1% v/v, or 0.3% v/v to 0.5% v/v of the mixture, or any percentage v/v therebetween.

### Cell-Penetrating Agents

The cell-penetrating agent described herein can include any substance that enhances delivering a polyribonucleotide into a cell. The cell-penetrating agent can include an organic compound or an inorganic molecule. In some cases, the cell-penetrating agent is an organic compound having one or more functional groups such as, but not limited to, alkane, alkene, and arene; halogen-substituted alkane, alkenes, and arenes; alcohols, phenols (derivatives of benzene), ethers, aldehydes, ketones, and carboxylic acids; amines and nitriles. In some embodiments, the cell-penetrating agent is soluble in polar solvents. In some embodiments, the cell-penetrating agent is insoluble in polar solvents. The polyribonucleotide are present in circular form.

The cell-penetrating agent can include organic compounds such as alcohols having one or more hydroxyl function groups. In some cases, the cell-penetrating agent includes an alcohol such as, but not limited to, monohydric alcohols, polyhydric alcohols, unsaturated aliphatic alcohols, and alicyclic alcohols. The cell-penetrating agent can include one or more of methanol, ethanol, isopropanol, butanol, pentanol, cetyl alcohol, ethylene glycol, propylene glycol, denatured alcohol, benzyl alcohol, specially denatured alcohol, glycol, stearyl alcohol, cetearyl alcohol, menthol, polyethylene glycols (PEG)-400, isopropyl alcohol, dimethylaminoethanol, retinol, d-alpha-tocopherol, SD alcohol, SD alcohol 40, c12-16, lauryl alcohol, myristyl alcohol, butylene glycol, or propanediol. In certain embodiments, the cell-penetrating agent comprises ethanol.

In other cases, the compositions and methods provided herein only include an alcohol as the cell-penetrating agent, and do not have or use any other agent to enhance the delivery of the polyribonucleotide into a cell. In some cases, the cell-penetrating agent comprises ethanol and any other alcohol that can enhance delivery of polyribonucleotide into a cell. In some cases, the cell-penetrating agent comprises ethanol and any other organic or inorganic molecules that can enhance delivery of polyribonucleotide into a cell. In some cases, the cell-penetrating agent comprises ethanol and liposome or nanoparticles such as those described in International Publication Nos. WO2013006825, WO2016036735, WO2018112282A1, and WO2012031043A1. In some cases, the cell-penetrating agent comprises ethanol and cell-penetrating peptides or proteins such as those described in Bechara et al, Cell-penetrating peptides: 20 years later, where do we stand? FEBS Letters 587(12):1693-1702 (2013); Langel, Cell-Penetrating Peptides: Processes and Applications (CRC Press, Boca Raton FL, 2002); El-Andaloussi et al., Curr. Pharm. Des. 11(28):3597-611 (2003); Deshayes et al, Cell. Mol. Life Sci. 62(16): 1839-49 (2005), US Patent Publication Nos. US20130129726, US20130137644 and US20130164219). In some cases, the ratio of ethanol versus other cell-penetrating agent is about 1:0.001, 1:0.002, 1: 005, 1:008, 1:0.01, 1:0.02, 1:0.05, 1:0.08, 1: 0.1, 1: 0.2, 1: 0.3, 1:0.4, 1:0.5, 1:0.6, 1:0.7, 1:0.8, 1:0.9, 1:1, 1:1.2, 1: 1.5, 1: 1.8, 1: 2, 1:2.5, 1:3, 1:3.5, 1:4, 1:5, 1:6, 1:7, 1:8, 1:9, 1:10, 1:15, 1:20, 1:30, 1:40, 1:50, 1:60, 1:70, 1:80, 1:90, 1:100, 1:120, 1:150, 1:200, 1:250, 1:500, or 1:1000. In some cases, the ratio of ethanol versus other cell-penetrating agent is at least about 1:0.001, 1:0.002, 1: 005, 1:008, 1:0.01, 1:0.02, 1:0.05, 1:0.08, 1: 0.1, 1: 0.2, 1: 0.3, 1:0.4, 1:0.5, 1:0.6, 1:0.7, 1:0.8, 1:0.9, 1:1, 1:1.2, 1: 1.5, 1: 1.8, 1: 2, 1:2.5, 1:3, 1:3.5, 1:4, 1:5, 1:6, 1:7, 1:8, 1:9, 1:10, 1:15, 1:20, 1:30, 1:40, 1:50, 1:60, 1:70, 1:80, 1:90, 1:100, 1:120, 1:150, 1:200, 1:250, or 1:500.

The composition disclosed herein can include a mixture of a cell-penetrating agent and a polyribonucleotide. In some cases, the polyribonucleotide is present in a pre-mixed mixture with the cell-penetrating agent. In some cases, the polyribonucleotides is provided separately from the cell-penetrating agent prior to contact to a cell. In these instances, the polyribonucleotide is contacted with the cell-penetrating agent when being applied to a cell, and becomes mixed together for delivery of the polyribonucleotide into the cell. Without being bound to a certain theory, the concentration of the cell-penetrating agent in the mixture can contribute to the efficiency of delivery. Therefore, in some cases, the cell-penetrating agent is provided at a predetermined concentration in the mixture. In some other cases, when the cell-penetrating agent and the polyribonucleotide are separate initially but mixed together when being applied for delivery, the cell-penetrating agent is provided at a sufficient amount relative to the polyribonucleotide that would ensure it reach a minimum predetermined concentration in the mixture.

In some cases, the cell-penetrating agent constitutes at least about 0.01%, at least about 0.02%, at least about 0.03%, at least about 0.04%, at least about 0.05%, at least about 0.06%, at least about 0.07%, at least about 0.08%, at least about 0.09%, at least about 0.1%, at least about 0.2%, at least about 0.3%, at least about 0.4%, at least about 0.5%, at least about 0.6%, at least about 0.7%, at least about 0.9%, at least about 1%, at least about 2%, at least about 3%, at least about 4%, at least about 5%, at least about 6%, at least about 7%, at least about 8%, at least about 9%, at least about 10%, at least about 20%, at least about 30%, at least about 40%, at least about 50%, at least about 60%, at least about 70%, at least about 80%, at least about 90%, at least about 95%, or at least about 98% volume per volume (v/v) of the mixture. In some cases, the cell-penetrating agent constitutes at most about 0.01%, at most about 0.02%, at most about 0.03%, at most about 0.04%, at most about 0.05%, at most about 0.06%, at most about 0.07%, at most about 0.08%, at most about 0.09%, at most about 0.1%, 0.2%, 0.3%, 0.4%, 0.5%, 0.6%, 0.7%, 0.9%, 1%, 2%, 3%, 4%, 5%, 6%, 7%, 8%, 9%, 10%, 20%, 30%, 40%, 50%, 60%, 70%, 80%, or 90% v/v of the mixture. In some cases, the cell-penetrating agent constitutes about 10%, about 20%, about 30%, about 40%, about 50%, about 60%, about 70%, about 80%, about 90%, about 95%, about 98%, or about 100% v/v of the mixture.

In some cases, the cell-penetrating agent constitutes at least about 0.01%, at least about 0.02%, at least about 0.03%, at least about 0.04%, at least about 0.05%, at least about 0.06%, at least about 0.07%, at least about 0.08%, at least about 0.09%, at least about 0.1%, at least about 0.2%, at least about 0.3%, at least about 0.4%, at least about 0.5%, at least about 0.6%, at least about 0.7%, at least about 0.9%, at least about 1%, at least about 2%, at least about 3%, at least about 4%, at least about 5%, at least about 6%, at least about 7%, at least about 8%, at least about 9%, at least about 10%, at least about 20%, at least about 30%, at least about 40%, at least about 50%, at least about 60%, at least about 70%, at least about 80%, at least about 90%, at least about 95%, or at least about 98% weight per weight (w/w) of the mixture. In some cases, the cell-penetrating agent constitutes at most about 0.01%, at most about 0.02%, at most about 0.03%, at most about 0.04%, at most about 0.05%, at most about 0.06%, at most about 0.07%, at most about 0.08%, at most about 0.09%, at most about 0.1%, 0.2%, 0.3%, 0.4%, 0.5%, 0.6%, 0.7%, 0.9%, 1%, 2%, 3%, 4%, 5%, 6%, 7%, 8%, 9%, 10%, 20%, 30%, 40%, 50%, 60%, 70%, 80%, or 90% w/w of the mixture. In some cases, the cell-penetrating agent constitutes about 10%, about 20%, about 30%, about 40%, about 50%, about 60%, about 70%, about 80%, about 90%, about 95%, or about 98% w/w of the mixture. In some cases, the cell-penetrating agent constitutes about 10% v/v of the mixture.

In some cases, the mixture described herein is a liquid solution. For instance, the cell-penetrating agent is a liquid substance itself. Alternatively, the cell-penetrating agent is a solid, liquid, or gas substance and dissolved in a liquid carrier, e.g., water. In these cases, the polyribonucleotide can also be dissolved in the liquid solution.

In some cases, ethanol constitutes at least about 0.1%, at least about 0.2%, at least about 0.3%, at least about 0.4%, at least about 0.5%, at least about 0.6%, at least about 0.7%, at least about 0.9%, at least about 1%, at least about 2%, at least about 3%, at least about 4%, at least about 5%, at least about 6%, at least about 7%, at least about 8%, at least about 9%, at least about 10%, at least about 20%, at least about 30%, at least about 40%, at least about 50%, at least about 60%, at least about 70%, at least about 80%, at least about 90%, at least about 95%, or at least about 98% volume per volume (v/v) of the mixture. In some cases, ethanol constitutes at most about 0.1%, 0.2%, 0.3%, 0.4%, 0.5%, 0.6%, 0.7%, 0.9%, 1%, 2%, 3%, 4%, 5%, 6%, 7%, 8%, 9%, 10%, 20%, 30%, 40%, 50%, 60%, 70%, 80%, or 90% v/v of the mixture. In some cases, ethanol constitutes about 10%, about 20%, about 30%, about 40%, about 50%, about 60%, about 70%, about 80%, about 90%, about 95%, about 98%, or about 100% v/v of the mixture. In some cases, ethanol constitutes about 10% v/v of the mixture.

### Polyribonucleotides

The present disclosure relates to compositions and methods for delivering circular polyribonucleotides, e.g., cosmetic circular polyribonucleotides, into a cell. The polyribonucleotides are circular. In some cases, the cosmetic polyribonucleotides have cosmetic effects on the appearance of an integumentary member of a subject that the polyribonucleotides are administered to. An integumentary member of a subject can include, but not limited to, skin, eyelashes, nails, and hair. The disclosure provides cosmetic compositions comprising cosmetic circular polyribonucleotides that have cosmetic effects on a subject, when the composition is delivered into a cell in the subject by topical administration. The polyribonucleotides as described herein can be mixed with an alcohol (e.g., ethanol) in a cosmetic composition. The polyribonucleotides can be mixed with a cell-penetrating agent. The polyribonucleotides as described herein can be mixed with a cosmetically acceptable diluent in cosmetic composition that is free of any carrier.

The polyribonucleotide can include sequences for expression products. Expression of sequences from the polyribonucleotide and/or binding of the polyribonucleotide to a target can have various cosmetic effects. In some cases, the polyribonucleotide modulates a cellular function, e.g., transiently or in a long term. The cosmetic cellular function may be stably altered, such as a modulation that persists for at least about 1 hr to about 30 days, or at least about 2 hrs, 6 hrs, 12 hrs, 18 hrs, 24 hrs, 2 days, 3, days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, 60 days, or longer or any time therebetween. The cosmetic cellular function may be transiently altered, e.g., such as a modulation that persists for no more than about 30 mins to about 7 days, or no more than about 1 hr, 2 hrs, 3 hrs, 4 hrs, 5 hrs, 6 hrs, 7 hrs, 8 hrs, 9 hrs, 10 hrs, 11 hrs, 12 hrs, 13 hrs, 14 hrs, 15 hrs, 16 hrs, 17 hrs, 18 hrs, 19 hrs, 20 hrs, 21 hrs, 22 hrs, 24 hrs, 36 hrs, 48 hrs, 60 hrs, 72 hrs, 4 days, 5 days, 6 days, 7 days, or any time therebetween.

The polyribonucleotide can be at least about 20 nucleotides, at least about 30 nucleotides, at least about 40 nucleotides, at least about 50 nucleotides, at least about 75 nucleotides, at least about 100 nucleotides, at least about 200 nucleotides, at least about 300 nucleotides, at least about 400 nucleotides, at least about 500 nucleotides, or at least about 1,000 nucleotides. In some cases, the polyribonucleotide is of a sufficient size to accommodate a binding site for a ribosome for expression of sequences from the polyribonucleotide. One of skill in the art can appreciate that the maximum size of a linear or circular polyribonucleotide can be as large as is within the technical constraints of producing a linear or circular polyribonucleotide, and/or using the circular polyribonucleotide. In some cases, the maximum size of a linear or circular polyribonucleotide provided herein can be limited by the ability of packaging and delivering the RNA to a target. In some cases, the size of a polyribonucleotide is a length sufficient to encode useful polypeptides, and thus, lengths of less than about 20,000 nucleotides, less than about 15,000 nucleotides, less than about 10,000 nucleotides, less than about 7,500 nucleotides, or less than about 5,000 nucleotides, less than about 4,000 nucleotides, less than about 3,000 nucleotides, less than about 2,000 nucleotides, less than about 1,000 nucleotides, less than about 500 nucleotides, less than about 400 nucleotides, less than about 300 nucleotides, less than about 200 nucleotides, less than about 100 nucleotides may be useful.

The polyribonucleotide provided herein can have one or more modifications, such as substitutions, insertions and/or additions, deletions, and covalent modifications with respect to reference sequences, in particular, the parent polyribonucleotide. For example, the polyribonucleotide includes one or more post-transcriptional modifications (e.g., capping, cleavage, polyadenylation, splicing, poly-A sequence, methylation, acylation, phosphorylation, methylation of lysine and arginine residues, acetylation, and nitrosylation of thiol groups and tyrosine residues, etc). The polyribonucleotide can include at least one nucleoside selected from the group consisting of pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, 1-taurinomethyl-4-thio-uridine, 5-methyl-uridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, and 4-methoxy-2-thio-pseudouridine. In some cases, the mRNA includes at least one nucleoside selected from the group consisting of 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, and 4-methoxy-1-methyl-pseudoisocytidine. In some cases, the mRNA includes at least one nucleoside selected from the group consisting of 2-aminopurine, 2, 6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl) adenosine, N6-glycinylcarbamoyladenosine, N6-threonylcarbamoyladenosine, 2-methylthio-N6-threonyl carbamoyladenosine, N6,N6-dimethyladenosine, 7-methyladenine, 2-methylthio-adenine, and 2-methoxy-adenine. In some cases, mRNA includes at least one nucleoside selected from the group consisting of inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, 1-methyl-6-thio-guanosine, N2-methyl-6-thio-guanosine, and N2,N2-dimethyl-6-thio-guanosine. In some embodiments, the circular polyribonucleotide lacks a cap. In some embodiments, the polyribonucleotide lacks a poly-A tail. The circular polyribonucleotide may be non-immunogenic. The circular polyribonucleotide may be immunogenic.

The polyribonucleotide can include any useful modification, such as to the sugar, the nucleobase, or the internucleoside linkage (e.g., to a linking phosphate / to a phosphodiester linkage / to the phosphodiester backbone). One or more atoms of a pyrimidine nucleobase may be replaced or substituted with optionally substituted amino, optionally substituted thiol, optionally substituted alkyl (e.g., methyl or ethyl), or halo (e.g., chloro or fluoro). Modifications (e.g., one or more modifications) may be present in each of the sugar and the internucleoside linkage. Modifications may be modifications of ribonucleic acids (RNAs) to deoxyribonucleic acids (DNAs), threose nucleic acids (TNAs), glycol nucleic acids (GNAs), peptide nucleic acids (PNAs), locked nucleic acids (LNAs) or hybrids thereof). Additional modifications are described herein.

Different sugar modifications, nucleotide modifications, and/or internucleoside linkages (e.g., backbone structures) can exist at various positions in a polyribonucleotide provided herein. One of ordinary skill in the art will appreciate that the nucleotide analogs or other modification(s) can be located at any position(s) of the polyribonucleotide, such that the function of the polyribonucleotide is not substantially decreased. The polyribonucleotide can include from about 1% to about 100% modified nucleotides (either in relation to overall nucleotide content, or in relation to one or more types of nucleotide, e.g., any one or more of A, G, U, or C) or any intervening percentage (e.g., from 1% to 20%>, from 1% to 25%, from 1% to 50%, from 1% to 60%, from 1% to 70%, from 1% to 80%, from 1% to 90%, from 1% to 95%, from 10% to 20%, from 10% to 25%, from 10% to 50%, from 10% to 60%, from 10% to 70%, from 10% to 80%, from 10% to 90%, from 10% to 95%, from 10% to 100%, from 20% to 25%, from 20% to 50%, from 20% to 60%, from 20% to 70%, from 20% to 80%, from 20% to 90%, from 20% to 95%, from 20% to 100%, from 50% to 60%, from 50% to 70%, from 50% to 80%, from 50% to 90%, from 50% to 95%, from 50% to 100%, from 70% to 80%, from 70% to 90%, from 70% to 95%, from 70% to 100%, from 80% to 90%, from 80% to 95%, from 80% to 100%, from 90% to 95%, from 90% to 100%, and from 95% to 100%).

In some cases, a concentration of the polyribonucleotide in the mixture is at least about 0.1 ng/mL, at least about 0.2 ng/mL, at least about 0.5 ng/mL, at least about 1 ng/mL, at least about 5 ng/mL, at least about 10 ng/mL, at least about 20 ng/mL, at least about 50 ng/mL, at least about 100 ng/mL, at least about 200 ng/mL, at least about 500 ng/mL, at least about 1 µg/mL, at least about 2 µg/mL, at least about 3 µg/mL, at least about 4 µg/mL, at least about 5 µg/mL, at least about 10 µg/mL, at least about 20 µg/mL, at least about 30 µg/mL, at least about 40 µg/mL, at least about 50 µg/mL, at least about 100 µg/mL, at least about 200 µg/mL, at least about 300 µg/mL, at least about 400 µg/mL, at least about 500 µg/mL, at least about 1 mg/mL, at least about 2 mg/mL, at least about 5 mg/mL, at least about 10 mg/mL, at least about 20 mg/mL, at least about 50 mg/mL, or at least about 100 mg/mL. In some cases, a concentration of the polyribonucleotide in the mixture is at most about 0.1 ng/mL, at most about 0.2 ng/mL, at most about 0.5 ng/mL, at most about 1 ng/mL, at most about 5 ng/mL, at most about 10 ng/mL, at most about 20 ng/mL, at most about 50 ng/mL, at most about 100 ng/mL, at most about 200 ng/mL, at most about 500 ng/mL, at most about 1 µg/mL, at most about 2 µg/mL, at most about 3 µg/mL, at most about 4 µg/mL, at most about 5 µg/mL, at most about 10 µg/mL, at most about 20 µg/mL, at most about 30 µg/mL, at most about 40 µg/mL, at most about 50 µg/mL, at most about 100 µg/mL, at most about 200 µg/mL, at most about 300 µg/mL, at most about 400 µg/mL, at most about 500 µg/mL, at most about 1 mg/mL, at most about 2 mg/mL, at most about 5 mg/mL, at most about 10 mg/mL, at most about 20 mg/mL, at most about 50 mg/mL, or at most about 100 mg/mL.

In some cases, a concentration of the polyribonucleotide in the mixture is about 0.1 ng/mL, about 0.2 ng/mL, about 0.5 ng/mL, about 1 ng/mL, about 5 ng/mL, about 10 ng/mL, about 20 ng/mL, about 50 ng/mL, about 100 ng/mL, about 200 ng/mL, about 500 ng/mL, about 1 µg/mL, about 2 µg/mL, about 3 µg/mL, about 4 µg/mL, about 5 µg/mL, about 10 µg/mL, about 20 µg/mL, about 30 µg/mL, about 40 µg/mL, about 50 µg/mL, about 100 µg/mL, about 200 µg/mL, about 300 µg/mL, about 400 µg/mL, about 500 µg/mL, about 1 mg/mL, about 2 mg/mL, about 5 mg/mL, about 10 mg/mL, about 20 mg/mL, about 50 mg/mL, or about 100 mg/mL.

In certain particular embodiments, the composition comprises a circular polyribonucleotide (circRNA) and an alcohol. Due to the circular structure, circRNA have improved stability, increased half-life, reduced immunogenicity, and/or improved functionality (e.g., of a function described herein) compared to a corresponding linear RNA.

The composition can comprise a polyribonucleotide and a cell-penetrating agent. In certain particular embodiments, the composition comprises a circular polyribonucleotide (circRNA) and a cell-penetrating agent. Due to the circular structure, circRNA have improved stability, increased half-life, reduced immunogenicity, and/or improved functionality (e.g., of a function described herein) compared to a corresponding linear RNA.

In some cases, the circular polyribonucleotide provided herein has a half-life of at least that of a linear counterpart, e.g., linear expression sequence, or linear circular polyribonucleotide. In some cases, the circular polyribonucleotide has a half-life that is increased over that of a linear counterpart. In some cases, the half-life is increased by about 5%, 10%, 15%, 20%, 25%, 30%, 35%, 40%, 45%, 50%, or greater. In some cases, the circular polyribonucleotide has a half-life or persistence in a cell for at least about 1 hr to about 30 days, or at least about 2 hrs, 6 hrs, 12 hrs, 18 hrs, 24 hrs, 2 days, 3, days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, 60 days, or longer or any time therebetween. The circular polyribonucleotide may have a half-life or persistence in a cell for no more than about 10 mins to about 7 days, or no more than about 1 hr, 2 hrs, 3 hrs, 4 hrs, 5 hrs, 6 hrs, 7 hrs, 8 hrs, 9 hrs, 10 hrs, 11 hrs, 12 hrs, 13 hrs, 14 hrs, 15 hrs, 16 hrs, 17 hrs, 18 hrs, 19 hrs, 20 hrs, 21 hrs, 22 hrs, 24 hrs, 36 hrs, 48 hrs, 60 hrs, 72 hrs, 4 days, 5 days, 6 days, 7 days, or any time therebetween.

### Payload and Cosmetic Effects

In some cases, the polyribonucleotide comprises at least one payload that can have cosmetic effects on the subject that the polyribonucleotide is delivered into. The polyribonucleotide are present in circular form. The payload can be a cosmetic payload. A cosmetic payload can be any payload that has a cosmetic effect after administration to a cell, tissue, or integumentary member. The payload can be one or more sequences in the polyribonucleotide that can be responsible for expression products. In some embodiments, the payload is a cosmetic protein or peptide selected from the group consisting of collagen, keratin, elastin, and botulinum toxin, or any a fragment thereof.

The cosmetic payload can be any protein or peptide that has cosmetic effect after administration. For example, the cosmetic payload can be collagen or fragments thereof, elastin or fragments thereof, fibronectin or fragments thereof, keratin or fragments thereof, botulinum toxin or fragments thereof, fragments of α-mealnocyte stimulating hormone. A cosmetic payload can be any extracellular matrix protein or fragments thereof. The cosmetic payload may be any bioactive peptide that has a cosmetic effect, such as palmitoyl-KTTKS peptide, a GEKG peptide, or a peptide comprising a KEK motif. A bioactive peptide is a peptide comprising a short sequence of amino acids (5-20 amino acids) derived from the enzymatic processing of endogenous proteins throughtout the body (e.g., from extracellular matrix proteins in the skin). A bioactive peptide that has a cosmetic effect can be any peptide that modulates a cosmetic cellular function, e.g., modulates collagen or melanin homeostasis The cosmetic payload may be a carrier peptide, such as a copper peptide or X-50 Myocept, which carries trace element, such as manganese and copper. A cosmetic effect modulated by a carrier peptide can be skin anti-aging. The cosmetic payload may be a neurotransmitter inhibitor peptide, such as XEP-30, palmitoyl tripeptide-38, palmitoyl tetrapeptide-28 argirelin, and syn-ake. A cosmetic effect modulated by a neurotransmitter inhibitor peptide can be reducing expression line wrinkles. The cosmetic payload may be an enzyme inhibitor peptide, such as trylagen, trifluoroacetyl tripeptide-2, silk fibroin peptides, and soybean peptides. A cosmetic effect modulated by an enzyme inhibitor can be anti-aging. The cosmetic payload may be a signal peptide, such as palmitoyl oligopeptide-7, copper peptides, palmitoyl pentapeptide-4, and matrixyl-3000. A cosmetic effect modulated by a signal peptide can be anti-aging.

In some cases, the payload has a cosmetic effect on the subject after administration. The cosmetic effect that the polyribonucleotide has can include modification of the appearance of an integumentary member of a subject, such as, but not limited to, improving skin appearance, enhancing skin rejuvenation, increasing moisturization and/or elasticity, preventing or reducing wrinkles, lightening or darking skin or hair, changing hair color or density, and/or providing other anti-aging care.

### Expression Sequence

In some cases, the polyribonucleotide as described herein comprises at least one expression sequence that encodes a peptide or polypeptide. The polyribonucleotide as described herein comprises at least one expression sequence that encodes a peptide or polypeptide that has a cosmetic effect. The peptide may include, but is not limited to, a protein, a small peptide, a peptidomimetic (e.g., peptoid), amino acids, and amino acid analogs. The peptide can be linear or branched. The peptide can have a molecular weight less than about 500,000 grams per mole, a molecular weight less than about 200,000 grams per mole, a molecular weight less than about 100,000 grams per mole, a molecular weight less than about 50,000 grams per mole, a molecular weight less than about 20,000 grams per mole, a molecular weight less than about 10,000 grams per mole, a molecular weight less than about 5,000 grams per mole, a molecular weight less than about 2,000 grams per mole, a molecular weight less than about 1,000 grams per mole, a molecular weight less than about 500 grams per mole, and salts, esters, and other pharmaceutically acceptable forms of such compounds. A polypeptide of interest can comprise, for example, whole polypeptides, a plurality of polypeptides or fragments of polypeptides, which independently may be encoded by one or more nucleic acids, a plurality of nucleic acids, fragments of nucleic acids or variants of any of the aforementioned. The polyribonucleotide is present in circular form.

In some cases, the polyribonucleotide comprises an expression sequence encoding a cosmetic protein that can contribute to modification of the appearance of an integumentary member of a subject such as, but not limited, to skin, hair, eyelashes, nails, and other tissues. Some examples of cosmetic proteins can include, but are not limited to, a protein replacement (e.g., replacement of proteins whose expression is reduced due to aging), protein supplementation, hormones, cytokines, cellular reprogramming/transdifferentiation factor, transcription factors. The protein may be a cosmetic protein, such as a hair, skin, or nail protein.

In some cases, the polyribonucleotide comprises a regulatory element, e.g., a sequence that modifies expression of an expression sequence within a circular polyribonucleotide or a linear polyribonucleotide. A regulatory element can encode a sequence that is located adjacent to an expression sequence that encodes an expression product. A regulatory element can be linked operatively to the adjacent sequence. A regulatory element can increase an amount of product expressed as compared to an amount of the expressed product without the presence of the regulatory element. In addition, one regulatory element can increase an amount of products expressed for multiple expression sequences attached in tandem. Hence, one regulatory element can enhance the expression of one or more expression sequences. Multiple regulatory elements are well-known to persons of ordinary skill in the art.

In some cases, the polyribonucleotide comprises a translation initiation sequence, e.g., a start codon. In some cases, the translation initiation sequence is a Kozak or Shine-Dalgarno sequence.

In some cases, the polyribonucleotide initiates at a codon which is not the first start codon, e.g., AUG. Translation of the polyribonucleotide can initiate at an alternative translation initiation sequence, such as, but not limited to, ACG, AGG, AAG, CTG/CUG, GTG/GUG, ATA/AUA, ATT/AUU, TTG/UUG. In some cases, translation is initiated by eukaryotic initiation factor 4A (eIF4ATranslation may be initiated from an internal ribosome entry site (IRES) element of the polyribonucleotide. An IRES element can comprise an RNA sequence capable of engaging a eukaryotic ribosome. In some cases, the IRES element is at least about 5 nt, at least about 8 nt, at least about 9 nt, at least about 10 nt, at least about 15 nt, at least about 20 nt, at least about 25 nt, at least about 30 nt, at least about 40 nt, at least about 50 nt, at least about 100 nt, at least about 200 nt, at least about 250 nt, at least about 350 nt, or at least about 500 nt. The IRES element may be derived from the DNA of an organism including, but not limited to, a virus, a mammal, and a Drosophila. Such viral DNA may be derived from, but is not limited to, picornavirus complementary DNA (cDNA), with encephalomyocarditis virus (EMCV) cDNA and poliovirus cDNA. Drosophila DNA from which an IRES element is derived includes, but is not limited to, an Antennapedia gene from Drosophila melanogaster.

In some cases, the polyribonucleotide comprises one or more expression sequences and each expression sequence can or cannot have a termination element. In some cases, the polyribonucleotide comprises one or more expression sequences and the expression sequences lack a termination element. The polyribonucleotide may be a circular polyribonucleotide that lacks a termination element such that the circular polyribonucleotide is continuously translated. In some cases, the polyribonucleotide includes a termination element at the end of one or more expression sequences. In some cases, one or more expression sequences lacks a termination element. Generally, termination elements comprise an in-frame nucleotide triplet that signals termination of translation, e.g., UAA, UGA, UAG.

The polyribonucleotide can comprise a regulatory nucleic acid, e.g., that modifies expression of an endogenous gene and/or an exogenous gene. In some cases, the polyribonucleotide comprises one or more expression sequences that encode (e.g., are complementary to) a regulatory nucleic acid. A regulatory nucleic acid can include, but is not limited to, a non-coding RNA, such as, but not limited to, tRNA, IncRNA, miRNA, rRNA, snRNA, microRNA, siRNA, piRNA, snoRNA, snRNA, exRNA, scaRNA, Y RNA, and hnRNA.

In one example, the regulatory nucleic acid targets a host gene. The regulatory nucleic acid can include, but is not limited to, a nucleic acid that hybridizes to an endogenous gene (e.g., miRNA, siRNA, mRNA, IncRNA, RNA, DNA, an antisense RNA, gRNA as described herein elsewhere), a nucleic acid that hybridizes to an exogenous nucleic acid such as a viral DNA or RNA, nucleic acid that hybridizes to an RNA, a nucleic acid that interferes with gene transcription, a nucleic acid that interferes with RNA translation, a nucleic acid that stabilizes RNA or destabilizes RNA such as through targeting for degradation, and a nucleic acid that modulates a DNA or RNA binding factor. The sequence may be an miRNA.

The polyribonucleotide can encode a regulatory nucleic acid substantially complementary, or fully complementary, to all or a fragment of an endogenous gene or gene product (e.g., mRNA). The regulatory nucleic acid can be complementary to sequences at the boundary between introns and exons, in between exons, or adjacent to exon, to prevent the maturation of newly-generated nuclear RNA transcripts of specific genes into mRNA for transcription. A regulatory nucleic acid that is complementary to a specific gene can hybridize with the mRNA for that gene and prevent its translation. The antisense regulatory nucleic acid can be DNA, RNA, or a derivative or hybrid thereof. In some cases, the regulatory nucleic acid comprises a protein-binding site that binds to a protein that participates in regulation of expression of an endogenous gene or an exogenous gene.

In some cases, the translation efficiency of a circular polyribonucleotide as provided herein is greater than a reference, e.g., a linear counterpart, a linear expression sequence, or a linear circular polyribonucleotide. In some cases, a circular polyribonucleotide as provided herein has the translation efficiency that is at least about 5%, 10%, 15%, 20%, 25%, 30%, 35%, 40%, 45%, 50%, 55%, 60%, 65%, 70%, 75%, 80%, 85%, 90%, 95%, 100%, 125%, 150%, 175%, 200%, 250%, 300%, 350%, 400%, 450%, 500%, 600%, 70%, 800%, 900%, 1000%, 2000%, 5000%, 10000%, 100000%, or more greater than that of a reference. In some cases, a circular polyribonucleotide has a translation efficiency 10% greater than that of a linear counterpart. In some cases, a circular polyribonucleotide has a translation efficiency 300% greater than that of a linear counterpart.

In some cases, the circular polyribonucleotide produces stoichiometric ratios of expression products. Rolling circle translation continuously produces expression products at substantially equivalent ratios. In some cases, the circular polyribonucleotide has a stoichiometric translation efficiency, such that expression products are produced at substantially equivalent ratios. In some cases, the circular polyribonucleotide has a stoichiometric translation efficiency of multiple expression products, e.g., products from 2, 3, 4, 5, 6, 7, 8, 9, 10, 11, 12, or more expression sequences.

In some cases, once translation of the circular polyribonucleotide is initiated, the ribosome bound to the circular polyribonucleotide does not disengage from the circular polyribonucleotide before finishing at least one round of translation of the circular polyribonucleotide. In some cases, the circular polyribonucleotide as described herein is competent for rolling circle translation. In some cases, during rolling circle translation, once translation of the circular polyribonucleotide is initiated, the ribosome bound to the circular polyribonucleotide does not disengage from the circular polyribonucleotide before finishing at least 2 rounds, at least 3 rounds, at least 4 rounds, at least 5 rounds, at least 6 rounds, at least 7 rounds, at least 8 rounds, at least 9 rounds, at least 10 rounds, at least 11 rounds, at least 12 rounds, at least 13 rounds, at least 14 rounds, at least 15 rounds, at least 20 rounds, at least 30 rounds, at least 40 rounds, at least 50 rounds, at least 60 rounds, at least 70 rounds, at least 80 rounds, at least 90 rounds, at least 100 rounds, at least 150 rounds, at least 200 rounds, at least 250 rounds, at least 500 rounds, at least 1000 rounds, at least 1500 rounds, at least 2000 rounds, at least 5000 rounds, at least 10000 rounds, at least 10⁵ rounds, or at least 10⁶ rounds of translation of the circular polyribonucleotide.

In some cases, the rolling circle translation of the circular polyribonucleotide leads to generation of polypeptide product that is translated from more than one round of translation of the circular polyribonucleotide ("continuous" expression product). In some cases, the circular polyribonucleotide comprises a stagger element (e.g., an element that causes a ribosomal pause during translation), and rolling circle translation of the circular polyribonucleotide leads to generation of polypeptide product that is generated from a single round of translation or less than a single round of translation of the circular polyribonucleotide (allows for production of "discrete" expression products). In some cases, the circular polyribonucleotide is configured such that at least 10%, 20%, 30%, 40%, 50%, at least 60%, at least 70%, at least 80%, at least 90%, at least 95%, at least 96%, at least 97%, at least 98%, at least 99%, or 100% of total polypeptides (molar/molar) generated during the rolling circle translation of the circular polyribonucleotide are discrete polypeptides. In some cases, the amount ratio of the discrete products over the total polypeptides is tested in an *in vitro* translation system. In some cases, the *in vitro* translation system used for the test of amount ratio comprises rabbit reticulocyte lysate. In some cases, the amount ratio is tested in an *in vivo* translation system, such as a eukaryotic cell or a prokaryotic cell, a cultured cell or a cell in an organism.

In some cases, the polyribonucleotide comprises untranslated regions (UTRs). UTRs of a genomic region comprising a gene may be transcribed but not translated. In some cases, a UTR may be upstream of a translation initiation sequence of an expression sequence described herein. In some cases, a UTR may be included downstream of an expression sequence described herein. In some instances, one UTR for first expression sequence is the same as, continuous with, or overlapping with another UTR for a second expression sequence. In some cases, the intron is a human intron. In some cases, the intron is a full length human intron, e.g., ZKSCAN1.

In some cases, the polyribonucleotide comprises a UTR with one or more stretches of Adenosines and Uridines embedded within. These AU rich signatures may increase turnover rates of the expression product.

Introduction, removal, or modification of UTR AU rich elements (AREs) may be useful to modulate the stability or immunogenicity of a polyribonucleotide. When engineering specific polyribonucleotides, one or more copies of an ARE may be introduced to the polyribonucleotide and the copies of an ARE may modulate translation and/or production of an expression product. Likewise, AREs may be identified and removed or engineered into the polyribonucleotide to modulate the intracellular stability and thus affect translation and production of the resultant protein.

It should be understood that any UTR from any gene may be incorporated into the respective flanking regions of the polyribonucleotide. As a non-limiting example, the UTR or a fragment thereof which may be incorporated is a UTR listed in US Provisional Application Nos. US 61/775,509 and US 61/829,372, or in International Patent Application No. PCT/US2014/021522. Furthermore, multiple wild-type UTRs of any known gene may be utilized. Artificial UTRs which are not variants of wild type genes may also be utilized. These UTRs or portions thereof may be placed in the same orientation as in the transcript from which they were selected or may be altered in orientation or location. Hence a 5' or 3' UTR may be inverted, shortened, lengthened, made chimeric with one or more other 5' UTRs or 3' UTRs. As used herein, the term "altered" as it relates to a UTR sequence, means that the UTR has been changed in some way in relation to a reference sequence. For example, a 3' or 5' UTR may be altered relative to a wild type or native UTR by the change in orientation or location as taught above or may be altered by the inclusion of additional nucleotides, deletion of nucleotides, swapping or transposition of nucleotides. Any of these changes producing an "altered" UTR (whether 3' or 5') comprise a variant UTR.

A double, triple, or quadruple UTR, such as a 5' or 3' UTR, may be used. As used herein, a "double" UTR is one in which two copies of the same UTR are encoded either in series or substantially in series. For example, a double beta- globin 3' UTR may be used as described in US Patent publication 20100129877.

In some cases, the polyribonucleotide may include a poly-A sequence. In some cases, the length of a poly-A sequence is greater than 10 nucleotides in length. The poly-A sequence may be greater than 15 nucleotides in length (e.g., at least or greater than about 10, 15, 20, 25, 30, 35, 40, 45, 50, 55, 60, 70, 80, 90, 100, 120, 140, 160, 180, 200, 250, 300, 350, 400, 450, 500, 600, 700, 800, 900, 1,000, 1,100, 1,200, 1,300, 1,400, 1,500, 1,600, 1,700, 1,800, 1,900, 2,000, 2,500, and 3,000 nucleotides). In some cases, the poly-A sequence is from about 10 to about 3,000 nucleotides (e.g., from 30 to 50, from 30 to 100, from 30 to 250, from 30 to 500, from 30 to 750, from 30 to 1,000, from 30 to 1,500, from 30 to 2,000, from 30 to 2,500, from 50 to 100, from 50 to 250, from 50 to 500, from 50 to 750, from 50 to 1,000, from 50 to 1,500, from 50 to 2,000, from 50 to 2,500, from 50 to 3,000, from 100 to 500, from 100 to 750, from 100 to 1,000, from 100 to 1,500, from 100 to 2,000, from 100 to 2,500, from 100 to 3,000, from 500 to 750, from 500 to 1,000, from 500 to 1,500, from 500 to 2,000, from 500 to 2,500, from 500 to 3,000, from 1,000 to 1,500, from 1,000 to 2,000, from 1,000 to 2,500, from 1,000 to 3,000, from 1,500 to 2,000, from 1,500 to 2,500, from 1,500 to 3,000, from 2,000 to 3,000, from 2,000 to 2,500, and from 2,500 to 3,000).

The poly-A sequence may be designed relative to the length of the overall polyribonucleotide. This design may be based on the length of the coding region, the length of a particular feature or region (such as the first or flanking regions), or based on the length of the ultimate product expressed from the polyribonucleotide. In this context, the poly-A sequence may be 10, 20, 30, 40, 50, 60, 70, 80, 90, or 100% greater in length than the circular polyribonucleotide or a feature thereof. The poly-A sequence may also be designed as a fraction of polyribonucleotide to which it belongs. In this context, the poly-A sequence may be 10, 20, 30, 40, 50, 60, 70, 80, or 90% or more of the total length of the construct or the total length of the construct minus the poly-A sequence. Further, engineered binding sites and conjugation of circular polyribonucleotide for Poly-A binding protein may enhance expression.

The polyribonucleotide may be designed to include a polyA-G quartet. The G-quartet can be a cyclic hydrogen bonded array of four guanine nucleotides that can be formed by G-rich sequences in both DNA and RNA. The G-quartet may be incorporated at the end of the poly-A sequence. In some cases, the polyA-G quartet results in protein production equivalent to at least 75% of that seen using a poly-A sequence of 120 nucleotides alone.

In some cases, the polyribonucleotide comprises a polyA, lacks a polyA, or has a modified polyA to modulate one or more characteristics of the polyribonucleotide. In some cases, the polyribonucleotide lacking a polyA or having modified polyA improves one or more functional characteristics, e.g., immunogenicity, half-life, expression efficiency, etc.

### Encryptogen

As described herein, a polyribonucleotide can comprise an encryptogen to reduce, evade, or avoid the innate immune response of a cell. The polyribonucleotide is present in circular form. Circular polyribonucleotides provided herein may result in a reduced immune response from the host as compared to the response triggered by a reference compound, e.g., a circular polyribonucleotide producing a reduced immune response compared to a corresponding linear polynucleotide or a linear polynucleotide comprising an encryptogen, producing a reduced immune response compared to the corresponding linear polyribonucleotide lacking an encryptogen. The polyribonucleotide has less immunogenicity than a counterpart lacking an encryptogen.

The polyribonucleotide may be non-immunogenic in a mammal, e.g., a human. The polyribonucleotide may be capable of replicating in a mammalian cell, e.g., a human cell.

The polyribonucleotide may include sequences or expression products.

The polyribonucleotide comprising the encryptogen may have a half-life of at least that of a counterpart lacking the encryptogen. The polyribonucleotide may have a half-life that is increased over that of a counterpart. The half-life may be increased by about 5%, 10%, 15%, 20%, 25%, 30%, 35%, 40%, 45%, 50%, or greater. The polyribonucleotide may have has a half-life or persistence in a cell for at least about 1 hr to about 30 days, or at least about 2 hrs, 6 hrs, 12 hrs, 18 hrs, 24 hrs, 2 days, 3, days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, 60 days, or longer or any time there between. The polyribonucleotide may have has a half-life or persistence in a cell for no more than about 10 mins to about 7 days, or no more than about 1 hr, 2 hrs, 3 hrs, 4 hrs, 5 hrs, 6 hrs, 7 hrs, 8 hrs, 9 hrs, 10 hrs, 11 hrs, 12 hrs, 13 hrs, 14 hrs, 15 hrs, 16 hrs, 17 hrs, 18 hrs, 19 hrs, 20 hrs, 21 hrs, 22 hrs, 24 hrs, 36 hrs, 48 hrs, 60 hrs, 72 hrs, 4 days, 5 days, 6 days, 7 days, or any time there between.

In The polyribonucleotide comprising the encryptogen may modulate a cosmetic cellular function, e.g., transiently or long term. The cosmetic cellular function may be stably altered, such as a modulation that persists for at least about 1 hr to about 30 days, or at least about 2 hrs, 6 hrs, 12 hrs, 18 hrs, 24 hrs, 2 days, 3, days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, 60 days, or longer or any time there between. The cosmetic cellular function may be transiently altered, e.g., such as a modulation that persists for no more than about 30 mins to about 7 days, or no more than about 1 hr, 2 hrs, 3 hrs, 4 hrs, 5 hrs, 6 hrs, 7 hrs, 8 hrs, 9 hrs, 10 hrs, 11 hrs, 12 hrs, 13 hrs, 14 hrs, 15 hrs, 16 hrs, 17 hrs, 18 hrs, 19 hrs, 20 hrs, 21 hrs, 22 hrs, 24 hrs, 36 hrs, 48 hrs, 60 hrs, 72 hrs, 4 days, 5 days, 6 days, 7 days, or any time there between.

The polyribonucleotide comprising the encryptogen may be at least about 20 base pairs, at least about 30 base pairs, at least about 40 base pairs, at least about 50 base pairs, at least about 75 base pairs, at least about 100 base pairs, at least about 200 base pairs, at least about 300 base pairs, at least about 400 base pairs, at least about 500 base pairs, or at least about 1,000 base pairs. The polyribonucleotide comprising the encryptogen can be of a sufficient size to accommodate a binding site for a ribosome. One of skill in the art can appreciate that the maximum size of a polyribonucleotide comprising the encryptogen can be as large as is within the technical constraints of producing a polyribonucleotide, and/or using the polyribonucleotide.

The circular polyribonucleotide comprising the encryptogen may have a half-life of at least that of a linear counterpart, e.g., linear expression sequence, or linear circular polyribonucleotide. The circular polyribonucleotide may have a half-life that is increased over that of a linear counterpart. The half-life may be increased by about 5%, 10%, 15%, 20%, 25%, 30%, 35%, 40%, 45%, 50%, or greater. The circular polyribonucleotide may have a half-life or persistence in a cell for at least about 1 hr to about 30 days, or at least about 2 hrs, 6 hrs, 12 hrs, 18 hrs, 24 hrs, 2 days, 3, days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, 60 days, or longer or any time there between. The circular polyribonucleotide may have a half-life or persistence in a cell for no more than about 10 mins to about 7 days, or no more than about 1 hr, 2 hrs, 3 hrs, 4 hrs, 5 hrs, 6 hrs, 7 hrs, 8 hrs, 9 hrs, 10 hrs, 11 hrs, 12 hrs, 13 hrs, 14 hrs, 15 hrs, 16 hrs, 17 hrs, 18 hrs, 19 hrs, 20 hrs, 21 hrs, 22 hrs, 24 hrs, 36 hrs, 48 hrs, 60 hrs, 72 hrs, 4 days, 5 days, 6 days, 7 days, or any time there between.

The circular polyribonucleotide comprising the encryptogen may modulate a cosmetic cellular function, e.g., transiently or long term. The cosmetic cellular function may be stably altered, such as a modulation that persists for at least about 1 hr to about 30 days, or at least about 2 hrs, 6 hrs, 12 hrs, 18 hrs, 24 hrs, 2 days, 3, days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, 60 days, or longer or any time there between. The cosmetic cellular function may be transiently altered, e.g., such as a modulation that persists for no more than about 30 mins to about 7 days, or no more than about 1 hr, 2 hrs, 3 hrs, 4 hrs, 5 hrs, 6 hrs, 7 hrs, 8 hrs, 9 hrs, 10 hrs, 11 hrs, 12 hrs, 13 hrs, 14 hrs, 15 hrs, 16 hrs, 17 hrs, 18 hrs, 19 hrs, 20 hrs, 21 hrs, 22 hrs, 24 hrs, 36 hrs, 48 hrs, 60 hrs, 72 hrs, 4 days, 5 days, 6 days, 7 days, or any time there between.

The circular polyribonucleotide comprising the encryptogen may be at least about 20 base pairs, at least about 30 base pairs, at least about 40 base pairs, at least about 50 base pairs, at least about 75 base pairs, at least about 100 base pairs, at least about 200 base pairs, at least about 300 base pairs, at least about 400 base pairs, at least about 500 base pairs, or at least about 1,000 base pairs. The circular polyribonucleotide can be of a sufficient size to accommodate a binding site for a ribosome. One of skill in the art can appreciate that the maximum size of a circular polyribonucleotide can be as large as is within the technical constraints of producing a circular polyribonucleotide, and/or using the circular polyribonucleotide. While not being bound by theory, it is possible that multiple segments of RNA can be produced from DNA and their 5' and 3' free ends annealed to produce a "string" of RNA, which ultimately can be circularized when only one 5' and one 3' free end remains. The maximum size of a circular polyribonucleotide can be limited by the ability of packaging and delivering the RNA to a target. The size of a circular polyribonucleotide may be a length sufficient to encode useful polypeptides, and thus, lengths of less than about 20,000 base pairs, less than about 15,000 base pairs, less than about 10,000 base pairs, less than about 7,500 base pairs, or less than about 5,000 base pairs, less than about 4,000 base pairs, less than about 3,000 base pairs, less than about 2,000 base pairs, less than about 1,000 base pairs, less than about 500 base pairs, less than about 400 base pairs, less than about 300 base pairs, less than about 200 base pairs, less than about 100 base pairs can be useful.

### Cleavage sequences

The polyribonucleotide may comprise at least one cleavage sequence. The polyribonucleotide is present in circular form. The cleavage sequence may be adjacent to an expression sequence. The polyribonucleotide may comprise a cleavage sequence, such as in an immolating polyribonucleotide, a cleavable polyribonucleotide, or a self-cleaving polyribonucleotide. The polyribonucleotide may comprise two or more cleavage sequences, leading to separation of the polyribonucleotide into multiple products, e.g., miRNAs, linear RNAs, smaller circular polyribonucleotide, etc.

### Other Reagents

In some cases, the composition or cosmetic composition comprises reagents besides the alcohol or the cell-penetrating agent and the polyribonucleotide. For example, the composition or cosmetic composition can further comprise one or more active agents, e.g., cosmetic agents, besides the polyribonucleotide. In some cases, the composition comprising one or more active agents, e.g., cosmetic agents, can be formulated using one or more cosmetically acceptable carriers, comprising excipients, diluents, and/or auxiliaries, e.g., which facilitate processing of the one or more active agents into preparations that can be administered. The polyribonucleotide can be present in either linear or circular form.

In some cases, the cosmetic composition further comprises a cosmetic agent that causes a change in the appearance of an integumentary member of subject as described herein. A cosmetic agent can include, but not limited to, Glycerin, Ricinus Communis (Castor) Seed Oil, Cetearyl Olivate, Lecithin, Caprylic/Capric Triglyceride, Sorbitan Olivate, Cetearyl Alcohol, Dimethicone, Ergothioneine, Isosorbide Dicaprylate, Glyceryl Behenate, Ethyl Linoleate, Ethylhexyl Palmitate, Silica Dimethyl Silylate, Butylene Glycol, Sodium Hyaluronate, Magnolia Officinalis Bark Extract, Ectoin, Tetrahexyldecyl Ascorbate, Panthenol, Hydrogenated Castor Oil, Cera Alba, Copernicia Cerifera (Carnauba) Wax, Sodium PCA, Urea, Trehalose, Triacetin, Polyquatemium-51 , Methylglucoside Phosphate, Copper Lysinate/Prolinate, Ceteareth-20, Magnesium Aspartate, Zinc Gluconate, Copper Gluconate, C8-22 Alkyl Acrylates/Methacrylic Acid Crosspolymer, Persea Gratissima (Avocado) Oil, Squalane, Oenothera Biennis (Evening Primrose) Oil, Fragrance, Allantoin, Stearyl Glycyrrhetinate, Maltodextrin, Tocopherol, Vitis Vinifera (Grape) Seed Extract, Xanthan Gum, Arginine, Aloe Barbadensis Leaf Juice, Macadamia Ternifolia Seed Oil, Phenoxyethanol, Ethylhexylglycerin, Sodium Phytate, and Tricholoma Matsutake Extract, cyclopentasiloxane, CI 2- 15 Alkyl Benzoate, Titanium Dioxide, Zinc Oxide, Dimethicone, Polyglyceryl-3 Polydimethylsiloxyethyl Dimethicone, Dimethicone/PEG-10/15 Crosspolymer, Stearic Acid, Aluminum Hydroxide, Sodium Chloride, Glycerin, Magnolia Officinalis Bark Extract, Ectoin, Caprylyl Glycol, Teprenone, Sodium Hyaluronate, Vitis Vinifera (Grape) Seed Extract, Tetrahexyldecyl Ascorbate, Ergothioneine, Tocopheryl Acetate, Tocopherol, Maltodextrin, Lecithin, Ethylhexyl Palmitate, Caprylic/Capric Triglyceride, PEG/PPG-18/18 Dimethicone, Polyglycerin-3 Crosspolymer, Hexyl Laurate, Cyclohexasiloxane, Triethoxysilylethyl Polydimethylsiloxyethyl Hexyl Dimethicone, Dimethicone /Vinyl Dimethicone Crosspolymer, Polyglyceryl-4 Isostearate, Cetyl PEG/PPG-10/1 Dimethicone, Silica Dimethyl Silylate, Triethoxycaprylylsilane, Phenoxyethanol, Ethylhexylglycerin, Butylene Glycol, Hexylene Glycol, Fragrance, Mica and Silica, Potassium Azeloyl Diglycinate, Hamamelis Virginiana (Witch Hazel) Extract, Glycerin, Methyl Gluceth-20, Magnolia Officinalis Bark Extract, Ergothioneine, Ectoin, Pentylene Glycol, Salix Nigra (Willow) Bark Extract, Magnesium Aspartate, Zinc Gluconate, Copper Gluconate, Microcitrus Austraiasica Fruit Extract, Mucor Miehei Extract, Glucosamine HC1, Urea, Phenoxyethanol, Ethylhexylglycerin, Polysorbate 20, Magnolia Glauca Flower Water, Sodium PCA, Potassium Sorbate, Allantoin, Dipotassium Glycyrrhizate, Alcohol Denatured, Disodium EDTA, Lactic Acid, Maltodextrin, Lecithin, Tocopherol, Vitis Vinifera (Grape) Seed Extract, Sodium Hydroxide, Caprylic/Capric Triglyceride, Jasminum Officinale (Jasmine) Extract, Rosa Damascena Flower Oil, Pelargonium Graveolens Oil, Citrus Medica Limonum (Lemon) Peel Oil, Camellia Sinensis Leaf (Green Tea) Extract, Mentha Viridis (Spearmint) Leaf Oil, and Juniperus Virginiana Oil.

In some cases, the cosmetic agent comprises, but not limited to, Pentapeptide-18, Acetyl Hexapeptide-8, Caprylyl Methicone, Glycerin, Butylene Glycol, Beta Glucan (Oat), Isosorbide Dicaprylate, Dimethicone, Isostearyl Alcohol, Butylene Glycol Cocoate, Polyacrylate-13, Magnolia Officinalis Bark Extract, Ergothioneine, Ectoin, Palmitoyl Tetrapeptide-7, Acetyl Tetrapeptide-5, Tetrahexyldecyl Ascorbate, Tyrosine, Hesperidin Methyl Chalcone, Tricholoma Matsutake Extract, Corallina Officinalis Extract, Sodium Hyaluronate, Sodium Potassium Aluminum Silicate, Silica, Pullulan, Steareth-20, Dipeptide-2, Ethylcellulose, Polysorbate 20, Polyisobutene, Dimethiconol, Panthenol, Oryza Sativa (Rice) Bran Oil, Lecithin, Allantoin, Xanthan Gum, Elaeis Guineensis Oil, Tocotrienols, Tocopherol, Phenoxyethanol, Maltodextrin, Vitis Vinifera (Grape) Seed Extract, Sodium Phytate, Fragrance, Ethylhexylglycerin, Mica, and Titanium Dioxide.

A polypeptide as described herein may be formulated in a cosmetic formulation. A cosmetic formulation can comprise one or more of: silicones, aerosols, anti-oxidants, cleansing agents, colorants, additional conditioning agents, deposition agents, electrolytes, emollients and oils, exfoliating agents, foam boosters, fragrances, humectants, occlusive agents, pediculicides, pH control agents, pigments, preservatives, biocides, other solvents, stabilizers, sunscreening agents, suspending agents, tanning agents, other surfactants, thickeners, vitamins, botanicals, waxes, rheology-modifying agents, anti-dandruff, anti-acne, anti-carie and wound healing-promotion agents. The cosmetic formulation can comprise abrasives, anti-acne agents, agents against ageing of the skin, anti-cellulite agents, antidandruff agents, antiinflammatory agents, irritation-preventing agents, irritation-inhibiting agents, antioxidants, astringents, perspiration-inhibiting agents, antiseptic agents, anti-statics, binders, buffers, carrier materials, chelating agents, cell stimulants, cleansing agents, care agents, depilatory agents, surface-active substances, deodorizing agents, antiperspirants, softeners, emulsifiers, enzymes, essential oils, fibres, film-forming agents, fixatives, foam-forming agents, foam stabilizers, substances for preventing foaming, foam boosters, gelling agents, gel-forming agents, hair care agents, hair-setting agents, hair-straightening agents, moisture-donating agents, moisturizing substances, moisture-retaining substances, bleaching agents, strengthening agents, stain-removing agents, optically brightening agents, impregnating agents, dirt-repellent agents, friction-reducing agents, lubricants, moisturizing creams, ointments, opacifying agents, plasticizing agents, covering agents, polish, gloss agents, polymers, powders, proteins, re-oiling agents, abrading agents, silicones, skin-soothing agents, skin-cleansing agents, skin care agents, skin-healing agents, skin-lightening agents, skin-protecting agents, skin-softening agents, hair promotion agents, cooling agents, skin-cooling agents, warming agents, skin-warming agents, stabilizers, UV-absorbing agents, UV filters, detergents, fabric conditioning agents, suspending agents, skin-tanning agents, thickeners, vitamins, oils, waxes, fats, phospholipids, saturated fatty acids, mono- or polyunsaturated fatty acids, a-hydroxy acids, polyhydroxyfatty acids, liquefiers, dyestuffs, colour-protecting agents, pigments, anti-corrosives, aromas, flavouring substances, odoriferous substances, polyols, surfactants, electrolytes, organic solvents or silicone derivatives and the like as additional auxiliaries and additives.

In some cases, the composition or cosmetic composition described herein can further comprise a cosmetically acceptable excipient such as, but not limited to, a solvent, aqueous solvent, non-aqueous solvent, dispersion media, diluent, dispersion, suspension aid, surface active agent, isotonic agent, thickening or emulsifying agent, preservative, lipid, lipidoids liposome, lipid nanoparticle, core-shell nanoparticles, polymer, lipoplex, peptide, protein, cell, hyaluronidase, and mixtures thereof. The compositions or cosmetic compositions can further comprise other reagents to maintain appropriate physical or chemical properties, such as, but not limited to, salt concentration, osmolality, pH, hydrophobicity/hydrophility, and solubility. For example, the composition comprises a stabilizer, e.g., glucose at an appropriate concentration, e.g., about 4.5 g/L. The polyribonucleotide may be non-immunogenic. The polyribonucleotide may be immunogenic. A composition or cosmetic composition can further comprise appropriate adjuvant(s) or other agents that can enhance or depress the immunogenicity of the polyribonucleotide. An adjuvant can be a hydrophilic and lipophilic gelling agent, hydrophilic and lipophilic active agent, preserving agent, antioxidant, solvent, fragrance, filler, screening agent, pigment, odor absorber, dyestuff, or any combination thereof.

Non-limiting examples of the cosmetically acceptable carriers include starch, glucose, lactose, sucrose, gelatin, saline, gum acacia, keratin, urea, malt, rice flour, chalk, silica gel, sodium stearate, glycerol monostearate, talc, sodium chloride, dried skim milk, glycerol, propylene, glycol, humectants (e.g., urea), glycols (e.g., propylene glycol), fatty acids (e.g., oleic acid), surfactants (e.g., isopropyl myristate and sodium lauryl sulfate), pyrrolidones, glycerol monolaurate, sulfoxides, terpenes (e.g., menthol), amines, amides, alkanes, alkanols, water, calcium carbonate, calcium phosphate, various sugars, starches, cellulose derivatives, gelatin, polymers such as polyethylene glycols, and water. If desired, the carrier can also contain minor amounts of wetting or emulsifying agents, or pH buffering agents.

### Stability

The polyribonucleotide provided herein can be stable after delivery into a cell or tissue, allowing for translation of the polyribonucleotide resulting in a cosmetic effect on the cell or tissue in which the polyribonucleotide was delivered. The polyribonucleotide is present in circular form. For reference, if the polyribonucleotide is a linear polyribonucleotide, it may be used for a short term cosmetic effect, such as for a cosmetic effect that lasts at least 1 hour, 6 hours, 12 hours, 24 hours, 48 hours, 72 hours, or any time therebetween. The circular polyribonucleotid may be used for a long term cosmetic effect, such as a cosmetic effect that last for at least 3 days, 4 days, 5 days, 6 days, 7 days, 8 days, 9 days, 10 days, 11 days, 12 days, 13 days, 14 days, or more, or any time therebetween. The polyribonucleotide may be substantially resistant to degradation, e.g., exonuclease. The polyribonucleotide may be resistant to self-degradation. The polyribonucleotide may lack an enzymatic cleavage site, e.g., a dicer cleavage site.

The polyribonucleotide may persist in a cell during cell division. The polyribonucleotide may persist in daughter cells after mitosis. The polyribonucleotide may be replicated within a cell and passed to daughter cells. The polyribonucleotide may comprise a replication element that mediates self-replication of the polyribonucleotide. The replication element may mediate transcription of the circular polyribonucleotide into a linear polyribonucleotide that is complementary to the circular polyribonucleotide (linear complementary). The linear complementary polyribonucleotide can be circularized *in vivo* in cells into a complementary circular polyribonucleotide. The complementary polyribonucleotide can further self-replicate into another circular polyribonucleotide, which has the same or similar nucleotide sequence as the starting circular polyribonucleotide. One exemplary self-replication element includes HDV replication domain (as described by Beeharry et al, *Virol*, 2014, 450-451:165-173). A cell may pass at least one polyribonucleotide to daughter cells with an efficiency of at least 25%, 50%, 60%, 70%, 80%, 85%, 90%, 95%, or 99%.A cell undergoing meiosis may pass the polyribonucleotide to daughter cells with an efficiency of at least 25%, 50%, 60%, 70%, 80%, 85%, 90%, 95%, or 99%.

### DELIVERY OF COSMETIC COMPOSITIONS

A cosmetic composition provided herein can be formulated based, in part, on the intended route of administration of the composition. A composition or cosmetic composition provided herein can be administered topically at or near particular sites of a subject. The composition can be as described herein and further comprise a cosmetically acceptable excipient. Direct topical application, e.g., of a viscous liquid, solution, suspension, gel, jelly, cream, lotion, ointment, foam, serum, or aerosol, can be used for local administration. The polyribonucleotide is present in circular form.

The composition or cosmetic composition can comprise an alcohol (e.g., ethanol) and the polyribonucleotide. The composition or cosmetic composition can comprise a cell-penetrating agent and the polyribonucleotide. The composition or cosmetic composition can comprise a diluent and the polyribonucleotide, wherein the composition or cosmetic composition is free of any carrier.

The cosmetic compositions provided herein can be used to alter, modify or change the appearance of an integumentary member of a subject, such as, but not limited to, improving skin appearance, enhancing skin rejuvenation, increasing moisturization and/or elasticity, preventing or reducing wrinkles, lightening or darking skin or hair, changing hair color or density, and/or providing other anti-aging care. The methods provided herein can reduce the visible signs of skin aging, including: exfoliating the skin with a composition described herein; treating the skin with a composition described herein; and/or moisturizing the skin with a composition described herein. The methods provided herein can bleach the skin. The methods provided herein can treat or care for skin (e.g., scalp, facial skin), nails, keratin fibers (e.g., hair or eyelashes), or any combination thereof.

A cosmetic composition provided herein can be formulated to be administered directly onto a surface area of a subject, such as, but not limited to, forehead, scalp, hair follicles, hair, upper eyelids, lower eyelids, eyebrows, eyelashes, infraorbital area, periorbital areas, temple, nose, nose bridge, cheeks, tongue, nasolabial folds, lips, periocular areas, jaw line, ears, neck, breast, forearm, upper arm, palm, hand, finger, nails, back, abdomen, sides, buttocks, thigh, calf, feet, toes, gums, tongue, and the like.

A cosmetic composition described herein can be a liquid preparation such as a suspension, syrup, or elixir. In some cases, an aqueous solution is packaged for use as is, or lyophilized, and the lyophilized preparation being combined with a sterile solution prior to administration. The cosmetic composition can be delivered as a solution or as a suspension. In general, formulations such as jellies, creams, lotions, and ointments can provide an area with more extended exposure to one or more active agents, while formulations in solution, e.g., sprays, can provide more immediate, short-term exposure.

A composition or cosmetic composition as described herein can have a pH of about 7. In some embodiments, the composition or cosmetic composition has a viscosity about the same as water. The composition or cosmetic composition can be substantially free of hydrophobic or lipophilic groups. In some embodiments, the composition or cosmetic compositions is substantially free of hydrocarbons. The composition or cosmetic composition can be substantially free of fatty acids, lipids, liposomes, cholesterol, or any combination thereof. The composition or cosmetic composition can be free of glycerin, propylene glycol, or a combination thereof.

In some embodiments, a method of delivery of the circular polyribonucleotides (e.g., a cosmetic circular polyribonucleotide) as described herein comprises applying a cosmetic composition comprising a mixture of a cosmetic circular polyribonucleotide and ethanol to an integumentary member of a subject, wherein the ethanol constitutes 0.3% v/v to 75% v/v of the mixture. In some embodiments, a method of delivery of the cosmetic circular polyribonucleotides as described herein comprises applying a cosmetic composition comprising a mixture of a cosmetic circular polyribonucleotide and alcohol to an integumentary member of a subject, wherein the alcohol constitutes 0.3% v/v to 75% v/v of the mixture. In some embodiments, a method of delivery of the cosmetic circular polyribonucleotides as described herein comprises applying a cosmetic composition comprising a mixture of a cosmetic circular polyribonucleotide and a cell-penetrating agent to an integumentary member of a subject, wherein the cell penetrating agent constitutes 0.3% v/v to 75% v/v of the mixture. In some embodiments, the ethanol, alcohol, or cell-penetrating agent constitutes 0.3% v/v to 70% v/v, 0.3% v/v to 60% v/v, 0.3% v/v to 50% v/v, 0.3% v/v to 40% v/v, 30% v/v to 20% v/v, 0.3% v/v to 15% v/v, 0.3% v/v to 10% v/v, 0.3% v/v to 5% v/v, 0.3% v/v to 1% v/v, or 0.3% v/v to 0.5% v/v of the mixture, or any percentage v/v therebetween. In some embodiments, the ethanol, alcohol, or cell-penetrating agent 0.5% v/v to 75% v/v, 1% v/v to 75% v/v, 5% v/v to 75% v/v, 10% v/v to 75% v/v, 15% v/v to 75% v/v, 20% v/v to 75% v/v, 30% v/v to 75% v/v, 40% v/v to 75% v/v, 50% v/v to 75% v/v, 60% v/v to 75% v/v, or 70% v/v to 75% v/v of the mixture, or any percentage v/v therebetween.

Often, the composition or the cosmetic composition as described herein delivers the polyribonucleotide to a dermal or epidermal tissue of a subject. In some embodiments, the polyribonucleotides are delivered without iontophoresis.

### Percutaneous Administration

Formulations for topical administration can take the form of a liquid, a semisolid dosage form (e.g., a paste, a cream, a lotion, a powder, an ointment or a gel), a patch, a film, or a spray. In some cases, the topical composition can be a cream or gel that can be applied to an affected area of the skin of a subject in need thereof (e.g., percutaneous or dermal administration). Different release profiles can be achieved with different forms, such as but not limited to controlled release, delayed release, extended release, or sustained release. The topical cosmetic composition can be applied multiple times a day, once per day, or as often as needed. The polyribonucleotide is present in circular form.

In some cases, the cosmetic composition can be formulated for direct application on a skin area (e.g., percutaneous or dermal administration). A cosmetic composition provided herein can comprise a dermatologically acceptable diluent. Such diluents are compatible with skin, nails, mucous membranes, tissues, and/or hair, and can include any dermatological diluents meeting these requirements. Such diluents can be readily selected by one of ordinary skill in the art. A cosmetic composition provided herein can comprise a cosmetically acceptable diluent. Such diluents are compatible with skin, nails, mucous membranes, tissues, and/or hair, and can include any cosmetic diluents meeting these requirements. Such diluents can be readily selected by one of ordinary skill in the art. In formulating skin ointments, one or more agents can be formulated in an oleaginous hydrocarbon base, an anhydrous absorption base, a water-in-oil absorption base, an oil-in-water water-removable base and/or a water-soluble base.

Ointments and creams are, for example, formulated with an aqueous or oily base with the addition of suitable thickening and/or gelling agents. Lotions can be formulated with an aqueous or oily base and will in general also comprise one or more emulsifying agents, stabilizing agents, dispersing agents, suspending agents, thickening agents, or coloring agents. The construction and use of transdermal patches for the delivery of cosmetic agents is known in the art. Such patches can be constructed for continuous, pulsatile, or on demand delivery of cosmetic agents.

Lubricants which can be used to form compositions and dosage forms can comprise calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (e.g., peanut oil, cottonseed oil, sunflower oil, sesame oil, olive oil, corn oil, and soybean oil), zinc stearate, ethyl oleate, ethyl laureate, agar, or mixtures thereof. Additional lubricants include, for example, a syloid silica gel, a coagulated aerosol of synthetic silica, or mixtures thereof. A lubricant can optionally be added, in an amount of less than about 1 weight percent of the composition.

A composition or cosmetic composition provided herein can be in any form suitable for topical administration, including an emollient or emulsion, aqueous, aqueous-alcoholic or oily solutions, lotion or serum dispersions, aqueous, anhydrous or oily gels, emulsions obtained by dispersion of a fatty phase in an aqueous phase (O/W or oil in water) or, conversely, (W/O or water in oil), microemulsions or alternatively microcapsules, microparticles or lipid vesicle dispersions of ionic and/or nonionic type. The composition or cosmetic composition may be in the form of an emulsion comprising at least one oil phase. The composition or cosmetic composition may be in the form of an emulsion comprising an emulsifier, such as an amphoteric, anionic, cationic, non-ionic emulsifier, or any mixture thereof. Other than the polyribonucleotides and other active ingredient(s), the amount of the various constituents of the compositions provided herein can be those used in the art. These compositions can constitute protection, treatment or care creams, milks, lotions, gels or foams for the face, for the hands, for the body and/or for the mucous membranes, or for cleansing the skin. The compositions can also consist of solid preparations constituting soaps or cleansing bars.

Compositions or cosmetic compositions can comprise various levels of fluidity. The composition or cosmetic composition can be a white or coloured cream, a balm, a milk, a lotion, a serum, a paste, or a foam. The compositions or cosmetic compositions can be applied as an aerosol. The compositions or cosmetic compositions can be applied as a solid form, e.g., as a stick.

A composition or cosmetic composition can comprise a humectant. For example, a humectant is a urea and pyrrolidone carboxylic acid in combination with steryl, cetyl, and polowaxes.

A composition or cosmetic composition provided herein for local/topical administration can comprise one or more antimicrobial preservatives such as quaternary ammonium compounds, organic mercurials, p-hydroxy benzoates, aromatic alcohols, chlorobutanol, and the like.

### Ophthalmic administration

A composition or cosmetic composition provided herein can be administered through eyes, e.g. delivered in eye drops or ointment. The topical application of the composition to eyes can contact the cells in eyes, for instance, retina, with the mixture of the alcohol (e.g., ethanol) and the polyribonucleotide. The topical application of the composition to eyes can contact the cells in eyes, for instance, retina, with the mixture of the cell-penetrating agent and the polyribonucleotide. The polyribonucleotide is present in circular form.

Eye drops can be prepared by the mixture of the cell-penetrating agent and the polyribonucleotide alone, if the mixture is in liquid phase itself. Alternatively, eye drops are prepared by dissolving a solid mixture of the cell-penetrating agent and the polyribonucleotide in a sterile aqueous solution such as physiological saline, buffering solution, etc., or by combining powder compositions to be dissolved before use. Alternatively, eye drops are prepared by dissolving a solid mixture of the alcohol (e.g., ethanol) and the polyribonucleotide in a sterile aqueous solution such as physiological saline, buffering solution, etc., or by combining powder compositions to be dissolved before use. Other vehicles can be chosen, as is known in the art, including but not limited to: balance salt solution, saline solution, water soluble poly ethers such as polyethyene glycol, polyvinyls, such as polyvinyl alcohol and povidone, cellulose derivatives such as methylcellulose and hydroxypropyl methylcellulose, petroleum derivatives such as mineral oil and white petrolatum, animal fats such as lanolin, polymers of acrylic acid such as carboxypolymethylene gel, vegetable fats such as peanut oil and polysaccharides such as dextrans, and glycosaminoglycans such as sodium hyaluronate. If desired, additives ordinarily used in the eye drops can be added. Such additives include isotonizing agents (e.g., sodium chloride, etc.), buffer agent (e.g., boric acid, sodium monohydrogen phosphate, sodium dihydrogen phosphate, etc.), preservatives (e.g., benzalkonium chloride, benzethonium chloride, chlorobutanol, etc.), thickeners (e.g., saccharide such as lactose, mannitol, maltose, etc.; e.g., hyaluronic acid or its salt such as sodium hyaluronate, potassium hyaluronate, etc.; e.g., mucopolysaccharide such as chondroitin sulfate, etc.; e.g., sodium polyacrylate, carboxyvinyl polymer, crosslinked polyacrylate, polyvinyl alcohol, polyvinyl pyrrolidone, methyl cellulose, hydroxy propyl methylcellulose, hydroxyethyl cellulose, carboxymethyl cellulose, hydroxy propyl cellulose, or other agents known to those skilled in the art).

### Other Administration

A composition or a cosmetic composition described herein may be formulated for mucosal membranes, e.g., lips. The polyribonucleotide can be a circular RNA.

In some cases, a composition or cosmetic composition is formulated for administration to epithelial cells. This composition or cosmetic composition can be free of any carrier and comprises a diluent and the polyribonucleotide as described herein. This composition or cosmetic composition may be directly applied to epithelial cells for the delivery of the polyribonucleotide (e.g., cosmetic polyribonucleotide). The polyribonucleotide can be a circular RNA.

### Pre-treatment

A composition or cosmetic composition as described herein can be applied to a surface (e.g., an integumentary member) after application of a sterilizing agent to that surface (e.g., pre-treatment of the surface (e.g., the integumentary member) with a sterilizing agent for delivery of the polyribonucleotide). The surface can be a surface area of a subject. The surface area of the subject can comprise cells, such as epithelial cells. The surface can be an integumentary member, such as hair, nails, or skin.

A sterilizing agent can be any agent that is bactericidal, bacteriostatic, and/or actively kills microorganisms, inactivates microorganism, or prevents microorganisms from growing. The sterilizing agent may be an alcohol, iodine, or hydrogen peroxide. The sterilizing agent can be UV light or laser light. The sterilizing agent may be heat delivered electrically or through other means, such as by vapor or contact.

The sterilizing agent can be applied to the surface area (e.g., the integumentary member) of the subject by various methods. For example, a sterilizing agent can be applied by a wipe or swab comprising the sterilizing agent. The wipe or swab can be a pad, a cotton ball, or a cotton-tipped applicator. The sterilizing agent may be applied as a spray. Various devices can be used to apply the sterilizing agent. For example, a device that produces UV light or laser light can be used. In other embodiments, a device that produces heat can be used.

The composition or cosmetic composition applied to the surface area after pre-treatment can be free of any carrier and comprise the polyribonucleotide and a diluent. The polyribonucleotide is a circular polyribonucleotide.

The sterilizing agent, the cosmetic composition, or a combination thereof can be applied by massaging into the skin. The sterilizing agent may be allowed to dry before administering the cosmetic composition.

### Preservatives

A composition or cosmetic composition provided herein can comprise material for a single administration, or can comprise material for multiple administrations (e.g., a "multidose" kit). The composition or cosmetic composition can include one or more preservatives such as thiomersal or 2-phenoxyethanol. Preservatives can be used to prevent microbial contamination during use. Suitable preservatives include: benzalkonium chloride, thimerosal, chlorobutanol, methyl paraben, propyl paraben, phenylethyl alcohol, edetate disodium, sorbic acid, Onamer M, or other agents known to those skilled in the art. In ophthalmic products, e.g., such preservatives can be employed at a level of from 0.004% to 0.02%. In the compositions or cosmetic compositions described herein the preservative, e.g., benzalkonium chloride, can be employed at a level of from 0.001% to less than 0.01%, e.g., from 0.001% to 0.008%, preferably about 0.005% by weight. The polyribonucleotide is present in circular form.

Polyribonucleotides can be susceptible to RNase that can be abundant in ambient environment. Compositions or cosmetic compositions provided herein can include reagents that inhibit RNase activity, thereby preserving the polyribonucleotide from degradation. In some cases, the composition includes any RNase inhibitor known to one skilled in the art. Alternatively or additionally, the polyribonucleotide, and cell-penetrating agent and/or pharmaceutically acceptable diluents or carriers, vehicles, excipients, or other reagents in the composition provided herein can be prepared in RNase-free environment. The composition can be formulated in RNase-free environment.

In some cases, a composition provided herein can be sterile. The composition can be formulated as a sterile solution or suspension, in suitable vehicles, known in the art. The composition can be sterilized by conventional, known sterilization techniques, e.g., the composition can be sterile filtered.

### Salts

In some cases, a composition or cosmetic composition provided herein comprises one or more salts. For controlling the tonicity, a physiological salt such as sodium salt can be included a composition provided herein. Other salts can comprise potassium chloride, potassium dihydrogen phosphate, disodium phosphate, and/or magnesium chloride, or the like. In some cases, the composition or cosmetic composition is formulated with one or more pharmaceutically acceptable salts. The one or more pharmaceutically acceptable salts can comprise those of the inorganic ions, such as, for example, sodium, potassium, calcium, magnesium ions, and the like. Such salts can comprise salts with inorganic or organic acids, such as hydrochloric acid, hydrobromic acid, phosphoric acid, nitric acid, sulfuric acid, methanesulfonic acid, p-toluenesulfonic acid, acetic acid, fumaric acid, succinic acid, lactic acid, mandelic acid, malic acid, citric acid, tartaric acid, or maleic acid. The polyribonucleotide is present in circular form.

### Buffers/pH

A composition or cosmetic composition provided herein can comprise one or more buffers, such as a Tris buffer; a borate buffer; a succinate buffer; a histidine buffer (e.g., with an aluminum hydroxide adjuvant); or a citrate buffer. Buffers, in some cases, are included in the 5-20 mM range. The polyribonucleotide is present in circular form.

A composition or cosmetic composition provided herein can have a pH between about 5.0 and about 8.5, between about 6.0 and about 8.0, between about 6.5 and about 7.5, or between about 7.0 and about 7.8. The composition or cosmetic compositions can have a pH of about 7.

### Detergents/surfactants

A composition or cosmetic composition provided herein can comprise one or more detergents and/or surfactants, depending on the intended administration route, e.g., polyoxyethylene sorbitan esters surfactants (commonly referred to as "Tweens"), e.g., polysorbate 20 and polysorbate 80; copolymers of ethylene oxide (EO), propylene oxide (PO), and/or butylene oxide (BO), sold under the DOWFAX^{™} tradename, such as linear EO/PO block copolymers; octoxynols, which can vary in the number of repeating ethoxy (oxy-l,2-ethanediyl) groups, e.g., octoxynol-9 (Triton X-100, or t-octylphenoxypolyethoxyethanol); (octylphenoxy)polyethoxyethanol (IGEPAL CA-630/NP-40); phospholipids such as phosphatidylcholine (lecithin); nonylphenol ethoxylates, such as the Tergitol^{™} NP series; polyoxyethylene fatty ethers derived from lauryl, cetyl, stearyl and oleyl alcohols (known as Brij surfactants), such as triethyleneglycol monolauryl ether (Brij 30); and sorbitan esters (commonly known as "SPANs"), such as sorbitan trioleate (Span 85) and sorbitan monolaurate, an octoxynol (such as octoxynol-9 (Triton X-100) or t-octylphenoxypolyethoxyethanol), a cetyl trimethyl ammonium bromide ("CTAB"), or sodium deoxycholate. The one or more detergents and/or surfactants can be present only at trace amounts. In some cases, the composition can include less than 1 mg/ml of each of octoxynol-10 and polysorbate 80. Non-ionic surfactants can be used herein. Surfactants can be classified by their "HLB" (hydrophile/lipophile balance). In some cases, surfactants have a HLB of at least 10, at least 15, and/or at least 16. The polyribonucleotide is present in circular form.

### Oils

A composition or cosmetic composition provided herein can comprise one or more oils. The composition or cosmetic composition may comprise an oil phase containing at least one oil. For example, an oil is a hydrocarbon oil from animal origin, such as perhydrosqualene. An oil can be hydrocarbon oils from plant origin, such as liquid fatty acid triglycerides comprising from 4 to 10 carbon atoms such as heptanoic or octanoic acid triglycerides or, additionally, sunflower, maize, soya, marrow, grape seeds, sesame, hazelnut, apricot, macadamia, arara, avocado and castor oils, caprylic/capric acid triglycerides, such as those sold by Stearineries Dubois company or those sold under the designations Miglyol 810, 812 and 818 by Dynamit Nobel company, jojoba oil, shea butter oil. The oil may comprise synthesis esters and ethers, including from fatty acids, such as oils with the formulas R1COOR2 and R1OR2 wherein R1 represents the moiety of a fatty acid comprising 8 to 29 carbon atoms, and R2 represents a branched or unbranched hydrocarbon chain, containing from 3 to 30 carbon atoms, such as, for instance, Purcellin oil, isononyl isononanoate, isopropyl myristate, ethyl-2-hexyl palmitate, octyl-2-dodecyl stearate, octyl-2-dodecyl erucate, isostearyl isostearate; hydroxyl esters such as isostearyl lactate, octyl hydroxystearate, octyldodecyl hydroxystearate, diisocstearyl malate, triisocetyl citrate, fatty alcohol heptanoates, octanoates and decanoates; polyol esters such as propylene glycol dioctanoate, neopentyl glycol diheptanoate and diethylene glycol diisononanoate; and pentaerythritol esters such as pentaerythrityl tetraisostearate. The oil may comprise straight or branched hydrocarbons, from mineral or synthetic origin, such as volatile or not volatile paraffin oils and their derivatives, vaseline, polydecenes, hydrogenated polyisobutene, such as Parleam^{™} (sold by Nippon Gel Fats Company). The oil can comprise fatty alcohols with from 8 to 26 carbon atoms, such as cetyl alcohol, stearyl alcohol and the mixtures thereof (cetylstearyl alcohol), octyldodecanol, 2-butyloctanol, 2-hexyldecanol, 2-undecylpentadecanol, oleic alcohol or linoleic alcohol; alkyl fatty alcohols, including ethoxylated, such as oleth-12; partially hydrocarbon and/or silicon fluorinated oils, such as those described in document JP-A-2-295912. Fluorinated oils include perfluoromethylcyclopentane and perfluoro-1,3 dimethylcyclohexane, sold under trademarks FLUTEC PC 1^{™} and FLUTEC PC3^{™} by BNFL Fluorochemicals company; perfluoro-1,2-dimethylcyclobutane; perfluoroalkanes such as dodecafluoropentane and tetradecafluorohexane sold under trademarks PF 5050^{™} and PF 5060^{™} by 3M Company or also bromoperfluorooctyl sold under trademark FORALKYL^{™} by Atochem company; nonafluoromethoxybutane sold under trademark MSX 4518^{™} by 3M company and nonafluoroethoxyisobutane; perfluoromorpholine derivatives, such as 4-trifluoromethyl perfluoromorpholine sold under trademark PF-5052^{™} by 3M company; silicone oils such as polymethylsiloxanes (PDMS), either volatile or not, with a straight or cyclic silicone chain, liquid or pasty at room temperature, including cyclopolydimethylsiloxanes (cyclomethicones) such as cyclohexasiloxane; polydimethylsiloxanes comprising alkyl, alkoxy or phenyl groups, either pendant or at silicone chain end, having from 2 to 24 carbon atoms; phenylated silicones such as phenyltrimethicones, phenyldimethicones, phenyltrimethylsiloxydiphenyl-siloxanes, diphenyldimethicones, diphenylmethyldiphenyl trisiloxanes, 2-phenylethyltrimethyl-siloxysilicates, and polymethylphenyl-siloxanes; andmixtures thereof.

### EFFECTIVE DELIVERY

Compositions, cosmetic compositions, methods, and kits provided herein can offer an easy-to-operate and effective solution for delivery of polyribonucleotides (e.g., cosmetic polyribonucleotides) into cells. In some case, the delivery of polyribonucleotides into cells is cosmetic. In some cases, the delivery efficiency can be relatively high in the presence of the alcohol described herein as compared to delivery without the alcohol. In some cases, the delivery efficiency can be relatively high in the presence of the cell-penetrating agent described herein as compared to delivery without the cell-penetrating agent. In some cases, the delivery efficiency can be relatively high after pre-treatment using a sterilizing agent (e.g., alcohol) described herein as compared to delivery without the pre-treatment. The polyribonucleotide is present in circular form. The polyribonucleotide comprise a payload. The payload can be a cosmetic payload. A cosmetic payload can be any payload that has a cosmetic effect after administration to a cell, tissue, or integumentary member. The payload can be one or more sequences in the polyribonucleotide that can be responsible for expression products. In some embodiments, the payload is a cosmetic protein or peptide selected from the group consisting of collagen, keratin, elastin, and botulinum toxin, or any a fragment thereof.

In some cases, the delivery efficiency is expressed as a ratio of the amount of the polyribonucleotides that are delivered into a cell over the amount of the total polyribonucleotides that are brought into contact with the cell. In some cases, the delivery efficiency is expressed as a ratio of the amount of the polyribonucleotides that are delivered into cells over the amount of the total polyribonucleotides that are administered near the cells (for instance, the amount delivered into skin cells when the polyribonucleotides are applied directly on a skin area). The delivery efficiency of the methods provided herein can be at least about 0.5%, 1%, 1.5%, 2%, 3%, 4%, 5%, 10%, 15%, 20%, 25%, 30%, 35%, 40%, 45%, 50%, 55%, 60%, 65%, 70%, 75%, 80%, 85%, 90%, or 95%. In some cases, the delivery efficiency of the methods provided herein can be about 0.5%, 1%, 1.5%, 2%, 3%, 4%, 5%, 10%, 15%, 20%, 25%, 30%, 35%, 40%, 45%, 50%, 55%, 60%, 65%, 70%, 75%, 80%, 85%, 90%, 95%, or 100%.

In some cases, the cosmetic effects of the compositions and methods provided herein are measured in terms of observed changes in the appearance of an integumentary member of a subject due to the administration of the composition. Alternatively, the cosmetic effects are determined by the abundance of the polyribonucleotides in the subject or one or more cells of the subject. In other cases, expression product can be measured as an indicator of the delivery efficiency of the compositions and methods provided herein, if the compositions include expression sequence that encodes a protein to be expressed in the subject.

The compositions and methods provided herein can be particularly more effective for delivery of circular polyribonucleotide as compared to linear polyribonucleotide. An amount of the circular polyribonucleotide delivered to a cell is at least 1.1, 1.2, 1.3, 1.4, 1.5, 1.6, 1.7, 1.8, 1.9, 2.0, 2.2, 2.4, 2.5, 2.6, 2.8, 3.0, 3.5, 4.0, 4.5, 5.0, 6.0, 7.0, 8.0, 9.0, or 10.0 fold higher than an amount of a linear polyribonucleotide contacted to the cell with a mixture comprising the linear polyribonucleotide and the cell-penetrating agent. In some cases, an amount of the circular polyribonucleotide delivered to a cell is at least 1.1 fold higher than an amount of a linear polyribonucleotide contacted to the cell with a mixture comprising the linear polyribonucleotide and the cell-penetrating agent.

When administered in vivo, a polyribonucleotide can be delivered, according to methods provided herein, into any type of cells that are in proximity to a surface area of the subject, depending on the administration route. For example, polyribonucleotides can be delivered by the methods provided herein to epithelial cells that are located under skin or on the surface of cavities or tracts by the methods provided herein. Non-limiting types of epithelial cell include simple squamous epithelium, simple cuboidal epithelium, simple columnar epithelium, pseudostratified columnar epithelium, stratified squamous epithelium, stratified cuboidal epithelium, stratified columnar epithelium, and transitional epithelium. Poyribonucleotides can be delivered by the methods provided herein to any type of cells, including, but not limited to, keratinocytes, Merkel cells, melanocytes, Langerhans cells, fibroblasts, macrophages, and adipocytes under the surface area, for example, skin. Polyribonucletides can be delivered by the methodsprovided here to any part of the tissue underneath skin, such as, epidermis, basement membrane, dermis, and subcutaneous tissue. The compositions, methods, and kits provided herein can be suitable for extended delivery of the polyribonucleotides. For example, the polyribonucleotide and alcohol (e.g., ethanol) can be formulated for extended release, controlled release, delayed release, or sustained release, so that particular cosmetic effect can be achieved or the polyribonucleotide can be delivered into desired locations of the subject. For instance, the polyribonucleotide and alcohol (e.g., ethanol) can be formulated in a form of a patch that is to be adhered to a skin area of a subject for an extended period, e.g., at least about 2 hrs, 4 hrs, 6 hrs, 8 hrs, 10 hrs, 15 hrs, 20 hrs, 24 hrs, 36 hrs, 2 days, 3 days, 4 days, 5 days, 6 days, 7 days, 8 days, 9 days, 10 days, 11 days, 12 days, 13 days, 2 weeks, 3 weeks, 4 weeks, 5 weeks, 2 months, 3 months, 4 months, or even longer. In some cases, the polyribonucleotide and cell-penetrating agent in the patch are delivered over an extended period, e.g., as long as the patch is adhered on the skin area. The polyribonucleotide and cell-penetrating agent can be formulated for extended release, controlled release, delayed release, or sustained release, so that particular cosmetic effect can be achieved or the polyribonucleotide can be delivered into desired locations of the subject. For instance, the polyribonucleotide and cell-penetrating agent can be formulated in a form of a patch that is to be adhered to a skin area of a subject for an extended period, e.g., at least about 2 hrs, 4 hrs, 6 hrs, 8 hrs, 10 hrs, 15 hrs, 20 hrs, 24 hrs, 36 hrs, 2 days, 3 days, 4 days, 5 days, 6 days, 7 days, 8 days, 9 days, 10 days, 11 days, 12 days, 13 days, 2 weeks, 3 weeks, 4 weeks, 5 weeks, 2 months, 3 months, 4 months, or even longer. In some cases, the polyribonucleotide and cell-penetrating agent in the patch are delivered over an extended period, e.g., as long as the patch is adhered on the skin area.

In some cases, the compositions, cosmetic compositions, methods, and kits provide polyribonucleotides that are delivered into a cell and have cosmetic effects over extended period of time. For example, the polyribonucleotides have little to no susceptibility to RNase, or they have very long half-life inside the cell. As a result, the polyribonucleotides can be present and potentially active throughout an extended period of time, for instance, at least about 24 hrs, 36 hrs, 2 days, 3 days, 4 days, 5 days, 6 days, 7 days, 8 days, 9 days, 10 days, 11 days, 12 days, 13 days, 2 weeks, 3 weeks, 4 weeks, 5 weeks, 2 months, 3 months, 4 months, 5 months, 6 months, 7 months, 8 months, 9 months, 10 months, 1 year, 2 years, 3 years, or even longer. In some cases, the polyribonucleotides can have a half-life that is about 24 hrs, 36 hrs, 2 days, 3 days, 4 days, 5 days, 6 days, 7 days, 8 days, 9 days, 10 days, 11 days, 12 days, 13 days, 2 weeks, 3 weeks, 4 weeks, 5 weeks, 2 months, 3 months, 4 months, 5 months, 6 months, 7 months, 8 months, 9 months, 10 months, 1 year, 2 years, or 3 years.

### KITS AND APPLICATION TOOLS

Depending on the different intended administration routes, the composition provided herein can be packaged in different manners, and/or in some cases, in different kits that include the composition and one or more application tools configured to administer the composition to a subject via the intended routes. As discussed above, a composition provided herein can be formulated for administered through different routes, including direct topic administration (*e.g*., percutaneous), or ophthalmic. In aspects, the present disclosure provides kits for administration of the compositions comprising the circular polyribonucleotide and the cell-penetrating agent. In aspects, the present disclosure provides kits for administration of the cosmetic compositions comprising the cosmetic circular polyribonucleotide and alcohol. In aspects, the present disclosure provides kits for administration of the cosmetic compositions comprising the cosmetic circular polyribonucleotide and ethanol. In aspects, the present disclosure provides kits for administration of the cosmetic compositions free of any carrier comprising the cosmetic circular polyribonucleotide and diluent after pre-treatment as described herein. The polyribonucleotide is in circular form. The polyribonucleotide can comprise a payload. The payload can be a cosmetic payload. A cosmetic payload can be any payload that has a cosmetic effect after administration to a cell, tissue, or integumentary member. The payload can be one or more sequences in the polyribonucleotide that can be responsible for expression products. In some embodiments, the payload is a cosmetic protein or peptide selected from the group consisting of collagen, keratin, elastin, and botulinum toxin, or any a fragment thereof.

A kit can be configured for direct topical administration, e.g., direct application on skin. In some cases, the kit comprises a substrate or scaffold comprising a composition as provided herein. The substrate or scaffold can be in a form of a patch, a wipe, Q-tip, or any other form that allows direct application of the composition onto a subject's skin surface. The wipe or swab can be a pad, a cotton ball, or a cotton-tipped applicator. The substrate or scaffold can be made of disposable material, or biodegradable materials.

In some cases, provided herein is a kit that comprises a peel pad and a composition described herein. In some cases, the peel pad comprises a composition described herein adsorbed or absorbed upon it. In some cases, the substrate described herein is the facial pad. In some cases, the substrate can be a fiber layer, e.g., a fiber layer constituted using a non-elastomer raw material and having elongatability at least in one direction, e.g., cotton, eucalyptus or biocellulose. As the fiber layer, paper, a nonwoven fabric, a woven fabric and so forth can be utilized. The fiber layer can be hydrophilic and have liquid retention properties. The fiber layer can be made of hydrophilic fibers obtained using a hydrophilic raw material, and from non-elastomer raw material or the fibers formed therefrom. In some cases, the kit includes a liquid composition provided herein and a transfer tool configured to transfer or dispense the liquid composition. The transfer tool can be simple as a straw, an aurilave, or a transfer pipette. The transfer tool can also be designed with additional features, such as graduation, actuation, alarm system, or automatic dispensing system.

A kit provided herein can further comprise a container, such as a bottle, box, capsule, or dispenser, comprising the formulated composition. A container as disclosed can be an application tool as well. In some cases, a dispenser is provided for dispensing a liquid or solid formulation of the composition described herein. For example, a transfer pipette can be used to drop liquid onto a skin surface or onto eyes for intraocular delivery. Sterility, humidity, and/or temperature can be maintained, if required, in the containers as described herein. In some cases, an inhaler as discussed above can be a container for storing the composition in its liquid, solid, or aerosol form if needed. Certainly, alternatively, a container and an application tool can be provided separately.

A kit provided herein can comprise a first application tool, a second application tool, a sterilizing agent, and a cosmetic composition free of any carrier comprising the cosmetic polyribonucleotide and diluent, wherein the first application tool is configured to apply a sterilizing agent to a surface area (e.g., integumentary member) of a subject and the second application tool is configured to apply the cosmetic composition to the surface area (e.g., the integumentary member) of the subject. The sterilizing agent can be an alcohol, iodine, hydrogen peroxide, UV light, laser light, or heat. In some embodiments, the alcohol is selected from the group consisting of: methanol, ethanol, isopropanol, butanol, pentanol, cetyl alcohol, ethylene glycol, propylene glycol, denatured alcohol, benzyl alcohol, specially denatured alcohol, glycol, stearyl alcohol, cetearyl alcohol, menthol, polyethylene glycols (PEG)-400, isopropyl alcohol, dimethylaminoethanol, retinol, d-alpha-tocopherol, SD alcohol, SD alcohol 40, c12-16, lauryl alcohol, myristyl alcohol, butylene glycol, and propanediol. The first application tool can be a wipe or swab, wherein the wipe or swab comprises the sterilizing agent. Alternatively, the first application tool can be a device that applies UV light or laser light, or a device that applies heat. The second application tool can comprise a pipette. In some embodiments, the second application tool comprises a substrate, and wherein the substrate is embedded with the mixture. Often, the substrate is made of natural or artificial fibers. In some embodiments, the second application tool comprises a patch, a sprayer, or a peel pad. In some cases, the second application tool is configured to release the mixture in a controlled manner. The surface area can be an integumentary member, for example, hair, eyelash, nail, and skin (e.g., facial skin, scalp, etc.) and any combination thereof.

### METHODS OF PRODUCTION

In some cases, the polyribonucleotide in the composition or cosmetic composition provided herein comprises a deoxyribonucleic acid sequence that is non-naturally occurring and can be produced using recombinant DNA technology (methods described in detail below; e.g., derived *in vitro* using a DNA plasmid) or chemical synthesis. The polyribonucleotide is present in circular form.

A DNA molecule used to produce an RNA circle can include a DNA sequence of a naturally-occurring original nucleic acid sequence, a modified version thereof, or a DNA sequence encoding a synthetic polypeptide not normally found in nature (e.g., chimeric molecules or fusion proteins). DNA molecules can be modified using a variety of techniques including, but not limited to, classic mutagenesis techniques and recombinant DNA techniques, such as site-directed mutagenesis, chemical treatment of a nucleic acid molecule to induce mutations, restriction enzyme cleavage of a nucleic acid fragment, ligation of nucleic acid fragments, polymerase chain reaction (PCR) amplification and/or mutagenesis of selected regions of a nucleic acid sequence, synthesis of oligonucleotide mixtures and ligation of mixture groups to "build" a mixture of nucleic acid molecules and combinations thereof.

The polyribonucleotide can be prepared according to any available technique including, but not limited to chemical synthesis and enzymatic synthesis. In some cases, a linear polyribonucleotide can be synthesized from ribonucleotide or transcribed from a DNA construct. The transcription from DNA construct can take place inside a cell or in vitro, using techniques available to one skilled in the art.

In some cases, a linear primary construct or linear mRNA can be cyclized, or concatemerized to create a circular polyribonucleotide described herein. The mechanism of cyclization or concatemerization may occur through methods such as, but not limited to, chemical, enzymatic, splint ligation), or ribozyme catalyzed methods. The newly formed 5 '-/3 '-linkage may be an intramolecular linkage or an intermolecular linkage.

Methods of making the circular polyribonucleotides described herein are described in, for example, Khudyakov & Fields, Artificial DNA: Methods and Applications, CRC Press (2002); in Zhao, Synthetic Biology: Tools and Applications, (First Edition), Academic Press (2013); and Egli & Herdewijn, Chemistry and Biology of Artificial Nucleic Acids, (First Edition), Wiley-VCH (2012).

Various methods of synthesizing circular polyribonucleotides are also described in the art (see, e.g., US Patent No. US6210931, US Patent No. US5773244, US Patent No. US5766903, US Patent No. US5712128, US Patent No. US5426180, US Publication No. US20100137407, International Publication No. WO1992001813 and International Publication No. WO2010084371).

In some aspects, a cosmetic composition of the present disclosure comprises a mixture of a circular polyribonucleotide and a cell-penetrating agent, wherein the cell-penetrating agent constitutes 0.3% v/v of the mixture.

A cosmetic composition of the present disclosure may comprise a circular polyribonucleotide and a cell-penetrating agent, wherein the cell-penetrating agent is configured for topical administration.

A cosmetic composition of the present disclosure may comprise a circular polyribonucleotide and a cell-penetrating agent, wherein the circular polyribonucleotide comprises a payload or a sequence encoding a payload and wherein the payload has a biological effect on a cell or a tissue.

A cosmetic composition of the present disclosure may comprise a circular polyribonucleotide and a cell-penetrating agent, wherein the circular polyribonucleotide is in an amount effective to have a cosmetic effect on a cell or a tissue and wherein the cell-penetrating agent is in an amount effective have a cosmetic effect on the cell or the tissue.

A cosmetic composition of the present disclosure may comprise a circular polyribonucleotide, a cell-penetrating agent, and a topical delivery excipient, wherein the topical delivery excipient comprises a stabilizer or other carrier.

A cosmetic composition of the present disclosure may comprise a biodegradable scaffold loaded with circular polyribonucleotide and a cell-penetrating agent.

A method of delivering a cosmetic polyribonucleotide to a cell or a tissue may comprise contacting the cell or the tissue to a mixture comprising the cosmetic circular polyribonucleotide and a cell-penetrating agent, wherein the cell-penetrating agent constitutes 0.3% v/v of the mixture.

A method of delivering a cosmetic composition to a cell or a tissue may comprise contacting the cell or the tissue to the cosmetic composition comprising a cosmetic circular polyribonucleotide and a cell-penetrating agent, wherein the cell-penetrating agent is configured for topical administration. In some embodiments, the cell-penetrating agent comprises an alcohol. In some embodiments, the alcohol is selected from the group consisting of: methanol, ethanol, isopropanol, butanol, pentanol, cetyl alcohol, ethylene glycol, propylene glycol, denatured alcohol, benzyl alcohol, specially denatured alcohol, glycol, stearyl alcohol, cetearyl alcohol, menthol, polyethylene glycols (PEG)-400, isopropyl alcohol, dimethylaminoethanol, retinol, d-alpha-tocopherol, SD alcohol, SD alcohol 40, c12-16, lauryl alcohol, myristyl alcohol, butylene glycol, and propanediol. In some embodiments, the alcohol comprises ethanol. The cell-penetrating agent may constitute at least about 10%, at least about 20%, at least about 30%, at least about 40%, at least about 50%, at least about 60%, at least about 70%, at least about 80%, at least about 90%, at least about 95%, at least about 98%, or about 100% v/v of the mixture. The cell-penetrating agent constitutes about 10% v/v of the mixture. In some embodiments, the method further comprises mixing the cosmetic circular polyribonucleotide with the cell-penetrating agent. The cosmetic circular polyribonucleotide may be in a solid form before the mixing. The cosmetic circular polyribonucleotide may be lyophilized before the mixing. The cosmetic circular polyribonucleotide may be in a liquid form before the mixing. The cosmetic circular polyribonucleotide may be dissolved in a solvent before the mixing.

In some aspects, a method comprises applying a cosmetic composition onto an integumentary member of a subject, wherein the composition comprises a mixture comprising a cosmetic circular polyribonucleotide and a cell-penetrating agent. In some embodiments, the cell-penetrating agent comprises an alcohol. In some embodiments, the alcohol is selected from the group consisting of: methanol, ethanol, isopropanol, butanol, pentanol, cetyl alcohol, ethylene glycol, propylene glycol, denatured alcohol, benzyl alcohol, specially denatured alcohol, glycol, stearyl alcohol, cetearyl alcohol, menthol, polyethylene glycols (PEG)-400, isopropyl alcohol, dimethylaminoethanol, retinol, d-alpha-tocopherol, SD alcohol, SD alcohol 40, c12-16, lauryl alcohol, myristyl alcohol, butylene glycol, and propanediol. In some embodiments, the alcohol comprises ethanol. The delivery may be localized. The cell-penetrating agent may constitute at least about 1%, at least about 5%, at least about 10%, at least about 20%, at least about 30%, at least about 40%, at least about 50%, at least about 60%, at least about 70%, at least about 80%, at least about 90%, at least about 95%, at least about 98%, or about 100% v/v of the mixture. In some embodiments, the cell-penetrating agent constitutes about 10% v/v of the mixture. In some embodiments, the integumentary member comprises skin, nails, or hair. Applying may comprise depositing a drop of the mixture directly onto the integumentary member. Applying may comprise wiping the integumentary member with a patch, a gel, or a film embedded with the mixture. Applying may comprise spraying the mixture onto the integumentary member. The cell may comprise an epithelial cell. In some embodiments, the cosmetic polyribonucleotide comprises a circular polyribonucleotide. The circular polyribonucleotide may have a translation efficiency at least 5%, at least 10%, at least 15%, at least 20%, at least 30%, at least 40%, at least 50%, at least 60%, at least 70%, at least 80%, at least 90%, at least 100%, at least 150%, at least 2 fold, at least 3 fold, at least 4 fold, at least 5 fold, at least 6 fold, at least 7 fold, at least 8 fold, at least 9 fold, at least 10 fold, at least 20 fold, at least 50 fold, or at least 100 fold greater than a linear counterpart. The circular polyribonucleotide may have a translation efficiency at least 5 fold greater than a linear counterpart. The polyribonucleotide may have a short term cosmetic effect. The polyribonucleotide may have a long term cosmetic effect. The polyribonucleotide is a circular polyribonucleotide. A concentration of the cosmetic polyribonucleotide in the mixture may be at least about 1 ng/mL, at least about 5 ng/mL, at least about 10 ng/mL, at least about 50 ng/mL, at least about 100 ng/mL, at least about 500 ng/mL, at least about 1 µg/mL, at least about 2 µg/mL, at least about 3 µg/mL, at least about 4 µg/mL, at least about 5 µg/mL, at least about 10 µg/mL, at least about 20 µg/mL, at least about 50 µg/mL, at least about 100 µg/mL, at least about 200 µg/mL, at least about 500 µg/mL, at least about 1 mg/mL, at least about 2 mg/mL, at least about 5 mg/mL, at least about 10 mg/mL, at least about 20 mg/mL, at least about 50 mg/mL, or at least about 100 mg/mL.

In some aspects, a kit of the present disclosure comprises an application tool and a cosmetic composition comprising a mixture that comprises a cosmetic circular polyribonucleotide and a cell-penetrating agent, wherein the application tool is configured to apply the mixture to an integumentary member of a subject. In some embodiments, the application tool comprises a pipette. In some embodiments, the application tool comprises a substrate, and wherein the substrate is embedded with the cosmetic composition. In some embodiments, the substrate is made of natural or artificial fibers. In some embodiments, the application tool comprises a patch. In some embodiments, the application tool comprises a peel pad. In some embodiments, the application tool comprises a sprayer. In some embodiments, the application tool is configured to release the mixture in a controlled manner. In some embodiments, the integumentary member comprises skin, nails, or hair.

### EXAMPLES

The following examples are provided to further illustrate some embodiments of the present invention.

### Example 1: Formulation of RNA for topical delivery

This Example demonstrates formulation of RNA for topical delivery.

To determine topical effects of RNA, RNA was formulated for delivery to epithelial tissues.

As described herein, RNA was formulated with a cell penetrating agent according to the following:
lOng RNA linear or circular comprising an EGF ORF
5uL 80% ethanol
35uL PBS+glucose (4.5g/L)

### Example 2: Topical delivery of linear RNA

This Example demonstrates topical delivery of RNA.

To determine topical delivery effects, RNA was formulated and delivered to epithelial tissues. As described herein, linear RNA formulated with a cell penetrating agent was delivered topically to ear tissue.

Samples of linear RNA were formulated as in Example 1 (50µL) and applied to an ear of a mouse. Ears were wiped with an isopropyl alcohol wipe prior to application of the samples to the ears. Samples were dried by exposing the ears briefly to a heatlamp and fan in a sterilized hood. Mice were placed back in cages under normal conditions.

At select timepoints (6 hrs, 1day, 3 days, or 12 days post application), ear tissues (through a single ear punch) were collected for each RNA sample and stored in a tissue storage reagent (e.g., permeates the tissue to stabilize and protect cellular RNA in unfrozen samples).

### Example 3: Topical delivery of circular RNA

This Example demonstrates topical delivery of RNA.

To determine topical delivery effects, RNA was formulated and delivered to epithelial tissues. As described herein, RNA was formulated with a cell penetrating agent and delivered topically to ear tissue.

Samples of circular RNA were formulated as in Example 1 (50µL) and applied to both ears of a mouse. Ears were wiped with an isopropyl alcohol wipe prior to application of the samples to the ears. Samples were dried on the ears by exposing the ears briefly to a heatlamp and fan in a sterilized hood. Mice were placed back in cages under normal conditions.
At select timepoints (6 hrs, 1 day, 3 days, or 12 days post application), ear tissues (through a single ear punch) were collected for each RNA sample.

### Example 4: Persistence of RNA after topical delivery

This Example demonstrates RNA presence after topical delivery.

To determine RNA persistence, tissue samples were analyzed for delivered RNA. As described herein, ear punches were analyzed for persistence at varying timepoints after topical delivery of the RNA.

Ear punch samples from Example 3 and untreated ear punch samples were collected in an RNA stabilization reagent and RNA was extracted using a standard RNA tissue extraction kit (Maxwell RSC simply RNA).

A volume of 200µl of 1-Thioglycerol / Homogenization Solution was added to each sample. A working solution was prepared by adding 20µl of 1-Thioglycerol per milliliter of Homogenization Solution. Alternatively, 600µl of 1-Thioglycerol was added to the 30ml bottle of Homogenization Solution. Before use, the 1-Thioglycerol/Homogenization Solution was chilled on ice or at 2-10°C.

The tissue samples were homogenized in 200µl of chilled 1- Thioglycerol / Homogenization Solution with a handheld homogenizer and sterile pestle until no visible tissue fragments remained. Each sample was homogenized an additional 15-30 seconds for complete homogenization.

To check for the presence of RNA at the different timepoints, the samples were checked for RNA via q-PCR. qPCR was used to measure the presence of both linear and circular RNA in the ear punches. To detect linear and circular RNA, primers that amplified the Nluc ORF were used. (F: AGATTTCGTTGGGGACTGGC (SEQ ID NO: 7), R: CACCGCTCAGGACAATCCTT (SEQ ID NO: 8)). To detect only circular RNA, primers that amplified the 5'-3' junction allowed for detection of circular but not linear RNA constructs (F: CTGGAGACGTGGAGGAGAAC (SEQ ID NO: 9), R: CCAAAAGACGGCAATATGGT (SEQ ID NO: 10)).

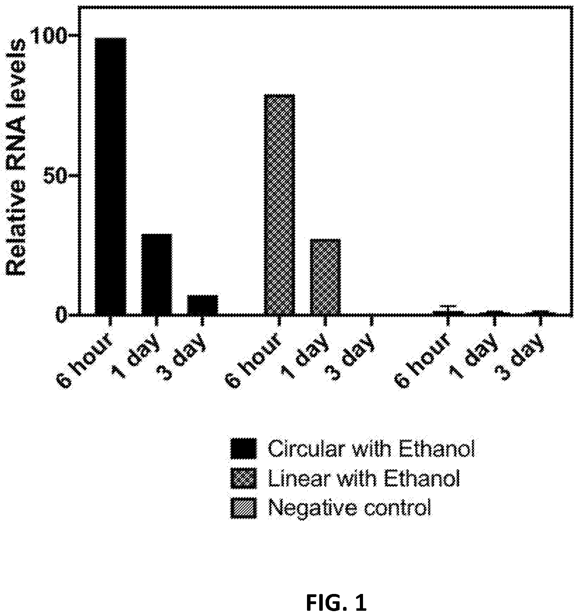

Linear and circular RNA was detected at 6 hrs, 24 hrs, and 72 hrs after topical delivery. Higher levels of circular RNA compared to linear RNA were detected in ears of mice at 3-days post-injection (**FIG. 1**).

As shown in this Example, linear and circular RNA administered topically were detectable *in vivo.*

### Example 5: Protein expression of mRNA after topical delivery

This Example describes protein presence after topical delivery.

To determine if topical delivered RNA can be translated, tissue samples are analyzed for protein expression at the different timepoints by western blot. Ear punches are analyzed for protein expression after topical delivery of the RNA.

In short, the ear punches are collected and stored in an RNA stabilization reagent (Invitrogen). The tissue is homogenized in RIPA buffer with micro tube homogenizer (Fisher scientific) and protein is extracted. Each sample is centrifuged at 14k x g for 15 mins.

The supernatant is removed and the pellet is dissolved in 2× SDS sample buffer (0.125 M Tris-HCl, pH 6.8, 4% SDS, 30% glycerol, 5% 2-mercaptoethanol, 0.01% bromophenol blue) at 70 °C for 15 min.

A commercially available standard (BioRad) is used as the size marker. After being electrotransferred to a polyvinylidene fluoride (PVDF) membrane (Millipore) using a semi-dry method, the blot is visualized using a chemiluminescent kit (Rockland).

It is expected that the GFP protein is visualized in ear punch samples and is detected in circular RNA and linear RNA.

### Example 6: Topical administration of RNA results in RNA delivery to tissue when ethanol is included in the RNA solution

This Example demonstrates the ability to deliver RNA to cells and tissues via topical administration *in vivo* when ethanol is included in the RNA solution.

In this example, circular RNA was designed with an EMCV IRES and ORF encoding Nanoluciferase (NLuc).

The circular RNA was generated *in vitro.* Unmodified linear RNA was transcribed *in vitro* from a DNA template. Transcribed RNA was purified with an RNA cleanup kit (New England Biolabs, T2050), treated with RNA 5'phosphohydrolase (RppH) (New England Biolabs, M0356) following the manufacturer's instructions, and purified again with an RNA purification column. RppH treated linear RNA circularized using a splint DNA and T4 RNA ligase 2 (New England Biolabs, M0239). Circular RNA was then Urea-PAGE purified, eluted in a buffer (0.5 M Sodium Acetate, 0.1% SDS, 1 mM EDTA), ethanol precipitated and resuspended in RNA storage solution (ThermoFisher Scientific, cat# AM7000). In this example, circular RNA was also HPLC-purified.

In this example, linear mRNA was designed with an ORF encoding a Nano Luciferase (NLuc). In this example, modified linear mRNA was made in-house by *in vitro* transcription. In this example, linear RNA was fully substituted with Pseudo-Uridine and 5-Methyl-C, capped with CleanCap^{™} AG, included 5' and 3' human alpha-globin UTRs, and is polyadenylated.

RNA was then diluted in PBS/Glucose (4.5g/L) and ethanol (10% v/v) such that total sample volume for each sample was 50 uL, and total RNA for each sample was 3.5 pmoles. All reagents are brought to room temperature prior to mixing and mixtures are prepared immediately prior to use.

At time = 0, 50 µL dose of each sample was applied topically to the ear of a BALB/c mouse dropwise using a pipet tip. As a negative control, an untreated mouse was used. Samples were dried by exposing the ears briefly to a heat lamp and fan in a sterilized hood. Mice were place back in cages under normal conditions.

To determine RNA persistence in tissue, tissue samples were analyzed for RNA at varying timepoints after delivery using RT-qPCR. At 6 hours, 1, 3, and 12 days post-administration, a 2 mm ear punch was taken from each animal and stored in RNAlater solution (ThermoFisher Scientific, cat# AM7020). Total RNA was isolated from ear punches using trizol extraction. The aqueous-phase was precipitated with isopropanol and the pellet was washed with 70% ETOH as per manufacturer's instructions. cDNA was synthesized from the total RNA and RT-PCR was performed on cDNA templates using primers specific to the NLuc ORF. All samples were assayed in triplicate on the Bio-rad CFX384 Thermal Cycler. RNA levels were then relativized to actin and to the untreated negative control.

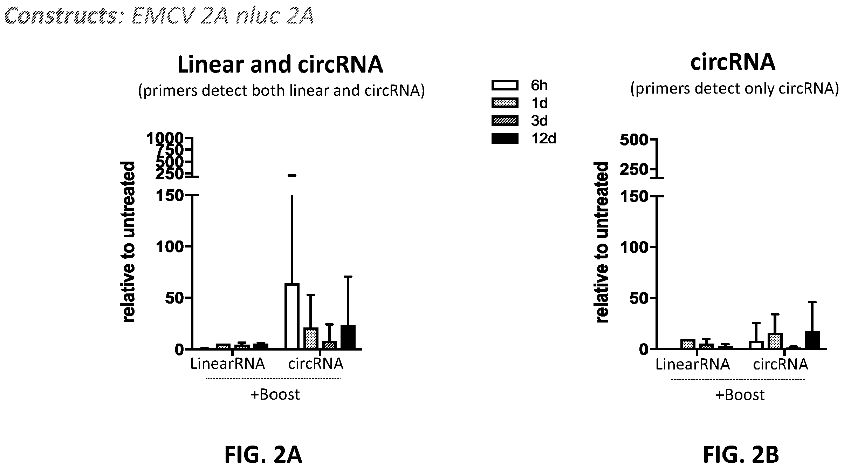

Circular RNA and linear RNA were detected in tissue samples at 6 hours and 1, 3 and 12 days after topical administration and showed greater signal than the vehicle only control (**FIG. 2A & FIG. 2B**).

This Example demonstrates that circular RNA and linear RNA are successfully delivered via topical administration to the tissue when delivered with ethanol and persists in tissue over prolonged periods of time.

### Example 7: Topical administration of RNA results in RNA delivery to tissue when formulated with TransIT

This Example demonstrates the ability to deliver RNA to cells and tissues via topical administration *in vivo* when TransIT is used to formulate in the RNA solution

In this example, circular RNA was designed with an EMCV IRES and ORF encoding Nanoluciferase (NLuc).

The circular RNA was generated in vitro. Unmodified linear RNA was transcribed in vitro from a DNA template. Transcribed RNA was purified with an RNA cleanup kit (New England Biolabs, T2050), treated with RNA 5'phosphohydrolase (RppH) (New England Biolabs, M0356) following the manufacturer's instructions, and purified again with an RNA purification column. RppH treated linear RNA circularized using a splint DNA and T4 RNA ligase 2 (New England Biolabs, M0239). Circular RNA was then Urea-PAGE purified, eluted in a buffer (0.5 M Sodium Acetate, 0.1% SDS, 1 mM EDTA), ethanol precipitated and resuspended in RNA storage solution (ThermoFisher Scientific, cat# AM7000). In this example, circular RNA was also HPLC-purified.

In this example, linear mRNA was designed with an ORF encoding a Nano Luciferase (NLuc). In this example, modified linear mRNA was made in-house by *in vitro* transcription. In this example, linear RNA was fully substituted with Pseudo-Uridine and 5-Methyl-C, capped with CleanCap^{™} AG, included 5' and 3' human alpha-globin UTRs, and is polyadenylated.

RNA was diluted in PBS/Glucose (4.5 g/L) to afford 3.5 pmoles in 10 uL of solution. This RNA solution was then added to TransIT (Mirus Bio, MIR5700) (10 uL), Boost (Mirus Bio, MIR5700) (5 uL), and PBS/Glucose (4.5 g/L) (25 uL). The total sample volume for each sample was 50 uL, and total RNA for each sample was 3.5 pmoles. All reagents are brought to room temperature prior to mixing and mixtures are prepared immediately prior to use.

At time = 0, 50 µL dose of each sample was applied topically to the ear of a BALB/c mouse dropwise using a pipet tip. As a negative control, an untreated mouse was used. Samples were dried by exposing the ears briefly to a heat lamp and fan in a sterilized hood. Mice were place back in cages under normal conditions.

To determine RNA persistence in tissue, tissue samples were analyzed for RNA at varying timepoints after delivery using RT-qPCR. At 6 hours, 1, 3, and 12 days post-administration, a 2 mm ear punch was taken from each animal and stored in RNAlater solution (ThermoFisher Scientific, cat# AM7020). Total RNA was isolated from ear punches using trizol extraction. The aqueous-phase was precipitated with isopropanol and the pellet was washed with 70% ETOH as per manufacturer's instructions. cDNA was synthesized from the total RNA and RT-PCR was performed on cDNA templates using primers specific to the NLuc ORF. All samples were assayed in triplicate on the Bio-rad CFX384 Thermal Cycler. RNA levels were then relativized to actin and the untreated negative control.

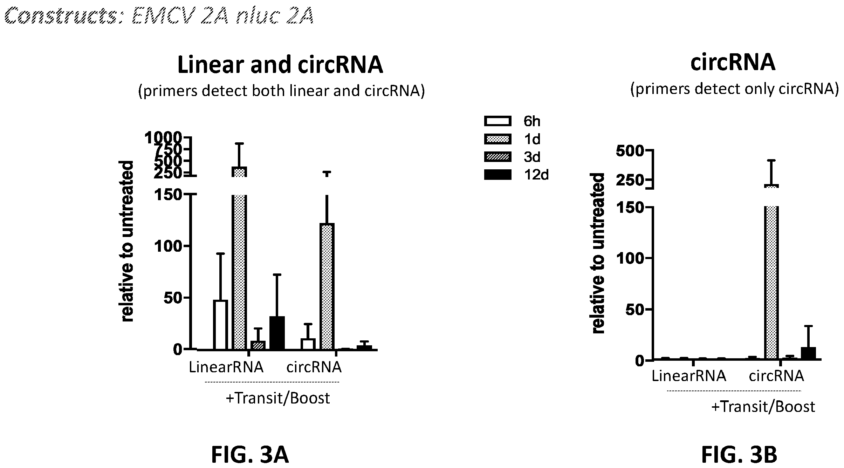

Circular RNA and linear RNA were detected in tissue samples at 6 hours, 1, 3 and 12 days after topical administration and showed greater signal than the vehicle only control (**FIG. 3A and FIG. 3B**).

This Example demonstrates that circular RNA and linear RNA are successfully delivered via topical administration to the tissue when delivered with TransIT and persists in tissue over prolonged periods of time.

### Example 8: Topical administration of modified linear RNA formulated with dimethyl sulfoxide (DMSO) gel in vivo

This Example demonstrates the ability to deliver linear RNA *in vivo* by topical administration when formulated with DMSO gel.

For this Example, RNAs included an ORF encoding Gaussia Luciferase (GLuc).

In this example, modified linear RNA was custom synthesized by Trilink Biotechnologies and included all the motifs listed above. In this example, RNA was fully substituted with Pseudo-Uridine and 5-Methyl-C, capped with CleanCap^{™} AG and is polyadenylated (120A). DMSO Medi Gel (21^{st} Century Chemical Inc.) was commercially available.

RNA was diluted to a concentration of 1 pmole/µL in RNA storage solution. 5 pmole of RNA was combined with 19 µL of DMSO Medi Gel (21^{st} Century Chemical Inc.) and 1 µL of Rnasin Plus RNase Inhibitor (Promega) for a total of 25 µL per application. Formulation without RNA was used as a control.

At time = 0, a 25 µL dose of each sample was applied topically to the ear of a mouse using a pipet tip. Samples were dried by exposing the ears briefly to a heat lamp and fan in a sterilized hood. Mice were place back in cages under normal conditions.

To determine RNA expression in tissue, tissue samples were analyzed for RNA expression at varying timepoints after topical delivery. Ear punches were taken from the mouse at 24 and 48 hours after delivery. Tissue samples were placed in 1× Luciferase Cell Lysis Buffer (Thermo Scientific) on ice for 30 minutes and then frozen.

The activity of Gaussia Luciferase was tested using a Gaussia Luciferase Activity assay (Thermo Scientific Pierce). Samples were thawed and spun briefly to remove any tissue debris. 20 µL of the buffer solution was added to a 96 well plate (Corning 3990). In brief, 1× coelenterazine substrate was added to each well. Plates were read immediately after substrate addition and mixing in a luminometer instrument (Promega).

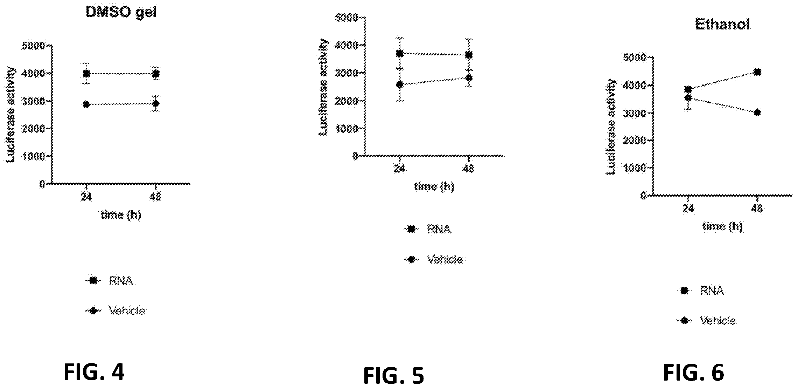

Gaussia Luciferase activity was detected in tissue samples at 24 and 48 hours after topical application and was observed to be higher than the vehicle only control (**FIG. 4**).

This Example demonstrated that linear RNA was successfully delivered via topical administration when formulated with DMSO Medi Gel (21^{st} Century Chemical Inc.) and was able to express functional protein detectable in tissue for prolonged periods of time.

### Example 9: Topical administration of modified linear RNA formulated with cream-based ointment in vivo

This Example demonstrates the ability to deliver linear RNA *in vivo* by topical administration when formulated with the cream-based ointment Johnson&Johnson's baby lotion.

For this Example, RNAs included an ORF encoding Gaussia Luciferase (GLuc).

In this example, modified linear RNA was custom synthesized by Trilink Biotechnologies and included all the motifs listed above. In this example, RNA was fully substituted with Pseudo-Uridine and 5-Methyl-C, capped with CleanCap^{™} AG and is polyadenylated (120A). Baby lotion (Johnson & Johnson) was available commercially.

RNA was diluted to a concentration of 1 pmole/µL. 5 pmole of RNA was combined with 19 µL of Johnson's baby lotion (no fragrance; Johnson & Johnson) and 1 µL of Rnasin Plus RNase Inhibitor (Promega) for a total of 25 µL per application. Formulation without RNA was used as a control.

At time = 0, a 25 µL dose of each sample was applied topically to the ear of a mouse using a pipet tip. Samples were dried by exposing the ears briefly to a heat lamp and fan in a sterilized hood. Mice were place back in cages under normal conditions.

To determine RNA expression in tissue, tissue samples were analyzed for RNA expression at varying timepoints after topical delivery. Ear punches were taken from the mouse at 24 and 48 hours after delivery. Tissue samples were placed in 1× Luciferase Cell Lysis Buffer (Thermo Scientific) on ice for 30 minutes and then frozen.

The activity of Gaussia Luciferase was tested using a Gaussia Luciferase Activity assay (Thermo Scientific Pierce). Samples were thawed and spun briefly to remove any tissue debris. 20 µL of the buffer solution was added to a 96 well plate (Corning 3990). In brief, 1× coelenterazine substrate was added to each well. Plates were read immediately after substrate addition and mixing in a luminometer instrument (Promega).

Gaussia Luciferase activity was detected in tissue samples at 24 and 48 hours after topical application and was observed to be higher than the vehicle only control (**FIG. 5**).

This Example demonstrated that linear RNA was successfully delivered via topical administration when formulated with a cream-based ointment and was able to express functional protein detectable in tissue for prolonged periods of time.

### Example 10: Topical administration of modified linear RNA using ethanol in vivo

This Example demonstrates the ability to deliver linear RNA *in vivo* by topical administration using ethanol.

For this Example, RNAs included an ORF encoding Gaussia Luciferase (GLuc).

In this example, modified linear RNA was custom synthesized by Trilink Biotechnologies and included all the motifs listed above. In this example, RNA was fully substituted with Pseudo-Uridine and 5-Methyl-C, capped with CleanCap^{™} AG and is polyadenylated (120A). Ethanol (Sigma Aldrich) was available commercially.

RNA was diluted to a concentration of 1 pmole/µL with RNA storage solution. 5 pmole of RNA was combined with 19 µL of ethanol and 1 µL of Rnasin Plus RNase Inhibitor (Promega) for a total of 25 µL per application. Vehicle only control was similarly prepared but did not contain RNA.

At time = 0, a 25 µL dose of each sample was applied topically to the ear of a mouse using a pipet tip. Samples were dried by exposing the ears briefly to a heat lamp and fan in a sterilized hood. Mice were place back in cages under normal conditions.

To determine RNA expression in tissue, tissue samples were analyzed for RNA expression at varying timepoints after topical delivery. Ear punches were taken from the mouse at 24 and 48 hours after delivery. Tissue samples were placed in 1× Luciferase Cell Lysis Buffer (Thermo Scientific) on ice for 30 minutes and then frozen.

The activity of Gaussia Luciferase was tested using a Gaussia Luciferase Activity assay (Thermo Scientific Pierce). Samples were thawed and spun briefly to remove any tissue debris. 20 µL of the buffer solution was added to a 96 well plate (Corning 3990). In brief, 1× coelenterazine substrate was added to each well. Plates were read immediately after substrate addition and mixing in a luminometer instrument (Promega).

Gaussia Luciferase activity was detected in tissue samples at 24 and 48 hours after topical application and was observed to be higher than the vehicle only control (**FIG. 6**).

This Example demonstrated that linear RNA was successfully delivered via topical administration with ethanol and was able to express functional protein detectable in tissue for prolonged periods of time.

### Example 11: Topical administration of circular RNA results in RNA delivery to tissue

This Example demonstrates the ability to delivery circular RNA to cells and tissues via topical administration *in vivo.*

In this example, circular RNA was designed with an ORF encoding an erythropoietin protein (EPO).

The circular RNA was generated *in vitro.* Linear RNA was transcribed in vitro from a DNA template including all the motifs listed above, as well as a T7 RNA polymerase promoter to drive transcription. In this example, Cy5-UTP is used to generate Cy5-labeled RNA. Transcribed RNA was purified with an RNA cleanup kit (New England Biolabs, T2050), treated with RNA 5'phosphohydrolase (RppH) (New England Biolabs, M0356) following the manufacturer's instructions, and purified again with an RNA purification column. RppH treated linear RNA was circularized using a splint DNA and T4 RNA ligase 2 (New England Biolabs, M0239). Circular RNA was Urea-PAGE purified, eluted in a buffer (0.5 M Sodium Acetate, 0.1% SDS, 1 mM EDTA), ethanol precipitated and resuspended in RNA storage solution (ThermoFisher Scientific, cat# AM7000).

Circular RNA was diluted in PBS/glucose (4.5g/L) with 5% ethanol such that total sample volume for each sample was 25 uL, and total RNA for each sample was 12 picomoles. All reagents were brought to room temperature prior to mixing and mixtures were prepared immediately prior to use.

At time = 0, the ear of the mouse was wiped with an isopropyl alcohol wipe, dried and a 25 µL dose of each sample was applied topically to the ear of a BALB/c mouse dropwise using a pipet tip. Samples were dried by exposing the ears briefly to a heat lamp and fan in a sterilized hood. Mice were place back in cages under normal conditions.

To determine circular RNA delivery to tissue, tissue samples were analyzed by fluorescence microscopy at varying timepoints after administration. At 6 hours, 1 day, and 3 days post administration, a 2 mm ear punch was taken from each animal and stored in ice-cold PBS. Tissue samples were then observed under an EVOS II fluorescent microscope. Images were then quantified for fluorescence using ImageJ.

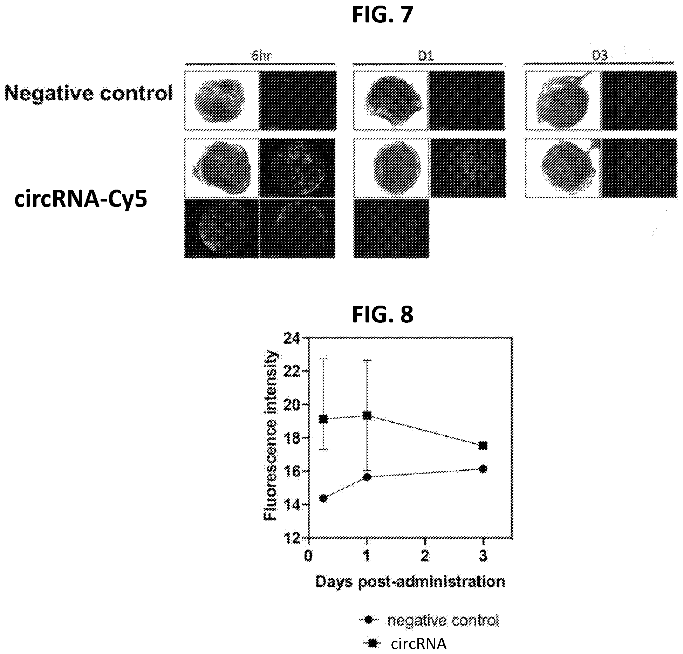

Cy5 signal was detected in tissue samples at 6 hours, 1 and 3 days after topical administration and showed greater signal than the negative control which did not show any fluorescence **(****FIG. 7** and **FIG. 8****)**. This indicates that circular RNA is successfully delivered to the tissue.

This Example demonstrates that circular RNA is successfully delivered via topical administration when the skin and persists in tissue over prolonged periods of time.

### Example 12: Topical administration of mRNA results in RNA delivery to tissue

This Example demonstrates the ability to delivery mRNA to cells and tissues via topical administration in vivo.

In this example, mRNA was designed with an ORF encoding a green fluorescent protein (eGFP). In this example, modified linear mRNA was custom synthesized by Trilink Biotechnologies and included all the motifs listed above. In this example, RNA was fully substituted with Pseudo-Uridine and 5-Methyl-C, capped with CleanCap^{™} AG, included 5' and 3' human alpha-globin UTRs, and is polyadenylated. mRNA included a Cy5 fluorophore label, covalently bound at the 3' end.

mRNA was diluted in PBS/glucose (4.5g/L) with 5% ethanol such that total sample volume for each sample was 25 uL, and total RNA for each sample was 12 picomoles. All reagents were brought to room temperature prior to mixing and mixtures were prepared immediately prior to use.

At time = 0, the ear of the mouse was wiped with an isopropyl alcohol wipe, dried and a 25 µL dose of each sample was applied topically to the ear of a BALB/c mouse dropwise using a pipet tip. Samples were dried by exposing the ears briefly to a heat lamp and fan in a sterilized hood. Mice were place back in cages under normal conditions.

To determine RNA delivery to tissue, tissue samples were analyzed by fluorescence microscopy at varying timepoints after administration. At 6 hours, 1 day, and 3 days post administration, a 2 mm ear punch was taken from each animal and stored in ice-cold PBS. Tissue samples were then observed under an EVOS II fluorescent microscope. Images were then quantified for fluorescence using ImageJ.

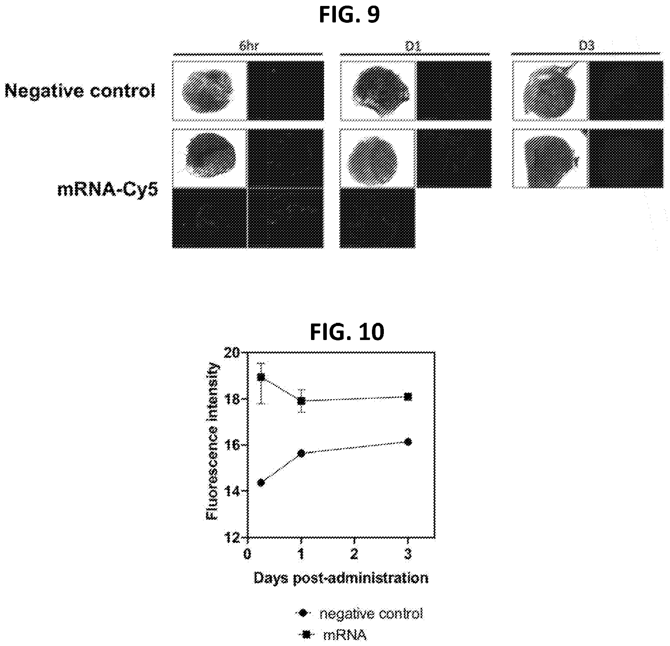

Cy5 signal was detected in tissue samples at 6 hours, 1 day, and 3 days after topical administration and showed greater signal than the negative control which did not show any fluorescence (**FIG. 9** and **FIG. 10**). This indicates that mRNA is successfully delivered to the tissue.

This Example demonstrates that mRNA is successfully delivered via topical administration when the skin and persists in tissue over prolonged periods of time.

### Example 13: Topical administration of mRNA results in RNA delivery to tissue when tissue is wiped with an ethanol wipe prior to application

This Example demonstrates the ability to delivery mRNA to cells and tissues via topical administration *in vivo.*

In this example, mRNA was designed with an ORF encoding a Nano Luciferase (NLuc). In this example, modified linear mRNA was made in-house by in vitro transcription. In this example, RNA was fully substituted with Pseudo-Uridine and 5-Methyl-C, capped with CleanCap^{™} AG, included 5' and 3' human alpha-globin UTRs, and is polyadenylated.

RNA was then diluted in PBS only, or with PBS and 10% (v/v) ethanol, such that total sample volume for each sample was 35 uL, and total RNA for each sample was 20 picomoles. As negative controls, vehicle only controls were prepared as described above but without RNA. All reagents are brought to room temperature prior to mixing and mixtures are prepared immediately prior to use.

At time = 0, the ear of the mouse was wiped with a cotton swab dipped in 70% ethanol, dried and a 35 µL dose of each sample was applied topically to the ear of a BALB/c mouse dropwise using a pipet tip. Samples were dried by exposing the ears briefly to a heat lamp and fan in a sterilized hood. Mice were place back in cages under normal conditions.

To determine RNA persistence in tissue, tissue samples were analyzed for RNA at varying timepoints after delivery using RT-qPCR. At 1 and 4 days post-administration, a 2 mm ear punch was taken from each animal and stored in RNAlater solution (ThermoFisher Scientific, cat# AM7020). Total RNA was isolated from ear punches by snap-cooling and homogenizing the tissue in liquid nitrogen with a glass mortar and pestle followed by trizol extraction (ThermoFisher Scientific cat # 15596026). The aqueous-phase was precipitated with isopropanol and the pellet was washed with 70% ETOH as per manufacturer's instructions. cDNA was synthesized from the total RNA using Superscript IV (Thermo Scientific, cat# 11766500). RT-PCR was performed on cDNA templates using iTaq^{™} Universal SYBR^{®}Green Supermix (Bio-rad, catalog #1725124) and primers specific to the NLuc ORF (F: CCGTATGAAGGTCTGAGCGG (SEQ ID NO: 12), R: CAGTGTGCCATAGTGCAGGA (SEQ ID NO: 13)). All samples were assayed in triplicate on the Bio-rad CFX384 Thermal Cycler. RNA levels were then relativized to housekeeping gene (28s).

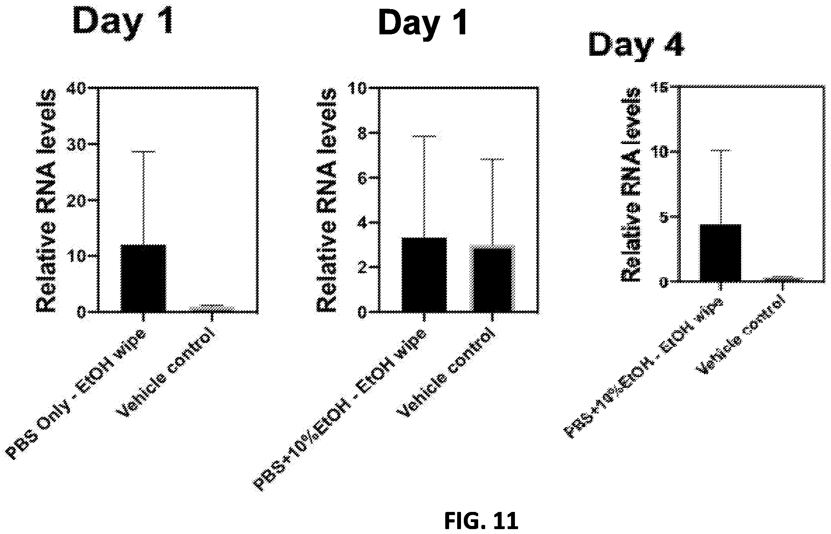

mRNA was detected in tissue samples at 1 day for mRNA in PBS only, and at 1 and 4 days for mRNA in PBS+10%EtOH after topical administration and showed greater signal than the negative controls (**FIG. 11**).

This Example demonstrates that mRNA is successfully delivered via topical administration when the skin is wiped with an ethanol wipe prior to administration and persists in tissue over prolonged periods of time.

### Example 14: Topical administration of mRNA results in RNA delivery to tissue when tissue is wiped with an isopropyl alcohol wipe prior to application

This Example demonstrates the ability to delivery mRNA to cells and tissues via topical administration in vivo.

In this example, mRNA was designed with an ORF encoding a Nano Luciferase (NLuc). In this example, modified linear mRNA was made in-house by *in vitro* transcription. In this example, RNA was fully substituted with Pseudo-Uridine and 5-Methyl-C, capped with CleanCap^{™} AG, included 5' and 3' human alpha-globin UTRs, and is polyadenylated.

RNA was then diluted in PBS only such that total sample volume for each sample was 35 uL, and total RNA for each sample was 20 picomoles. As negative controls, vehicle only controls were prepared as described above but without RNA. All reagents are brought to room temperature prior to mixing and mixtures are prepared immediately prior to use.

At time = 0, the ear of the mouse was wiped with a commercial isopropyl alcohol wipe (CVS, 297584), dried and a 35 µL dose of each sample was applied topically to the ear of a BALB/c mouse dropwise using a pipet tip. Samples were dried by exposing the ears briefly to a heat lamp and fan in a sterilized hood. Mice were place back in cages under normal conditions.

To determine RNA persistence in tissue, tissue samples were analyzed for RNA at varying timepoints after delivery using RT-qPCR. At 1 and 4 days post-administration, a 2 mm ear punch was taken from each animal and stored in RNAlater solution (ThermoFisher Scientific, cat# AM7020). Total RNA was isolated from ear punches by snap-cooling and homogenizing the tissue in liquid nitrogen with a glass mortar and pestle followed by trizol extraction (ThermoFisher Scientific cat # 15596026). The aqueous-phase was precipitated with isopropanol and the pellet was washed with 70% ETOH as per manufacturer's instructions. cDNA was synthesized from the total RNA using Superscript IV (Thermo Scientific, cat# 11766500). RT-PCR was performed on cDNA templates using iTaq^{™} Universal SYBR^{®}Green Supermix (Bio-rad, catalog #1725124) and primers specific to the NLuc ORF (F: CCGTATGAAGGTCTGAGCGG (SEQ ID NO: 12), R: CAGTGTGCCATAGTGCAGGA (SEQ ID NO: 13)). All samples were assayed in triplicate on the Bio-rad CFX384 Thermal Cycler. RNA levels were then relativized to housekeeping gene (28s).

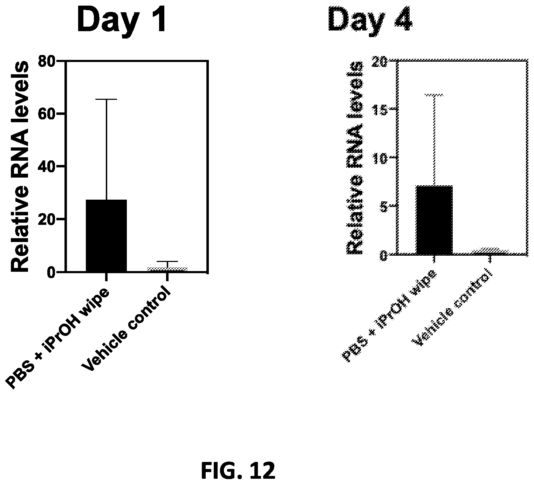

mRNA was detected in tissue samples at 1 and 4 days after topical administration and showed greater signal than the negative controls (**FIG. 12**).

This Example demonstrates that mRNA is successfully delivered via topical administration when the skin is wiped with an isopropyl alcohol wipe prior to administration and persists in tissue over prolonged periods of time.

### Example 15: Topical administration of circular RNA results in RNA delivery to tissue when tissue is wiped with an ethanol wipe prior to application

This Example demonstrates the ability to delivery unmodified circular RNA to cells and tissues via topical administration in vivo.

In this example, circular RNA was designed with an IRES and ORF encoding Nanoluciferase *(NLuc).*

The circular RNA was generated *in vitro.* Unmodified linear RNA was transcribed *in vitro* from a DNA template including all the motifs listed above, as well as a T7 RNA polymerase promoter to drive transcription. Transcribed RNA was purified with an RNA cleanup kit (New England Biolabs, T2050), treated with RNA 5'phosphohydrolase (RppH) (New England Biolabs, M0356) following the manufacturer's instructions, and purified again with an RNA purification column. RppH treated linear RNA was circularized using a splint DNA (5'- TTTTTCGGCTATTCCCAATAGCCGTTTTG-3' (SEQ ID NO: 11)) and T4 RNA ligase 2 (New England Biolabs, M0239). Circular RNA was Urea-PAGE purified, eluted in a buffer (0.5 M Sodium Acetate, 0.1% SDS, 1 mM EDTA), ethanol precipitated and resuspended in RNA storage solution (ThermoFisher Scientific, cat# AM7000).

RNA was then diluted in either PBS only, or PBS with 10% (v/v) ethanol, such that total sample volume for each sample was 35 uL, and total RNA for each sample was 20 pmoles. As negative controls, vehicle only controls were prepared as described above but without RNA. All reagents are brought to room temperature prior to mixing and mixtures are prepared immediately prior to use.

At time = 0, the ear of the mouse was wiped with an ethanol wipe, dried and a 35 µL dose of each sample was applied topically to the ear of a BALB/c mouse dropwise using a pipet tip. Samples were dried by exposing the ears briefly to a heat lamp and fan in a sterilized hood. Mice were place back in cages under normal conditions.

To determine RNA persistence in tissue, tissue samples were analyzed for RNA at varying timepoints after delivery using RT-qPCR. At 1 and 4 days post-administration, a 2 mm ear punch was taken from each animal and stored in RNAlater solution (ThermoFisher Scientific, cat# AM7020). Total RNA was isolated from ear punches by snap-cooling and homogenizing the tissue in liquid nitrogen with a glass mortar and pestle followed by trizol extraction (ThermoFisher Scientific cat # 15596026). The aqueous-phase was precipitated with isopropanol and the pellet was washed with 70% ETOH as per manufacturer's instructions. cDNA was synthesized from the total RNA using Superscript IV (Thermo Scientific, cat# 11766500). RT-PCR was performed on cDNA templates using iTaq^{™} Universal SYBR^{®}Green Supermix (Bio-rad, catalog #1725124) and primers specific to the NLuc ORF (F: CCGTATGAAGGTCTGAGCGG (SEQ ID NO: 12), R: CAGTGTGCCATAGTGCAGGA (SEQ ID NO: 13)). All samples were assayed in triplicate on the Bio-rad CFX384 Thermal Cycler. RNA levels were then relativized to housekeeping gene (28s).

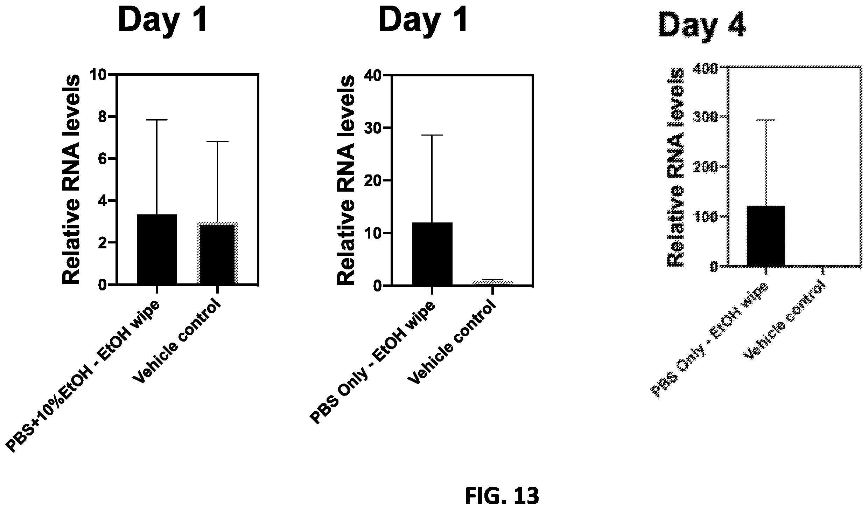

Circular RNA was detected in tissue samples at 1 and 4 days for circular RNA in PBS only, and at 1 days for circular RNA in PBS+10%EtOH after topical administration following wiping the skin with an ethanol wipe and showed greater signal than the relevant vehicle only control (**FIG. 13**).

This Example demonstrates that circular RNA is successfully delivered via topical administration when the skin is wiped with an ethanol wipe prior to administration and persists in tissue over prolonged periods of time.

### Example 16: Topical administration of circular RNA results in RNA delivery to tissue

This Example demonstrates the ability to delivery unmodified circular RNA to cells and tissues via topical administration in vivo.

In this example, circular RNA was designed with an IRES and ORF encoding Nanoluciferase (NLuc).

The circular RNA was generated in vitro. Unmodified linear RNA was transcribed in vitro from a DNA template including all the motifs listed above, as well as a T7 RNA polymerase promoter to drive transcription. Transcribed RNA was purified with an RNA cleanup kit (New England Biolabs, T2050), treated with RNA 5'phosphohydrolase (RppH) (New England Biolabs, M0356) following the manufacturer's instructions, and purified again with an RNA purification column. RppH treated linear RNA was circularized using a splint DNA (5'-TTTTTCGGCTATTCCCAATAGCCGTTTTG-3' (SEQ ID NO: 11)) and T4 RNA ligase 2 (New England Biolabs, M0239). Circular RNA was Urea-PAGE purified, eluted in a buffer (0.5 M Sodium Acetate, 0.1% SDS, 1 mM EDTA), ethanol precipitated and resuspended in RNA storage solution (ThermoFisher Scientific, cat# AM7000).

RNA was then diluted in PBS with 10% (v/v) ethanol, such that total sample volume for each sample was 35 uL, and total RNA for each sample was 20 pmoles. As negative controls, vehicle only controls were prepared as described above but without RNA. All reagents were brought to room temperature prior to mixing and mixtures were prepared immediately prior to use.

At time = 0, a 35 µL dose of each sample was applied topically to the ear of a BALB/c mouse dropwise using a pipet tip. Samples were dried by exposing the ears briefly to a heat lamp and fan in a sterilized hood. Mice were place back in cages under normal conditions.

To determine RNA persistence in tissue, tissue samples were analyzed for RNA at varying timepoints after delivery using RT-qPCR. At 1 day and 4 days post-administration, a 2 mm ear punch was taken from each animal and stored in RNAlater solution (ThermoFisher Scientific, cat# AM7020). Total RNA was isolated from ear punches by snap-cooling and homogenizing the tissue in liquid nitrogen with a glass mortar and pestle followed by trizol extraction (ThermoFisher Scientific cat # 15596026). The aqueous-phase was precipitated with isopropanol and the pellet was washed with 70% ETOH as per manufacturer's instructions. cDNA was synthesized from the total RNA using Superscript IV (Thermo Scientific, cat# 11766500). RT-PCR was performed on cDNA templates using iTaq^{™} Universal SYBR^{®}Green Supermix (Bio-rad, catalog #1725124) and primers specific to the NLuc ORF (F: CCGTATGAAGGTCTGAGCGG (SEQ ID NO: 12), R: CAGTGTGCCATAGTGCAGGA (SEQ ID NO: 13)). All samples were assayed in triplicate on the Bio-rad CFX384 Thermal Cycler. RNA levels were then relativized to housekeeping gene (28s).

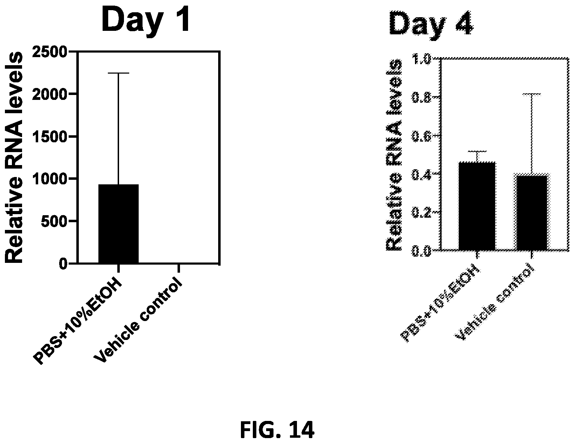

Circular RNA was detected in tissue samples at 1 day and 4 days after topical administration and showed greater signal than the relevant negative control (**FIG. 14**).

This Example demonstrates that circular RNA is successfully delivered to tissues via topical administration to the skin and persists in tissue over prolonged periods of time.

### Example 17: Topical administration of circular RNA results in RNA delivery to tissue

This Example demonstrates the ability to delivery unmodified circular RNA to cells and tissues via topical administration in vivo.

In this example, circular RNA was designed with an IRES and ORF encoding Nanoluciferase *(NLuc).*

The circular RNA was generated *in vitro.* Unmodified linear RNA was transcribed *in vitro* from a DNA template including all the motifs listed above, as well as a T7 RNA polymerase promoter to drive transcription. Transcribed RNA was purified with an RNA cleanup kit (New England Biolabs, T2050), treated with RNA 5'phosphohydrolase (RppH) (New England Biolabs, M0356) following the manufacturer's instructions, and purified again with an RNA purification column. RppH treated linear RNA was circularized using a splint DNA (5'-TTTTTCGGCTATTCCCAATAGCCGTTTTG-3' (SEQ ID NO: 11)) and T4 RNA ligase 2 (New England Biolabs, M0239). Circular RNA was Urea-PAGE purified, eluted in a buffer (0.5 M Sodium Acetate, 0.1% SDS, 1 mM EDTA), ethanol precipitated and resuspended in RNA storage solution (ThermoFisher Scientific, cat# AM7000).

RNA was then diluted in PBS with 10% (v/v) isopropyl alcohol, such that total sample volume for each sample was 35 uL, and total RNA for each sample was 20 pmoles. As negative controls, vehicle only controls were prepared as described above but without RNA. All reagents were brought to room temperature prior to mixing and mixtures were prepared immediately prior to use.

At time = 0, a 35 µL dose of each sample was applied topically to the ear of a BALB/c mouse dropwise using a pipet tip. Samples were dried by exposing the ears briefly to a heat lamp and fan in a sterilized hood. Mice were place back in cages under normal conditions.

To determine RNA persistence in tissue, tissue samples were analyzed for RNA at varying timepoints after delivery using RT-qPCR. At 1 day and 4 days post-administration, a 2 mm ear punch was taken from each animal and stored in RNAlater solution (ThermoFisher Scientific, cat# AM7020). Total RNA was isolated from ear punches by snap-cooling and homogenizing the tissue in liquid nitrogen with a glass mortar and pestle followed by trizol extraction (ThermoFisher Scientific cat # 15596026). The aqueous-phase was precipitated with isopropanol and the pellet was washed with 70% ETOH as per manufacturer's instructions. cDNA was synthesized from the total RNA using Superscript IV (Thermo Scientific, cat# 11766500). RT-PCR was performed on cDNA templates using iTaq^{™} Universal SYBR^{®}Green Supermix (Bio-rad, catalog #1725124) and primers specific to the NLuc ORF (F: CCGTATGAAGGTCTGAGCGG (SEQ ID NO: 12), R: CAGTGTGCCATAGTGCAGGA (SEQ ID NO: 13)). All samples were assayed in triplicate on the Bio-rad CFX384 Thermal Cycler. RNA levels were then relativized to housekeeping gene (28s).

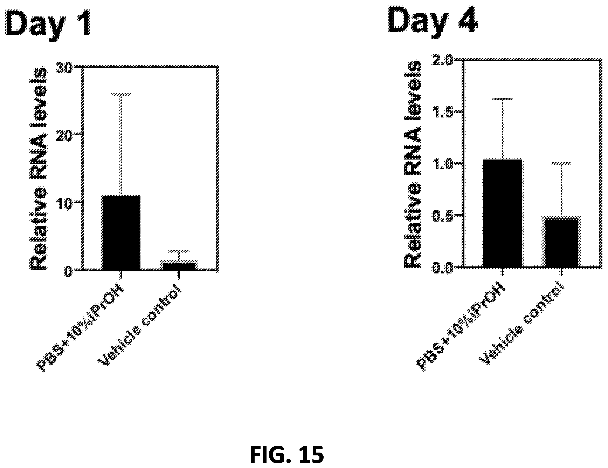

Circular RNA was detected in tissue samples at 1 day and 4 days after topical administration and showed greater signal than the relevant negative control (**FIG. 15**).

This Example demonstrates that endless RNA is successfully delivered to tissues via topical administration to the skin and persists in tissue over prolonged periods of time.

### Example 18: Topical administration of circular RNA results in RNA delivery to tissue when tissue is wiped with an isopropyl alcohol wipe prior to application

This Example demonstrates the ability to delivery unmodified circular RNA to cells and tissues via topical administration in vivo.

In this example, circular RNA was designed with an IRES and ORF encoding Nanoluciferase (NLuc).

The circular RNA was generated *in vitro.* Unmodified linear RNA was transcribed *in vitro* from a DNA template including all the motifs listed above, as well as a T7 RNA polymerase promoter to drive transcription. Transcribed RNA was purified with an RNA cleanup kit (New England Biolabs, T2050), treated with RNA 5'phosphohydrolase (RppH) (New England Biolabs, M0356) following the manufacturer's instructions, and purified again with an RNA purification column. RppH treated linear RNA circularized using a splint DNA (5'- TTTTTCGGCTATTCCCAATAGCCGTTTTG-3' (SEQ ID NO: 11)) and T4 RNA ligase 2 (New England Biolabs, M0239). Circular RNA Urea-PAGE purified, eluted in a buffer (0.5 M Sodium Acetate, 0.1% SDS, 1 mM EDTA), ethanol precipitated and resuspended in RNA storage solution (ThermoFisher Scientific, cat# AM7000).

RNA was then diluted in PBS only such that total sample volume for each sample was 35 uL, and total RNA for each sample was 20 pmoles. As negative controls, vehicle only controls were prepared as described above but without RNA. All reagents are brought to room temperature prior to mixing and mixtures are prepared immediately prior to use.

At time = 0, the ear of the mouse was wiped with an isopropyl alcohol wipe (CVS, 297584), dried and a 35 µL dose of each sample was applied topically to the ear of a BALB/c mouse dropwise using a pipet tip. As a negative control, an isopropyl alcohol wipe alone was used. Samples were dried by exposing the ears briefly to a heat lamp and fan in a sterilized hood. Mice were place back in cages under normal conditions.

To determine RNA persistence in tissue, tissue samples were analyzed for RNA at varying timepoints after delivery using RT-qPCR. At 1 day and 4 days post-administration, a 2 mm ear punch was taken from each animal and stored in RNAlater solution (ThermoFisher Scientific, cat# AM7020). Total RNA was isolated from ear punches by snap-cooling and homogenizing the tissue in liquid nitrogen with a glass mortar and pestle followed by trizol extraction (ThermoFisher Scientific cat # 15596026). The aqueous-phase was precipitated with isopropanol and the pellet was washed with 70% ETOH as per manufacturer's instructions. cDNA was synthesized from the total RNA using Superscript IV (Thermo Scientific, cat# 11766500). RT-PCR was performed on cDNA templates using iTaq^{™} Universal SYBR^{®}Green Supermix (Bio-rad, catalog #1725124) and primers specific to the NLuc ORF (F: CCGTATGAAGGTCTGAGCGG (SEQ ID NO: 12), R: CAGTGTGCCATAGTGCAGGA (SEQ ID NO: 13)). All samples were assayed in triplicate on the Bio-rad CFX384 Thermal Cycler. RNA levels were then relativized to housekeeping gene (28s).

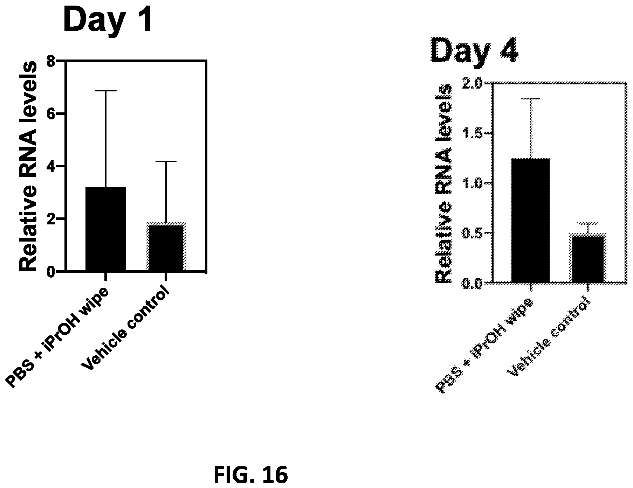

Circular RNA was detected in tissue samples at 1 day and 4 days after topical administration and showed greater signal than the vehicle only control (**FIG. 16**).

This Example demonstrates that endless RNA is successfully delivered via topical administration to the tissue after wiping the skin with an isopropyl alcohol wipe and persists in tissue over prolonged periods of time.

### Example 19: Topical administration of linear mRNA results in RNA delivery to tissue

This Example demonstrates the ability to delivery mRNA to cells and tissues via topical administration in vivo.

In this example, mRNA was designed with an ORF encoding a Nano Luciferase (NLuc). In this example, modified linear mRNA was made in-house by *in vitro* transcription. In this example, RNA was fully substituted with Pseudo-Uridine and 5-Methyl-C, capped with CleanCap^{™} AG, included 5' and 3' human alpha-globin UTRs, and is polyadenylated.

RNA was diluted in (1) PBS only, (2) PBS with 10% (v/v) ethanol, (3) PBS with 10% (v/v) isopropyl alcohol, such that total sample volume for each sample was 35 uL, and total RNA for each sample was 20 pmoles. As negative controls, vehicle only controls were prepared as described above but without RNA. All reagents are brought to room temperature prior to mixing and mixtures are prepared immediately prior to use.

At time = 0, a 35 µL dose of each sample was applied topically to the ear of a BALB/c mouse dropwise using a pipet tip. Samples were dried by exposing the ears briefly to a heat lamp and fan in a sterilized hood. Mice were place back in cages under normal conditions.

To determine RNA persistence in tissue, tissue samples were analyzed for RNA at varying timepoints after delivery using RT-qPCR. At 1 day and 4 days post-administration, a 2 mm ear punch was taken from each animal and stored in RNAlater solution (ThermoFisher Scientific, cat# AM7020). Total RNA was isolated from ear punches by snap-cooling and homogenizing the tissue in liquid nitrogen with a glass mortar and pestle followed by trizol extraction (ThermoFisher Scientific cat # 15596026). The aqueous-phase was precipitated with isopropanol and the pellet was washed with 70% ETOH as per manufacturer's instructions. cDNA was synthesized from the total RNA using Superscript IV (Thermo Scientific, cat# 11766500). RT-PCR was performed on cDNA templates using iTaq^{™} Universal SYBR^{®}Green Supermix (Bio-rad, catalog #1725124) and primers specific to the NLuc ORF (F: CCGTATGAAGGTCTGAGCGG (SEQ ID NO: 12), R: CAGTGTGCCATAGTGCAGGA (SEQ ID NO: 13)). All samples were assayed in triplicate on the Bio-rad CFX384 Thermal Cycler. RNA levels were then relativized to housekeeping gene (28s).

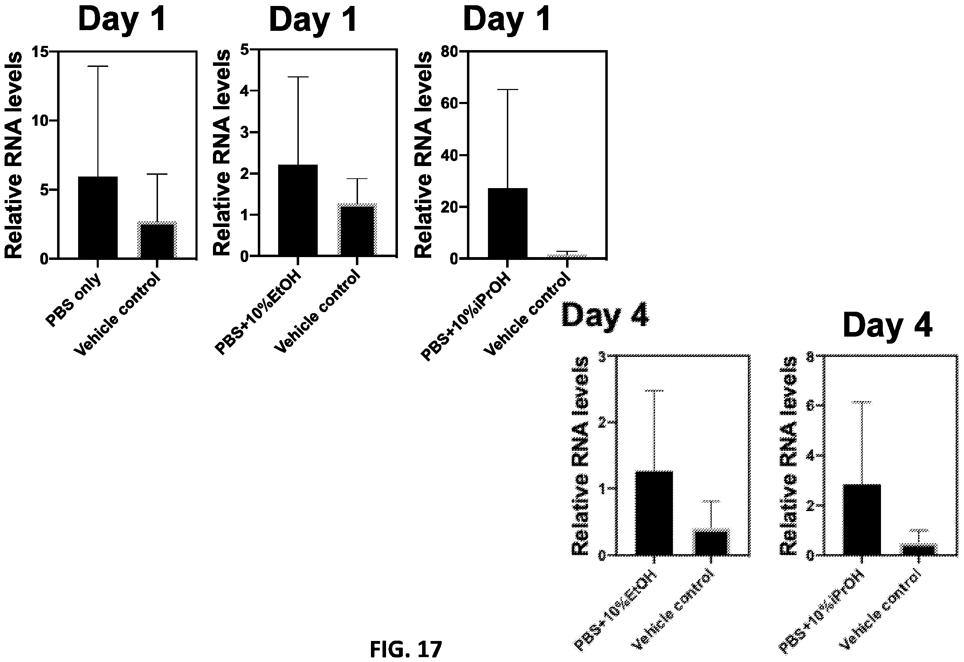

mRNA was detected in tissue samples at 1 day for mRNA in PBS only, and at 1 day and 4 days for both mRNA in PBS+10%EtOH and mRNA in PBS+10%iPrOH after topical administration and showed greater signal than the relevant negative control (FIG. 17).

This Example demonstrates that mRNA is successfully delivered to tissues via topical administration to the skin and persists in tissue over prolonged periods of time.

### Example 20: Topical administration of circular RNA results in protein expression in tissue when tissue is wiped with an ethanol wipe prior to application

This Example demonstrates the ability to deliver unmodified circular RNA to cells and tissues via topical administration *in vivo* and achieve subsequent protein expression.

In this example, circular RNA was designed with an IRES and ORF encoding Nanoluciferase (NLuc).

The circular RNA was generated in vitro. Unmodified linear RNA was transcribed *in vitro* from a DNA template including all the motifs listed above, as well as a T7 RNA polymerase promoter to drive transcription. Transcribed RNA was purified with an RNA cleanup kit (New England Biolabs, T2050), treated with RNA 5'phosphohydrolase (RppH) (New England Biolabs, M0356) following the manufacturer's instructions, and purified again with an RNA purification column. RppH treated linear RNA was circularized using a splint DNA (5'-TTTTTCGGCTATTCCCAATAGCCGTTTTG-3' (SEQ ID NO: 11)) and T4 RNA ligase 2 (New England Biolabs, M0239). Circular RNA was Urea-PAGE purified, eluted in a buffer (0.5 M Sodium Acetate, 0.1% SDS, 1 mM EDTA), ethanol precipitated and resuspended in RNA storage solution (ThermoFisher Scientific, cat# AM7000).

RNA was then diluted in PBS only such that total sample volume for each sample was 35 uL, and total RNA for each sample was 20 pmoles. Vehicle only control samples were prepared similarly but without RNA. All reagents were brought to room temperature prior to mixing and mixtures were prepared immediately prior to use.

At time = 0, the ear of the mouse was wiped with an ethanol wipe, dried and a 35 µL dose of each sample was applied topically to the ear of a BALB/c mouse dropwise using a pipet tip. Samples were dried by exposing the ears briefly to a heat lamp and fan in a sterilized hood. Mice were place back in cages under normal conditions.

To determine RNA expression in tissue, tissue samples were analyzed for NLuc activity at varying timepoints after topical delivery. Ear punches were taken from the mouse at 4 days after delivery. Each tissue sample was crushed into fragments and was placed in 50 µL of ice cold NLuc Lysis Assay Buffer with 1× Protease Inhibitor Cocktail and placed on ice. Samples were then incubated on an orbital shaker for 5 minutes at 700 rpm, and then centrifuged at room temperature to remove tissue debris. The 50 uL supernatant was then transferred to a fresh tube without disturbing the tissue pellet. 50 µL of each sample was transferred to a 96 well plate and Nano-Glo Luciferase Assay System (Promega, #N1110) assay was performed according to manufacturer's instruction. In brief, 1 uL of furimazine substrate and 49 uL of PBS were added to each well and mixed. Plates were incubated for 10 min after substrate addition and mixing and then read in a luminometer instrument (Promega).

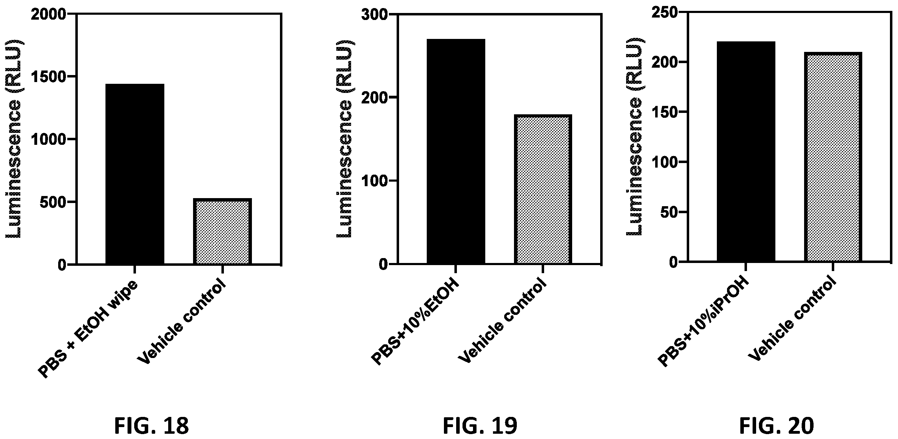

Nano Luciferase activity was detected in tissue samples at 4 days after topical administration for circular RNA in PBS only and was observed to be higher than the relevant vehicle only control (**FIG. 18**).

This Example demonstrated that circular RNA was successfully delivered via topical administration and was able to express functional protein, detectable in tissue for prolonged periods of time.

### Example 21: Topical administration of circular RNA results in protein expression in tissue

This Example demonstrates the ability to deliver unmodified circular RNA to cells and tissues via topical administration *in vivo* and achieve subsequent protein expression.

In this example, circular RNA was designed with an IRES and ORF encoding Nanoluciferase (NLuc).

The circular RNA was generated *in vitro.* Unmodified linear RNA was transcribed *in vitro* from a DNA template including all the motifs listed above, as well as a T7 RNA polymerase promoter to drive transcription. Transcribed RNA was purified with an RNA cleanup kit (New England Biolabs, T2050), treated with RNA 5'phosphohydrolase (RppH) (New England Biolabs, M0356) following the manufacturer's instructions, and purified again with an RNA purification column. RppH treated linear RNA was circularized using a splint DNA (5'-TTTTTCGGCTATTCCCAATAGCCGTTTTG-3' (SEQ ID NO: 11)) and T4 RNA ligase 2 (New England Biolabs, M0239). Circular RNA was Urea-PAGE purified, eluted in a buffer (0.5 M Sodium Acetate, 0.1% SDS, 1 mM EDTA), ethanol precipitated and resuspended in RNA storage solution (ThermoFisher Scientific, cat# AM7000).

RNA was then diluted in PBS with 10% (v/v) ethanol, such that total sample volume for each sample was 35 uL, and total RNA for each sample was 20 pmoles. As negative controls, vehicle only controls were prepared as described above but without RNA. All reagents were brought to room temperature prior to mixing and mixtures were prepared immediately prior to use.

At time = 0, a 35 µL dose of each sample was applied topically to the ear of a BALB/c mouse dropwise using a pipet tip. Samples were dried by exposing the ears briefly to a heat lamp and fan in a sterilized hood. Mice were place back in cages under normal conditions.

To determine RNA expression in tissue, tissue samples were analyzed for NLuc activity at varying timepoints after topical delivery. Ear punches were taken from the mouse at 4 days after delivery. Each tissue sample was crushed into fragments and was placed in 50 µL of ice cold NLuc Lysis Assay Buffer with 1× Protease Inhibitor Cocktail and placed on ice. Samples were then incubated on an orbital shaker for 5 minutes at 700 rpm, and then centrifuged at room temperature to remove tissue debris. The 50 uL supernatant was then transferred to a fresh tube without disturbing the tissue pellet. 50 µL of each sample was transferred to a 96 well plate and Nano-Glo Luciferase Assay System (Promega, #N1110) assay was performed according to manufacturer's instruction. In brief, 1 uL of furimazine substrate and 49 uL of PBS were added to each well and mixed. Plates were incubated for 10 min after substrate addition and mixing and then read in a luminometer instrument (Promega).

Nano Luciferase activity was detected in tissue samples at 4 days after topical administration for circular RNA in PBS with 10% ethanol (v/v) and was observed to be higher than the relevant vehicle only control (**FIG. 19**).

This Example demonstrated that circular RNA was successfully delivered via topical administration and was able to express functional protein, detectable in tissue for prolonged periods of time.

### Example 22: Topical administration of circular RNA results in protein expression in tissue

This Example demonstrates the ability to delivery unmodified circular RNA to cells and tissues via topical administration *in vivo* and achieve subsequent protein expression.

In this example, circular RNA was designed with an IRES and ORF encoding Nanoluciferase *(NLuc).*

The circular RNA was generated *in vitro.* Unmodified linear RNA was transcribed *in vitro* from a DNA template including all the motifs listed above, as well as a T7 RNA polymerase promoter to drive transcription. Transcribed RNA was purified with an RNA cleanup kit (New England Biolabs, T2050), treated with RNA 5'phosphohydrolase (RppH) (New England Biolabs, M0356) following the manufacturer's instructions, and purified again with an RNA purification column. RppH treated linear RNA will be circularized using a splint DNA (5'-TTTTTCGGCTATTCCCAATAGCCGTTTTG-3' (SEQ ID NO: 11)) and T4 RNA ligase 2 (New England Biolabs, M0239). Circular RNA was Urea-PAGE purified, eluted in a buffer (0.5 M Sodium Acetate, 0.1% SDS, 1 mM EDTA), ethanol precipitated and resuspended in RNA storage solution (ThermoFisher Scientific, cat# AM7000).

RNA was then diluted in PBS with 10% (v/v) isopropyl alcohol, such that total sample volume for each sample was 35 uL, and total RNA for each sample was 20 pmoles. As negative controls, vehicle only controls were prepared as described above but without RNA. All reagents were brought to room temperature prior to mixing and mixtures were prepared immediately prior to use.

At time = 0, a 35 µL dose of each sample was applied topically to the ear of a BALB/c mouse dropwise using a pipet tip. Samples were dried by exposing the ears briefly to a heat lamp and fan in a sterilized hood. Mice were place back in cages under normal conditions.

To determine RNA expression in tissue, tissue samples were analyzed for NLuc activity at varying timepoints after topical delivery. Ear punches were taken from the mouse at 4 days after delivery. Each tissue sample was crushed into fragments and was placed in 50 µL of ice cold NLuc Lysis Assay Buffer with 1× Protease Inhibitor Cocktail and placed on ice. Samples were then incubated on an orbital shaker for 5 minutes at 700 rpm, and then centrifuged at room temperature to remove tissue debris. The 50 uL supernatant was then transferred to a fresh tube without disturbing the tissue pellet. 50 µL of each sample was transferred to a 96 well plate and Nano-Glo Luciferase Assay System (Promega, #N1110) assay was performed according to manufacturer's instruction. In brief, 1 uL of furimazine substrate and 49 uL of PBS were added to each well and mixed. Plates were incubated for 10 min after substrate addition and mixing and then read in a luminometer instrument (Promega).

Nano Luciferase activity was detected in tissue samples at 4 days after topical administration for circular RNA in PBS with 10% isopropyl alcohol and was observed to be higher than the relevant vehicle only control (**FIG. 20**).

This Example demonstrated that circular RNA was successfully delivered via topical administration and was able to express functional protein, detectable in tissue for prolonged periods of time.

### Example 23: Topical administration of linear mRNA results in RNA delivery to tissue and subsequent protein expression

This Example demonstrates the ability to deliver mRNA to cells and tissues via topical administration *in vivo* and achieve subsequent protein expression.

In this example, mRNA was designed with an ORF encoding a Nano Luciferase (NLuc). In this example, modified linear mRNA was made in-house by *in vitro* transcription. In this example, RNA was fully substituted with Pseudo-Uridine and 5-Methyl-C, capped with CleanCap^{™} AG, included 5' and 3' human alpha-globin UTRs, and is polyadenylated.

RNA was diluted in PBS with 10% (v/v) ethanol, such that total sample volume for each sample was 35 uL, and total RNA for each sample was 20 pmoles. As negative controls, vehicle only controls were prepared as described above but without RNA. All reagents are brought to room temperature prior to mixing and mixtures are prepared immediately prior to use.

At time = 0, the ear of the mouse was wiped with an ethanol wipe, dried and a 35 µL dose of each sample was applied topically to the ear of a BALB/c mouse dropwise using a pipet tip. Samples were dried by exposing the ears briefly to a heat lamp and fan in a sterilized hood. Mice were place back in cages under normal conditions.

To determine RNA expression in tissue, tissue samples were analyzed for NLuc activity at varying timepoints after topical delivery. Ear punches were taken from the mouse at 4 days after delivery. Each tissue sample was crushed into fragments and was placed in 50 µL of ice cold NLuc Lysis Assay Buffer with 1× Protease Inhibitor Cocktail and placed on ice. Samples were then incubated on an orbital shaker for 5 minutes at 700 rpm, and then centrifuged at room temperature to remove tissue debris. The 50 uL supernatant was then transferred to a fresh tube without disturbing the tissue pellet. 50 µL of each sample was transferred to a 96 well plate and Nano-Glo Luciferase Assay System (Promega, #N1110) assay was performed according to manufacturer's instruction. In brief, 1 uL of furimazine substrate and 49 uL of PBS were added to each well and mixed. Plates were incubated for 10 min after substrate addition and mixing and then read in a luminometer instrument (Promega).

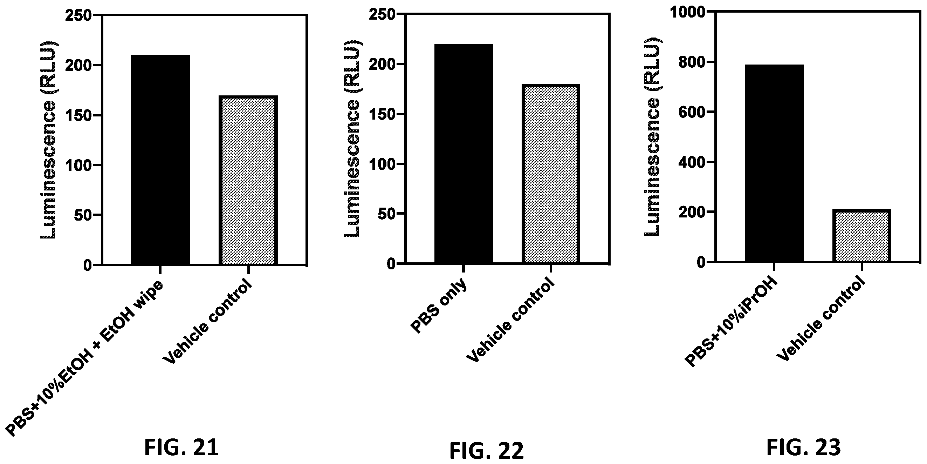

Nano Luciferase activity was detected in tissue samples at 4 days after topical administration for circular RNA in PBS with 10% ethanol and was observed in each case to be higher than the relevant vehicle only control (**FIG. 21**).

This Example demonstrates that mRNA is successfully delivered to tissues via topical administration to the skin when the skin is wiped with an ethanol wipe before administration and persists in tissue over prolonged periods of time and is able to express functional protein.

### Example 24: Topical administration of linear mRNA results in RNA delivery to tissue and subsequent protein expression

This Example demonstrates the ability to deliver mRNA to cells and tissues via topical administration *in vivo* and achieve subsequent protein expression.

In this example, mRNA was designed with an ORF encoding a Nano Luciferase (NLuc). In this example, modified linear mRNA was made in-house by *in vitro* transcription. In this example, RNA was fully substituted with Pseudo-Uridine and 5-Methyl-C, capped with CleanCap^{™} AG, included 5' and 3' human alpha-globin UTRs, and is polyadenylated.

RNA was diluted in (1) PBS only, or (2) PBS with 10% (v/v) isopropyl alcohol, such that total sample volume for each sample was 35 uL, and total RNA for each sample was 20 pmoles. As negative controls, vehicle only controls were prepared as described above but without RNA. All reagents are brought to room temperature prior to mixing and mixtures are prepared immediately prior to use.

At time = 0, a 35 µL dose of each sample was applied topically to the ear of a BALB/c mouse dropwise using a pipet tip. Samples were dried by exposing the ears briefly to a heat lamp and fan in a sterilized hood. Mice were place back in cages under normal conditions.

To determine RNA expression in tissue, tissue samples were analyzed for NLuc activity at varying timepoints after topical delivery. Ear punches were taken from the mouse at 4 days after delivery. Each tissue sample was crushed into fragments and was placed in 50 µL of ice cold NLuc Lysis Assay Buffer with 1× Protease Inhibitor Cocktail and placed on ice. Samples were then incubated on an orbital shaker for 5 minutes at 700 rpm, and then centrifuged at room temperature to remove tissue debris. The 50 uL supernatant was then transferred to a fresh tube without disturbing the tissue pellet. 50 µL of each sample was transferred to a 96 well plate and Nano-Glo Luciferase Assay System (Promega, #N1110) assay was performed according to manufacturer's instruction. In brief, 1 uL of furimazine substrate and 49 uL of PBS were added to each well and mixed. Plates were incubated for 10 min after substrate addition and mixing and then read in a luminometer instrument (Promega).

Nano Luciferase activity was detected in tissue samples at 4 days after topical administration for linear mRNA in PBS only, and linear mRNA in PBS with 10% isopropyl alcohol and was observed in each case to be higher than the relevant vehicle only control (**FIG. 22** and **FIG. 23**).

This Example demonstrates that mRNA is successfully delivered to tissues via topical administration to the skin and persists in tissue over prolonged periods of time and is able to express functional protein.

### Example 25: Topical administration of circular RNA results in RNA delivery to tissue when tissue is wiped with a povidone iodine prior to application

This Example describes the ability to deliver unmodified circular RNA to cells and tissues via topical administration *in vivo.*

In this example, circular RNA is designed with an IRES and ORF encoding Nanoluciferase (NLuc).

The circular RNA is generated *in vitro.* Unmodified linear RNA is transcribed in vitro from a DNA template including all the motifs listed above, as well as a T7 RNA polymerase promoter to drive transcription. Transcribed RNA is purified with an RNA cleanup kit (New England Biolabs, T2050), is treated with RNA 5'phosphohydrolase (RppH) (New England Biolabs, M0356) following the manufacturer's instructions, and is purified again with an RNA purification column. RppH treated linear RNA is circularized using a splint DNA (5'-TTTTTCGGCTATTCCCAATAGCCGTTTTG-3' (SEQ ID NO: 11)) and T4 RNA ligase 2 (New England Biolabs, M0239). Circular RNA is Urea-PAGE purified, eluted in a buffer (0.5 M Sodium Acetate, 0.1% SDS, 1 mM EDTA), is ethanol precipitated and is resuspended in RNA storage solution (ThermoFisher Scientific, cat# AM7000).

RNA is then diluted in PBS only such that total sample volume for each sample is 35 uL, and total RNA for each sample is 20 pmoles. As negative controls, vehicle only controls are prepared as described above but without RNA. All reagents are brought to room temperature prior to mixing and mixtures are prepared immediately prior to use.

At time = 0, the ear of the mouse is wiped with commercial povidone iodine (10%), which is a sterilizing agent. Excess povidone iodine is removed with a sterile cotton swab and a 35 µL dose of each sample is applied topically to the ear of a BALB/c mouse dropwise using a pipet tip. Samples are dried by exposing the ears briefly to a heat lamp and fan in a sterilized hood. Mice are placed back in cages under normal conditions.

To determine RNA persistence in tissue, tissue samples are analyzed for RNA at varying timepoints after delivery using RT-qPCR. At 1 day and 4 days post-administration, a 2 mm ear punch is taken from each animal and stored in RNAlater solution (ThermoFisher Scientific, cat# AM7020). Total RNA is isolated from ear punches by snap-cooling and homogenizing the tissue in liquid nitrogen with a glass mortar and pestle followed by trizol extraction (ThermoFisher Scientific cat # 15596026). The aqueous-phase is precipitated with isopropanol and the pellet is washed with 70% ETOH as per manufacturer's instructions. cDNA is synthesized from the total RNA using Superscript IV (Thermo Scientific, cat# 11766500). RT-PCR is performed on cDNA templates using iTaq^{™} Universal SYBR^{®}Green Supermix (Bio-rad, catalog #1725124) and primers specific to the NLuc ORF (F: CCGTATGAAGGTCTGAGCGG (SEQ ID NO: 12), R: CAGTGTGCCATAGTGCAGGA (SEQ ID NO: 13)). All samples are assayed in triplicate on the Bio-rad CFX384 Thermal Cycler. RNA levels are then be relativized to housekeeping gene (28s).

It is expected that circular RNA is detected in tissue samples at 1 day and 4 days after topical administration and greater signal than the vehicle only control is observed.

This Example describes that circular RNA is successfully delivered via topical administration to the tissue after wiping the skin with povidone iodine (10%) and persists in tissue over prolonged periods of time.

### Example 26: Topical administration of circular RNA results in RNA delivery to tissue when tissue is sprayed with a hydrogen peroxide spray prior to application

This Example describes the ability to deliver unmodified circular RNA to cells and tissues via topical administration *in vivo.*

In this example, circular RNA is designed with an IRES and ORF encoding Nanoluciferase (NLuc).

The circular RNA is generated *in vitro.* Unmodified linear RNA is transcribed *in vitro* from a DNA template including all the motifs listed above, as well as a T7 RNA polymerase promoter to drive transcription. Transcribed RNA is purified with an RNA cleanup kit (New England Biolabs, T2050), treated with RNA 5'phosphohydrolase (RppH) (New England Biolabs, M0356) following the manufacturer's instructions, and purified again with an RNA purification column. RppH treated linear RNA will be circularized using a splint DNA (5'-TTTTTCGGCTATTCCCAATAGCCGTTTTG-3' (SEQ ID NO: 11)) and T4 RNA ligase 2 (New England Biolabs, M0239). Circular RNA is Urea-PAGE purified, eluted in a buffer (0.5 M Sodium Acetate, 0.1% SDS, 1 mM EDTA), ethanol precipitated and resuspended in RNA storage solution (ThermoFisher Scientific, cat# AM7000).

RNA is then diluted in PBS only such that total sample volume for each sample is 35 uL, and total RNA for each sample is 20 pmoles. As negative controls, vehicle only controls are prepared as described above but without RNA. All reagents are brought to room temperature prior to mixing and mixtures are prepared immediately prior to use.

At time = 0, the ear of the mouse is sprayed with commercial hydrogen peroxide (3%), which is a sterilizing agent, dried with a sterile cotton swab and a 35 µL dose of each sample is applied topically to the ear of a BALB/c mouse dropwise using a pipet tip. Samples are dried by exposing the ears briefly to a heat lamp and fan in a sterilized hood. Mice are placed back in cages under normal conditions.

To determine RNA persistence in tissue, tissue samples are analyzed for RNA at varying timepoints after delivery using RT-qPCR. At 1 day and 4 days post-administration, a 2 mm ear punch is taken from each animal and stored in RNAlater solution (ThermoFisher Scientific, cat# AM7020). Total RNA is isolated from ear punches by snap-cooling and homogenizing the tissue in liquid nitrogen with a glass mortar and pestle followed by trizol extraction (ThermoFisher Scientific cat # 15596026). The aqueous-phase is precipitated with isopropanol and the pellet is washed with 70% ETOH as per manufacturer's instructions. cDNA is synthesized from the total RNA using Superscript IV (Thermo Scientific, cat# 11766500). RT-PCR is performed on cDNA templates using iTaq^{™} Universal SYBR^{®}Green Supermix (Bio-rad, catalog #1725124) and primers specific to the NLuc ORF (F: CCGTATGAAGGTCTGAGCGG (SEQ ID NO: 12), R: CAGTGTGCCATAGTGCAGGA (SEQ ID NO: 13)). All samples are assayed in triplicate on the Bio-rad CFX384 Thermal Cycler. RNA levels are then relativized to housekeeping gene (28s).

It is expected that circular RNA is detected in tissue samples at 1 day and 4 days after topical administration and greater signal than the vehicle only control is observed.

This Example describes that circular RNA is successfully delivered via topical administration to the tissue after spraying the skin with hydrogen peroxide (3%) and persists in tissue over prolonged periods of time.

### SEQUENCES

### Nano Luciferase DNA template

**SEQ ID NO: 1**

GTCTTCACACTCGAAGATTTCGTTGGGGACTGGCGACAGACAGCCGGCTACAACCT
GGACCAAGTCCTTGAACAGGGAGGTGTGTCCAGTTTGTTTCAGAATCTCGGGGTGTC
CGTAACTCCGATCCAAAGGATTGTCCTGAGCGGTGAAAATGGGCTGAAGATCGACA
TCCATGTCATCATCCCGTATGAAGGTCTGAGCGGCGACCAAATGGGCCAGATCGAA
AAAATTTTTAAGGTGGTGTACCCTGTGGATGATCATCACTTTAAGGTGATCCTGCAC
TATGGCACACTGGTAATCGACGGGGTTACGCCGAACATGATCGACTATTTCGGACG
GCCGTATGAAGGCATCGCCGTGTTCGACGGCAAAAAGATCACTGTAACAGGGACCC
TGTGGAACGGCAACAAAATTATCGACGAGCGCCTGATCAACCCCGACGGCTCCCTG
CTGTTCCGAGTAACCATCAACGGAGTGACCGGCTGGCGGCTGTGCGAACGCATTCT
GGCG

### EMCV IRES

**SEQ ID NO: 2**

ACGTTACTGGCCGAAGCCGCTTGGAATAAGGCCGGTGTGCGTTTGTCTATATGTTAT
TTTCCACCATATTGCCGTCTTTTGGCAATGTGAGGGCCCGGAAACCTGGCCCTGTCT
TCTTGACGAGCATTCCTAGGGGTCTTTCCCCTCTCGCCAAAGGAATGCAAGGTCTGT
TGAATGTCGTGAAGGAAGCAGTTCCTCTGGAAGCTTCTTGAAGACAAACAACGTCT
GTAGCGACCCTTTGCAGGCAGCGGAACCCCCCACCTGGCGACAGGTGCCTCTGCGG
CCAAAAGCCACGTGTATAAGATACACCTGCAAAGGCGGCACAACCCCAGTGCCACG
TTGTGAGTTGGATAGTTGTGGAAAGAGTCAAATGGCTCTCCTCAAGCGTATTCAACA
AGGGGCTGAAGGATGCCCAGAAGGTACCCCATTGTATGGGATCTGATCTGGGGCCT
CGGTGCACATGCTTTACATGTGTTTAGTCGAGGTTAAAAAACGTCTAGGCCCCCCGA
ACCACGGGGACGTGGTTTTCCTTTGAAAAACACGATGATAATA

### Gaussia Luciferase DNA template

**SEQ ID NO: 3**

ATGGGAGTCAAAGTTCTGTTTGCCCTGATCTGCATCGCTGTGGCCGAGGCCAAGCCC
ACCGAGAACAACGAAGACTTCAACATCGTGGCCGTGGCCAGCAACTTCGCGACCAC
GGATCTCGATGCTGACCGCGGGAAGTTGCCCGGCAAGAAGCTGCCGCTGGAGGTGC
TCAAAGAGATGGAAGCCAATGCCCGGAAAGCTGGCTGCACCAGGGGCTGTCTGATC
TGCCTGTCCCACATCAAGTGCACGCCCAAGATGAAGAAGTTCATCCCAGGACGCTG

CCACACCTACGAAGGCGACAAAGAGTCCGCACAGGGCGGCATAGGCGAGGCGATC
GTCGACATTCCTGAGATTCCTGGGTTCAAGGACTTGGAGCCCATGGAGCAGTTCATC
GCACAGGTCGATCTGTGTGTGGACTGCACAACTGGCTGCCTCAAAGGGCTTGCCAA
CGTGCAGTGTTCTGACCTGCTCAAGAAGTGGCTGCCGCAACGCTGTGCGACCTTTGC
CAGCAAGATCCAGGGCCAGGTGGACAAGATCAAGGGGGCCGGTGGTGACTAA

### EPO DNA template

**SEQ ID NO: 4**

ATGGGAGTGCACGAGTGTCCCGCGTGGTTGTGGTTGCTGCTGTCGCTCTTGAGCCTC
CCACTGGGACTGCCTGTGCTGGGGGCACCACCCAGATTGATCTGCGACTCACGGGT
ACTTGAGAGGTACCTTCTTGAAGCCAAAGAAGCCGAAAACATCACAACCGGATGCG
CCGAGCACTGCTCCCTCAATGAGAACATTACTGTACCGGATACAAAGGTCAATTTCT
ATGCATGGAAGAGAATGGAAGTAGGACAGCAGGCCGTCGAAGTGTGGCAGGGGCT
CGCGCTTTTGTCGGAGGCGGTGTTGCGGGGTCAGGCCCTCCTCGTCAACTCATCACA
GCCGTGGGAGCCCCTCCAACTTCATGTCGATAAAGCGGTGTCGGGGCTCCGCAGCT
TGACGACGTTGCTTCGGGCTCTGGGCGCACAAAAGGAGGCTATTTCGCCGCCTGAC
GCGGCCTCCGCGGCACCCCTCCGAACGATCACCGCGGACACGTTTAGGAAGCTTTTT
AGAGTGTACAGCAATTTCCTCCGCGGAAAGCTGAAATTGTATACTGGTGAAGCGTG
TAGGACAGGGGATCGC

### CVB3 IRES DNA template

**SEQ ID NO: 5**

TTAAAACAGCCTGTGGGTTGATCCCACCCACAGGCCCATTGGGCGCTAGCACTCTG
GTATCACGGTACCTTTGTGCGCCTGTTTTATACCCCCTCCCCCAACTGTAACTTAGA
AGTAACACACACCGATCAACAGTCAGCGTGGCACACCAGCCACGTTTTGATCAAGC
ACTTCTGTTACCCCGGACTGAGTATCAATAGACTGCTCACGCGGTTGAAGGAGAAA
GCGTTCGTTATCCGGCCAACTACTTCGAAAAACCTAGTAACACCGTGGAAGTTGCA
GAGTGTTTCGCTCAGCACTACCCCAGTGTAGATCAGGTCGATGAGTCACCGCATTCC
CCACGGGCGACCGTGGCGGTGGCTGCGTTGGCGGCCTGCCCATGGGGAAACCCATG
GGACGCTCTAATACAGACATGGTGCGAAGAGTCTATTGAGCTAGTTGGTAGTCCTC
CGGCCCCTGAATGCGGCTAATCCTAACTGCGGAGCACACACCCTCAAGCCAGAGGG
CAGTGTGTCGTAACGGGCAACTCTGCAGCGGAACCGACTACTTTGGGTGTCCGTGTT
TCATTTTATTCCTATACTGGCTGCTTATGGTGACAATTGAGAGATCGTTACCATATA
GCTATTGGATTGGCCATCCGGTGACTAATAGAGCTATTATATATCCCTTTGTTGGGT

TTATACCACTTAGCTTGAAAGAGGTTAAAACATTACAATTCATTGTTAAGTTGAATA
CAGCAAA

### Green Fluorescent Protein DNA template

**SEQ ID NO: 6**

AGCAAGGGCGAGGAGCTGTTCACCGGGGTGGTGCCCATCCTGGTCGAGCTGGACGG
CGACGTAAACGGCCACAAGTTCAGCGTGTCCGGCGAGGGCGAGGGCGATGCCACCT
ACGGCAAGCTGACCCTGAAGTTCATCTGCACCACCGGCAAGCTGCCCGTGCCCTGG
CCCACCCTCGTGACCACCCTGACCTACGGCGTGCAGTGCTTCAGCCGCTACCCCGAC
CACATGAAGCAGCACGACTTCTTCAAGTCCGCCATGCCCGAAGGCTACGTCCAGGA
GCGCACCATCTTCTTCAAGGACGACGGCAACTACAAGACCCGCGCCGAGGTGAAGT
TCGAGGGCGACACCCTGGTGAACCGCATCGAGCTGAAGGGCATCGACTTCAAGGAG
GACGGCAACATCCTGGGGCACAAGCTGGAGTACAACTACAACAGCCACAACGTCTA
TATCATGGCCGACAAGCAGAAGAACGGCATCAAGGTGAACTTCAAGATCCGCCACA
ACATCGAGGACGGCAGCGTGCAGCTCGCCGACCACTACCAGCAGAACACCCCCATC
GGCGACGGCCCCGTGCTGCTGCCCGACAACCACTACCTGAGCACCCAGTCCGCCCT
GAGCAAAGACCCCAACGAGAAGCGCGATCACATGGTCCTGCTGGAGTTCGTGACCG
CCGCCGGGATCACTCTCGGCATGGACGAGCTGTACAAG

## Claims

1. A cosmetic composition comprising a mixture of a circular polyribonucleotide and ethanol, wherein the ethanol constitutes:
(i) 0.3% v/v to 75% v/v of the mixture; or
(ii) 0.3% v/v to 70% v/v, 0.3% v/v to 60% v/v, 0.3% v/v to 50% v/v, 0.3% v/v to 40% v/v, 30% v/v to 20% v/v, 0.3% v/v to 15% v/v, 0.3% v/v to 10% v/v, 0.3% v/v to 5% v/v, 0.3% v/v to 1% v/v, or 0.3% v/v to 0.5% v/v of the mixture; or
(iii) 0.5% v/v to 75% v/v, 1% v/v to 75% v/v, 5% v/v to 75% v/v, 10% v/v to 75% v/v, 15% v/v to 75% v/v, 20% v/v to 75% v/v, 30% v/v to 75% v/v, 40% v/v to 75% v/v, 50% v/v to 75% v/v, 60% v/v to 75% v/v, or 70% v/v to 75% v/v of the mixture.

2. The cosmetic composition of claim 1, wherein the ethanol constitutes 5% v/v to 75% v/v of the mixture.

3. The cosmetic composition of claim 1 or 2, wherein:
(A) the circular polyribonucleotide encodes a cosmetic protein or peptide selected from the group consisting of collagen, keratin, elastin, and botulinum toxin, or any a fragment thereof; and/or
(B) the cosmetic composition is a liquid, gel, lotion, paste, cream, foam, serum, ointment, or stick; and/or
(C) the circular polyribonucleotide lacks a cap or lacks a poly-A tail, or comprises a modified ribonucleotide; and/or
(D) the cosmetic composition:
(i) has a pH of about 7; and/or
(ii) has a viscosity that is about the same as water; and/or
(iii) is substantially free of hydrophobic or lipophilic groups; and/or
(iv) is substantially free of hydrocarbons; and/or
(v) is substantially free of cationic liposomes; and/or
(vi) is substantially free of fatty acids, lipids, liposomes, cholesterol, or any combination thereof; and/or
(vii) is substantially free form glycerin, propylene glycol, or a combination thereof; and/or
(E) the cosmetic composition is an anti-wrinkle composition.

4. A non-therapeutic method of delivering a polyribonucleotide to a subject comprising applying a cosmetic composition comprising a mixture of a cosmetic polyribonucleotide and ethanol to an integumentary member of the subject, wherein the ethanol constitutes 0.3% v/v to 75% v/v of the mixture, and wherein the cosmetic polyribonucleotide is a circular polyribonucleotide.

5. A non-therapeutic method of delivering a cosmetic polyribonucleotide to a subject comprising:
(A)
a) applying a sterilizing agent to an integumentary member of the subject;
b) applying a cosmetic composition free of any carrier comprising the cosmetic polyribonucleotide and diluent to the integumentary member, wherein the cosmetic polyribonucleotide is a circular polyribonucleotide;
optionally wherein the sterilizing agent is an alcohol, UV light, laser light, or heat; or
(B)
a) applying an alcohol to an integumentary member of the subject;
b) applying a cosmetic composition free of any carrier comprising the cosmetic polyribonucleotide and cosmetically acceptable diluent to the integumentary member, wherein the cosmetic polyribonucleotide is a circular polyribonucleotide;
optionally wherein the alcohol is selected from the group consisting of: methanol, ethanol, isopropanol, butanol, pentanol, cetyl alcohol, ethylene glycol, propylene glycol, denatured alcohol, benzyl alcohol, specially denatured alcohol, glycol, stearyl alcohol, cetearyl alcohol, menthol, polyethylene glycols (PEG)-400, isopropyl alcohol, dimethylaminoethanol, retinol, d-alpha-tocopherol, SD alcohol, SD alcohol 40, c12-16, lauryl alcohol, myristyl alcohol, butylene glycol, and propanediol.

6. A non-therapeutic method of delivering a polyribonucleotide to:
(A) an epithelial cell comprising applying a cosmetic composition free of any carrier comprising a diluent and a cosmetic polyribonucleotide that is not modified to the epithelial cell, wherein the cosmetic polyribonucleotide is a circular polyribonucleotide; or
(B) a subject comprising applying a cosmetic composition comprising a mixture of a cosmetic polyribonucleotide and an alcohol to an integumentary member of the subject, wherein the alcohol constitutes 0.3% v/v to 75% v/v of the mixture, and wherein the cosmetic polyribonucleotide is a circular polyribonucleotide; or
(C) a subject comprising applying a cosmetic composition comprising a mixture of a cosmetic polyribonucleotide and a cell-penetrating agent to an integumentary member of the subject, wherein the cell-penetrating agent constitutes 0.3% v/v to 75% v/v of the mixture, and wherein the cosmetic polyribonucleotide is a circular polyribonucleotide;
preferably wherein the cosmetic composition delivers the cosmetic polyribonucleotide to a dermal or epidermal tissue of the subject, and more preferably, without iontophoresis.

7. A kit comprising an application tool and the cosmetic composition of any one of claims 1-3, wherein the application tool is configured to apply the cosmetic composition to an integumentary member of a subject.

8. A kit comprising a first application tool, a second application tool, a sterilizing agent, and a cosmetic composition free of any carrier comprising the cosmetic polyribonucleotide and diluent, wherein the first application tool is configured to apply a sterilizing agent to an integumentary member of a subject and the second application tool is configured to apply the composition to the integumentary member of the subject, and wherein the cosmetic polyribonucleotide is a circular polyribonucleotide.

9. The kit of claim 8, wherein the sterilizing agent is an alcohol, UV light, laser light, or heat;
optionally wherein the alcohol is selected from the group consisting of: methanol, ethanol, isopropanol, butanol, pentanol, cetyl alcohol, ethylene glycol, propylene glycol, denatured alcohol, benzyl alcohol, specially denatured alcohol, glycol, stearyl alcohol, cetearyl alcohol, menthol, polyethylene glycols (PEG)-400, isopropyl alcohol, dimethylaminoethanol, retinol, d-alpha-tocopherol, SD alcohol, SD alcohol 40, c12-16, lauryl alcohol, myristyl alcohol, butylene glycol, and propanediol.

10. The kit of any one of claims 7-9, wherein the first application tool is a wipe, and optionally wherein the wipe comprises the sterilizing agent.

11. A kit comprising an application tool and a mixture comprising a circular polyribonucleotide and a cell-penetrating agent or alcohol, wherein the application tool is configured to apply the mixture to a integumentary member of a subject.

12. The kit of any one of claims 6-11, wherein:
(A) the application tool or second application tool comprises a pipette; and/or
(B) the application tool or second application tool comprises a substrate, and wherein the substrate is embedded with the mixture, and optionally, wherein the substrate is made of natural or artificial fibers; and/or
(C) the application tool or second application tool comprises: (i) a patch; (ii) a sprayer; or (iii) a peel pad, and optionally, the application tool or second application tool is configured to release the mixture in a controlled manner; and/or
(D) the integumentary member comprises skin, nails, eyelashes or hair.

13. A cosmetic composition comprising a mixture of a circular polyribonucleotide and an alcohol, wherein the alcohol constitutes:
(i) 0.3% v/v to 75% v/v of the mixture; or
(ii)0.3% v/v to 70% v/v, 0.3% v/v to 60% v/v, 0.3% v/v to 50% v/v, 0.3% v/v to 40% v/v, 30% v/v to 20% v/v, 0.3% v/v to 15% v/v, 0.3% v/v to 10% v/v, 0.3% v/v to 5% v/v, 0.3% v/v to 1% v/v, or 0.3% v/v to 0.5% v/v of the mixture; or
(iii) 0.5% v/v to 75% v/v, 1% v/v to 75% v/v, 5% v/v to 75% v/v, 10% v/v to 75% v/v, 15% v/v to 75% v/v, 20% v/v to 75% v/v, 30% v/v to 75% v/v, 40% v/v to 75% v/v, 50% v/v to 75% v/v, 60% v/v to 75% v/v, or 70% v/v to 75% v/v of the mixture;
optionally, wherein the alcohol is selected from the group consisting of: methanol, ethanol, isopropanol, butanol, pentanol, cetyl alcohol, ethylene glycol, propylene glycol, denatured alcohol, benzyl alcohol, specially denatured alcohol, glycol, stearyl alcohol, cetearyl alcohol, menthol, polyethylene glycols (PEG)-400, isopropyl alcohol, dimethylaminoethanol, retinol, d-alpha-tocopherol, SD alcohol, SD alcohol 40, c12-16, lauryl alcohol, myristyl alcohol, butylene glycol, and propanediol.

14. A cosmetic composition comprising a mixture of a circular polyribonucleotide and a cell-penetrating agent, wherein the cell-penetrating agent constitutes:
(i) 0.3% v/v to 75% v/v of the mixture; or
(ii)0.3% v/v to 70% v/v, 0.3% v/v to 60% v/v, 0.3% v/v to 50% v/v, 0.3% v/v to 40% v/v, 30% v/v to 20% v/v, 0.3% v/v to 15% v/v, 0.3% v/v to 10% v/v, 0.3% v/v to 5% v/v, 0.3% v/v to 1% v/v, or 0.3% v/v to 0.5% v/v of the mixture; or
(iii) 0.5% v/v to 75% v/v, 1% v/v to 75% v/v, 5% v/v to 75% v/v, 10% v/v to 75% v/v, 15% v/v to 75% v/v, 20% v/v to 75% v/v, 30% v/v to 75% v/v, 40% v/v to 75% v/v, 50% v/v to 75% v/v, 60% v/v to 75% v/v, or 70% v/v to 75% v/v of the mixture;
preferably wherein the cell-penetrating agent is an alcohol; and
more preferably wherein the alcohol is selected from the group consisting of:
methanol, ethanol, isopropanol, butanol, pentanol, cetyl alcohol, ethylene glycol, propylene glycol, denatured alcohol, benzyl alcohol, specially denatured alcohol, glycol, stearyl alcohol, cetearyl alcohol, menthol, polyethylene glycols (PEG)-400, isopropyl alcohol, dimethylaminoethanol, retinol, d-alpha-tocopherol, SD alcohol, SD alcohol 40, c12-16, lauryl alcohol, myristyl alcohol, butylene glycol, and propanediol.

15. The cosmetic composition of claim 14, wherein the cell penetrating agent is: (i) soluble in polar solvents; or (ii) insoluble in polar solvents.

## Patentansprüche

1. Kosmetische Zusammensetzung, die ein Gemisch aus einem zirkulären Polyribonukleotid und Ethanol umfasst, wobei das Ethanol Folgendes ausmacht:
(i) 0,3 Vol.-% bis 75 Vol.-% des Gemischs; oder
(ii) 0,3 Vol.-% bis 70 Vol.-%, 0,3 Vol.-% bis 60 Vol.-%, 0,3 Vol.-% bis 50 Vol.-%, 0,3 Vol.-% bis 40 Vol.-%, 30 Vol.-% bis 20 Vol.-%, 0,3 Vol.-% bis 15 Vol.-%, 0,3 Vol.-% bis 10 Vol.-%, 0,3 Vol.-% bis 5 Vol.-%, 0,3 Vol.-% bis 1 Vol.-% oder 0,3 Vol.-% bis 0,5 Vol.-% des Gemischs; oder
(iii) 0,5 Vol.-% bis 75 Vol.-%, 1 Vol.-% bis 75 Vol.-%, 5 Vol.-% bis 75 Vol.-%, 10 Vol.-% bis 75 Vol.-%, 15 Vol.-% bis 75 Vol.-%, 20 Vol.-% bis 75 Vol.-%, 30 Vol.-% bis 75 Vol.-%, 40 Vol.-% bis 75 Vol.-%, 50 Vol.-% bis 75 Vol.-%, 60 Vol.-% bis 75 Vol.-% oder 70 Vol.-% bis 75 Vol.-% des Gemischs.

2. Kosmetische Zusammensetzung nach Anspruch 1, wobei das Ethanol 5 Vol.-% bis 75 Vol.-% des Gemischs ausmacht.

3. Kosmetische Zusammensetzung nach Anspruch 1 oder 2, wobei:
(A) das zirkuläre Polyribonukleotid für ein kosmetisches Protein oder Peptid kodiert, das aus der Gruppe ausgewählt ist, die aus Kollagen, Keratin, Elastin und Botulinumtoxin oder einem beliebigen Fragment davon besteht; und/oder
(B) die kosmetische Zusammensetzung eine Flüssigkeit, ein Gel, eine Lotion, eine Paste, eine Creme, ein Schaum, ein Serum, eine Salbe oder ein Stift ist; und/oder
(C) dem zirkulären Polyribonukleotid eine Kappe fehlt oder ein Poly-A-Schwanz fehlt, oder es ein modifiziertes Ribonukleotid umfasst; und/oder
(D) die kosmetische Zusammensetzung:
(i) einen pH-Wert von etwa 7 aufweist; und/oder
(ii) eine Viskosität aufweist, die etwa die gleiche ist wie Wasser; und/oder
(iii) im Wesentlichen frei von hydrophoben oder lipophilen Gruppen ist; und/oder
(iv) im Wesentlichen frei von Kohlenwasserstoffen ist; und/oder
(v) im Wesentlichen frei von kationischen Liposomen ist; und/oder
(vi) im Wesentlichen frei von Fettsäuren, Lipiden, Liposomen, Cholesterin oder einer Kombination davon ist; und/oder
(vii) im Wesentlichen frei von Glycerin, Propylenglycol oder einer Kombination davon ist; und/oder
(E) die kosmetische Zusammensetzung eine Anti-Falten-Zusammensetzung ist.

4. Nicht-therapeutisches Verfahren zur Abgabe eines Polyribonukleotids an ein Subjekt, das das Auftragen einer kosmetischen Zusammensetzung, die ein Gemisch aus einem kosmetischen Polyribonukleotid und Ethanol umfasst, auf ein integumentäres Element des Subjekts umfasst, wobei das Ethanol 0,3 Vol.-% bis 75 Vol.-% des Gemischs ausmacht, und wobei das kosmetische Polyribonukleotid ein zirkuläres Polyribonukleotid ist.

5. Nicht-therapeutisches Verfahren zur Verabreichung eines kosmetischen Polyribonukleotids an ein Subjekt, wobei das Verfahren Folgendes umfasst:
(A)
a) Auftragen eines Sterilisationsmittels auf ein integumentäres Glied des Subjekts;
b) Auftragen einer kosmetischen Zusammensetzung, die frei von jeglichem Träger ist und das kosmetische Polyribonukleotid und ein Verdünnungsmittel enthält, auf das Integumentarteil, wobei das kosmetische Polyribonukleotid ein zirkuläres Polyribonukleotid ist;
wobei das Sterilisationsmittel optional ein Alkohol, UV-Licht, Laserlicht oder Wärme ist; oder
(B)
a) Auftragen eines Alkohols auf ein integumentäres Glied des Subjekts;
b) Auftragen einer kosmetischen Zusammensetzung, die frei von jeglichem Träger ist und das kosmetische Polyribonukleotid und ein kosmetisch akzeptables Verdünnungsmittel enthält, auf das Integumentarteil, wobei das kosmetische Polyribonukleotid ein zirkuläres Polyribonukleotid ist;
wobei der Alkohol optional aus der Gruppe ausgewählt ist, die aus Folgendem besteht: Methanol, Ethanol, Isopropanol, Butanol, Pentanol, Cetylalkohol, Ethylenglykol, Propylenglykol, vergälltem Alkohol, Benzylalkohol, speziell vergälltem Alkohol, Glykol, Stearylalkohol, Cetearylalkohol, Menthol, Polyethylenglykolen (PEG)-400, Isopropylalkohol, Dimethylaminoethanol, Retinal, d-alpha-Tocopherol, SD-Alkohol, SD-Alkohol 40, cl2-16, Laurylalkohol, Myristylalkohol, Butylenglykol und Propandiol.

6. Nicht-therapeutisches Verfahren zur Verabreichung eines Polyribonukleotids an
(A) eine Epithelzelle, umfassend das Auftragen einer kosmetischen Zusammensetzung, die frei von jeglichem Träger ist, umfassend ein Verdünnungsmittel und ein kosmetisches Polyribonukleotid, das nicht modifiziert ist, auf die Epithelzelle, wobei das kosmetische Polyribonukleotid ein zirkuläres Polyribonukleotid ist; oder
(B) ein Subjekt, umfassend das Auftragen einer kosmetischen Zusammensetzung, die ein Gemisch aus einem kosmetischen Polyribonukleotid und einem Alkohol umfasst, auf ein integumentäres Glied des Subjekts, wobei der Alkohol 0,3 Vol.-% bis 75 Vol.-% des Gemischs ausmacht, und wobei das kosmetische Polyribonukleotid ein zirkuläres Polyribonukleotid ist; oder
(C) ein Subjekt, umfassend das Auftragen einer kosmetischen Zusammensetzung, die ein Gemisch aus einem kosmetischen Polyribonukleotid und einem zelldurchdringenden Mittel umfasst, auf ein integumentäres Glied des Subjekts, wobei das zelldurchdringende Mittel 0,3 Vol.-% bis 75 Vol.-% des Gemischs ausmacht, und wobei das kosmetische Polyribonukleotid ein zirkuläres Polyribonukleotid ist;
vorzugsweise, wobei die kosmetische Zusammensetzung das kosmetische Polyribonukleotid an ein dermales oder epidermales Gewebe des Subjekts abgibt, und noch bevorzugter, ohne Iontophorese.

7. Kit, das ein Applikationswerkzeug und die kosmetische Zusammensetzung nach einem der Ansprüche 1 bis 3 umfasst, wobei das Applikationswerkzeug dafür konfiguriert ist, die kosmetische Zusammensetzung auf ein integumentäres Element eines Subjekts aufzutragen.

8. Kit, das ein erstes Applikationswerkzeug, ein zweites Applikationswerkzeug, ein Sterilisationsmittel und eine kosmetische Zusammensetzung umfasst, die frei von jeglichem Träger ist und das kosmetische Polyribonukleotid und Verdünnungsmittel umfasst, wobei das erste Applikationswerkzeug dafür konfiguriert ist, ein Sterilisationsmittel auf ein Integumentalelement eines Subjekts aufzubringen und das zweite Applikationswerkzeug dafür konfiguriert ist, die Zusammensetzung auf das Integumentalelement des Subjekts aufzubringen, und wobei das kosmetische Polyribonukleotid ein zirkuläres Polyribonukleotid ist.

9. Kit nach Anspruch 8, wobei das Sterilisationsmittel ein Alkohol, UV-Licht, Laserlicht oder Wärme ist;
wobei der Alkohol optional aus der Gruppe ausgewählt ist, die aus Folgendem besteht: Methanol, Ethanol, Isopropanol, Butanol, Pentanol, Cetylalkohol, Ethylenglykol, Propylenglykol, vergälltem Alkohol, Benzylalkohol, speziell vergälltem Alkohol, Glykol, Stearylalkohol, Cetearylalkohol, Menthol, Polyethylenglykolen (PEG)-400, Isopropylalkohol, Dimethylaminoethanol, Retinal, d-alpha-Tocopherol, SD-Alkohol, SD-Alkohol 40, cl2-16, Laurylalkohol, Myristylalkohol, Butylenglykol und Propandiol.

10. Kit nach einem der Ansprüche 7 bis 9, wobei das erste Applikationswerkzeug ein Wischtuch ist, und optional wobei das Wischtuch das Sterilisationsmittel enthält.

11. Kit, das ein Applikationswerkzeug und ein Gemisch umfasst, das ein zirkuläres Polyribonukleotid und ein zelldurchdringendes Mittel oder Alkohol umfasst, wobei das Applikationswerkzeug dafür konfiguriert ist, das Gemisch auf ein integumentäres Element eines Subjekts aufzutragen.

12. Kit nach einem der Ansprüche 6 bis 11, wobei:
(A) das Applikationswerkzeug oder das zweite Applikationswerkzeug eine Pipette umfasst; und/oder
(B) das Applikationswerkzeug oder das zweite Applikationswerkzeug ein Substrat umfasst, und wobei das Substrat mit dem Gemisch eingebettet ist, und optional, wobei das Substrat aus natürlichen oder künstlichen Fasern hergestellt ist; und/oder
(C) das Applikationswerkzeug oder das zweite Applikationswerkzeug Folgendes umfasst: (i) ein Pflaster; (ii) einen Sprüher; oder (iii) ein Abziehkissen, und optional wobei das Applikationswerkzeug oder das zweite Applikationswerkzeug dafür konfiguriert ist, das Gemisch in kontrollierter Weise freizusetzen; und/oder
(D) das integumentäre Element Haut, Nägel, Wimpern oder Haare umfasst.

13. Kosmetische Zusammensetzung, die ein Gemisch aus einem zirkulären Polyribonukleotid und einem Alkohol umfasst, wobei der Alkohol Folgendes ausmacht:
(i) 0,3 Vol.-% bis 75 Vol.-% des Gemischs; oder
(ii) 0,3 Vol.-% bis 70 Vol.-%, 0,3 Vol.-% bis 60 Vol.-%, 0,3 Vol.-% bis 50 Vol.-%, 0,3 Vol.-% bis 40 Vol.-%, 30 Vol.-% bis 20 Vol.-%, 0,3 Vol.-% bis 15 Vol.-%, 0,3 Vol.-% bis 10 Vol.-%, 0,3 Vol.-% bis 5 Vol.-%, 0,3 Vol.-% bis 1 Vol.-% oder 0,3 Vol.-% bis 0,5 Vol.-% des Gemischs; oder
(iii) 0,5 Vol.-% bis 75 Vol.-%, 1 Vol.-% bis 75 Vol.-%, 5 Vol.-% bis 75 Vol.-%, 10 Vol.-% bis 75 Vol.-%, 15 Vol.-% bis 75 Vol.-%, 20 Vol.-% bis 75 Vol.-%, 30 Vol.-% bis 75 Vol.-%, 40 Vol.-% bis 75 Vol.-%, 50 Vol.-% bis 75 Vol.-%, 60 Vol.-% bis 75 Vol.-% oder 70 Vol.-% bis 75 Vol.-% des Gemischs;
wobei der Alkohol optional aus der Gruppe ausgewählt ist, die aus Folgendem besteht: Methanol, Ethanol, Isopropanol, Butanol, Pentanol, Cetylalkohol, Ethylenglykol, Propylenglykol, vergälltem Alkohol, Benzylalkohol, speziell vergälltem Alkohol, Glykol, Stearylalkohol, Cetearylalkohol, Menthol, Polyethylenglykolen (PEG)-400, Isopropylalkohol, Dimethylaminoethanol, Retinal, d-alpha-Tocopherol, SD-Alkohol, SD-Alkohol 40, cl2-16, Laurylalkohol, Myristylalkohol, Butylenglykol und Propandiol.

14. Kosmetische Zusammensetzung, die ein Gemisch aus einem zirkulären Polyribonukleotid und einem zelldurchdringenden Mittel umfasst, wobei das zelldurchdringende Mittel Folgendes ausmacht:
(i) 0,3 Vol.-% bis 75 Vol.-% des Gemischs; oder
(ii) 0,3 Vol.-% bis 70 Vol.-%, 0,3 Vol.-% bis 60 Vol.-%, 0,3 Vol.-% bis 50 Vol.-%, 0,3 Vol.-% bis 40 Vol.-%, 30 Vol.-% bis 20 Vol.-%, 0,3 Vol.-% bis 15 Vol.-%, 0,3 Vol.-% bis 10 Vol.-%, 0,3 Vol.-% bis 5 Vol.-%, 0,3 Vol.-% bis 1 Vol.-% oder 0,3 Vol.-% bis 0,5 Vol.-% des Gemischs; oder
(iii) 0,5 Vol.-% bis 75 Vol.-%, 1 Vol.-% bis 75 Vol.-%, 5 Vol.-% bis 75 Vol.-%, 10 Vol.-% bis 75 Vol.-%, 15 Vol.-% bis 75 Vol.-%, 20 Vol.-% bis 75 Vol.-%, 30 Vol.-% bis 75 Vol.-%, 40 Vol.-% bis 75 Vol.-%, 50 Vol.-% bis 75 Vol.-%, 60 Vol.-% bis 75 Vol.-% oder 70 Vol.-% bis 75 Vol.-% des Gemischs;
vorzugsweise wobei das zelldurchdringende Mittel ein Alkohol ist; und bevorzugter, wobei der Alkohol ausgewählt ist aus der Gruppe bestehend aus:
Methanol, Ethanol, Isopropanol, Butanol, Pentanol, Cetylalkohol, Ethylenglykol, Propylenglykol, vergälltem Alkohol, Benzylalkohol, speziell vergälltem Alkohol, Glykol, Stearylalkohol, Cetearylalkohol, Menthol, Polyethylenglykolen (PEG)-400, Isopropylalkohol, Dimethylaminoethanol, Retinal, d-alpha-Tocopherol, SD-Alkohol, SD-Alkohol 40, cl2-16, Laurylalkohol, Myristylalkohol, Butylenglykol und Propandiol.

15. Kosmetische Zusammensetzung nach Anspruch 14, wobei das zelldurchdringende Mittel: (i) in polaren Lösungsmitteln löslich ist; oder (ii) in polaren Lösungsmitteln unlöslich ist.

## Revendications

1. Composition cosmétique comprenant un mélange d'un polyribonucléotide circulaire et d'éthanol, dans laquelle l'éthanol constitue :
(i) 0,3 % v/v à 75 % v/v du mélange ; ou
(ii) 0,3 % v/v à 70 % v/v, 0,3 % v/v à 60 % v/v, 0,3 % v/v à 50 % v/v, 0,3 % v/v à 40 % v/ v, 30 % v/v à 20 % v/v, 0,3 % v/v à 15 % v/v, 0,3 % v/v à 10 % v/v, 0,3 % v/v à 5 % v/v, 0,3 % v/v à 1 % v/v, ou 0,3 % v/v à 0,5 % v/v du mélange ; ou
(iii) 0,5 % v/v à 75 % v/v, 1 % v/v à 75 % v/v, 5 % v/v à 75 % v/v, 10 % v/v à 75 % v/ v, 15 % v/v à 75 % v/v, 20 % v/v à 75 % v/v, 30 % v/v à 75 % v/v, 40 % v/v à 75 % v/v, 50 % v/v à 75 % v/v, 60 % v/v à 75 % v/v ou 70 % v/v à 75 % v/v du mélange.

2. Composition cosmétique selon la revendication 1, dans laquelle l'éthanol constitue 5% v/v à 75% v/v du mélange.

3. Composition cosmétique selon la revendication 1 ou 2, dans laquelle :
(A) le polyribonucléotide circulaire code pour une protéine ou un peptide cosmétique choisi dans le groupe constitué par le collagène, la kératine, l'élastine et la toxine botulique, ou l'un quelconque de leurs fragments ; et/ou
(B) la composition cosmétique est un liquide, un gel, une lotion, une pâte, une crème, une mousse, un sérum, une pommade ou un stick ; et/ou
(C) le polyribonucléotide circulaire est dépourvu de coiffe ou de queue poly-A, ou comprend un ribonucléotide modifié ; et/ou
(D) la composition cosmétique :
(i) a un pH d'environ 7 ; et/ou
(ii) a une viscosité qui est à peu près la même que celle de l'eau ; et/ou
(iii) est sensiblement dépourvue de groupes hydrophobes ou lipophiles ; et/ou
(iv) est sensiblement dépourvue d'hydrocarbures ; et/ou
(v) est sensiblement dépourvue de liposomes cationiques ; et/ou
(vi) est sensiblement dépourvue d'acides gras, de lipides, de liposomes, de cholestérol ou de toute combinaison de ceux-ci ; et/ou
(vii) est de la glycérine sous forme sensiblement libre, du propylèneglycol ou une combinaison de ceux-ci ; et/ou
(E) la composition cosmétique est une composition antirides.

4. Procédé non thérapeutique d'administration d'un polyribonucléotide à un sujet comprenant l'application d'une composition cosmétique comprenant un mélange d'un polynbonucléotide cosmétique et d'éthanol sur un membre tégumentaire du sujet, dans lequel l'éthanol constitue 0,3 % v/v à 75 % v/v du mélange, et dans lequel le polyribonucléotide cosmétique est un polyribonucléotide circulaire.

5. Procédé non thérapeutique d'administration d'un polyribonucléotide cosmétique à un sujet comprenant :
(A)
a) l'application d'un agent stérilisant sur un membre tégumentaire du sujet ;
b) l'application d'une composition cosmétique dépourvue de tout support comprenant le polyribonucléotide cosmétique et diluant sur le membre tégumentaire, dans lequel le polyribonucléotide cosmétique est un polyribonucléotide circulaire ;
éventuellement dans lequel l'agent stérilisant est un alcool, une lumière UV, une lumière laser ou de la chaleur ; ou
(B)
a) l'application d'un alcool sur un membre tégumentaire du sujet ;
b) l'application d'une composition cosmétique dépourvue de tout support comprenant le polyribonucléotide cosmétique et diluant cosmétiquement acceptable sur le membre tégumentaire, dans lequel le polyribonucléotide cosmétique est un polyribonucléotide circulaire ; éventuellement dans lequel l'alcool est choisi dans le groupe constitué de : méthanol, d'éthanol, d'isopropanol, de butanol, de pentanol, d'alcool cétylique, d'éthylène glycol, de propylène glycol, d'alcool dénaturé, d'alcool benzylique, d'alcool spécialement dénaturé, de glycol, d'alcool stéarylique, d'alcool cétéarylique, de menthol, de polyéthylène glycols (PEG)-400, d'alcool isopropylique, de diméthylaminoéthanol, de rétinol, de d-alpha-tocophérol, d'alcool SD, d'alcool SD 40, de c12-16, d'alcool laurylique, d'alcool myristylique, de butylène glycol et de propanediol.

6. Procédé non thérapeutique de délivrance d'un polyribonucléotide à :
(A) une cellule épithéliale comprenant l'application d'une composition cosmétique dépourvue de tout support comprenant un diluant et un polyribonucléotide cosmétique qui n'est pas modifié à la cellule épithéliale, dans lequel le polyribonucléotide cosmétique est un polyribonucléotide circulaire ; ou
(B) un sujet comprenant l'application d'une composition cosmétique comprenant un mélange d'un polyribonucléotide cosmétique et d'un alcool sur un membre tégumentaire du sujet, dans lequel l'alcool constitue 0,3 % v/v à 75 % v/v du mélange, et dans lequel le polyribonucléotide cosmétique est un polyribonucléotide circulaire ; ou
(C) un sujet comprenant l'application d'une composition cosmétique comprenant un mélange d'un polyribonucléotide cosmétique et d'un agent de pénétration cellulaire sur un membre tégumentaire du sujet, dans lequel l'agent de pénétration cellulaire constitue 0,3 % v/v à 75 % v/v du mélange, et dans lequel le polyribonucléotide cosmétique est un polyribonucléotide circulaire ;
préférablement dans lequel la composition cosmétique délivre le polyribonucléotide cosmétique sur un tissu dermique ou épidermique du sujet, et plus préférablement, sans ionophorèse.

7. Kit comprenant un outil d'application et la composition cosmétique selon l'une quelconque des revendications 1 à 3, dans lequel l'outil d'application est configuré pour appliquer la composition cosmétique sur un membre tégumentaire d'un sujet.

8. Kit comprenant un premier outil d'application, un deuxième outil d'application, un agent stérilisant et une composition cosmétique dépourvue de tout support comprenant le polyribonucléotide cosmétique et diluant, dans lequel le premier outil d'application est configuré pour appliquer un agent stérilisant sur un membre tégumentaire d'un sujet et le deuxième outil d'application est configuré pour appliquer la composition sur le membre tégumentaire du sujet, et dans lequel le polyribonucléotide cosmétique est un polyribonucléotide circulaire.

9. Kit selon la revendication 8, dans lequel l'agent stérilisant est un alcool, une lumière UV, une lumière laser ou la chaleur ;
éventuellement dans lequel l'alcool est choisi dans le groupe constitué de : méthanol, d'éthanol, d'isopropanol, de butanol, de pentanol, d'alcool cétylique, d'éthylène glycol, de propylène glycol, d'alcool dénaturé, d'alcool benzylique, d'alcool spécialement dénaturé, de glycol, d'alcool stéarylique, d'alcool cétéarylique, de menthol, de polyéthylène glycols (PEG)-400, d'alcool isopropylique, de diméthylaminoéthanol, de rétinol, de d-alpha-tocophérol, d'alcool SD, d'alcool SD 40, de c12-16, d'alcool laurylique, d'alcool myristylique, de butylène glycol et de propanediol.

10. Kit selon l'une quelconque des revendications 7 à 9, dans lequel le premier outil d'application est une lingette, et éventuellement dans lequel la lingette comprend l'agent stérilisant.

11. Kit comprenant un outil d'application et un mélange comprenant un polyribonucléotide circulaire et un agent de pénétration cellulaire ou un alcool, dans lequel l'outil d'application est configuré pour appliquer le mélange sur un membre tégumentaire d'un sujet.

12. Kit selon l'une quelconque des revendications 6 à 11, dans lequel :
(A) l'outil d'application ou le deuxième outil d'application comprend une pipette ; et/ou
(B) l'outil d'application ou le deuxième outil d'application comprend un substrat, et dans lequel le substrat est noyé avec le mélange, et éventuellement, dans lequel le substrat est fait de fibres naturelles ou artificielles ; et/ou
(C) l'outil d'application ou le deuxième outil d'application comprend : (i) un patch ; (ii) un pulvérisateur ; ou (iii) un tampon pelable, et éventuellement, l'outil d'application ou le deuxième outil d'application est configuré pour libérer le mélange d'une manière contrôlée ; et/ou
(D) le membre tégumentaire comprend la peau, les ongles, les cils ou les cheveux.

13. Composition cosmétique comprenant un mélange d'un polynbonucléotide circulaire et d'un alcool, dans laquelle l'alcool constitue :
(i) 0,3 % v/v à 75 % v/v du mélange ; ou
(ii) 0,3 % v/v à 70 % v/v, 0,3 % v/v à 60 % v/v, 0,3 % v/v à 50 % v/v, 0,3 % v/v à 40 % v/ v, 30 % v/v à 20 % v/v, 0,3 % v/v à 15 % v/v, 0,3 % v/v à 10 % v/v, 0,3 % v/v à 5 % v/v, 0,3 % v/v à 1 % v/v, ou 0,3 % v/v à 0,5 % v/v du mélange ; ou
(iii) 0,5 % v/v à 75 % v/v, 1 % v/v à 75 % v/v, 5 % v/v à 75 % v/v, 10 % v/v à 75 % v/ v, 15 % v/v à 75 % v/v, 20 % v/v à 75 % v/v, 30 % v/v à 75 % v/v, 40 % v/v à 75 % v/v, 50 % v/v à 75 % v/v, 60 % v/v à 75 % v/v ou 70 % v/v à 75 % v/v du mélange ;
éventuellement, dans laquelle l'alcool est choisi dans le groupe constitué de : méthanol, d'éthanol, d'isopropanol, de butanol, de pentanol, d'alcool cétylique, d'éthylène glycol, de propylène glycol, d'alcool dénaturé, d'alcool benzylique, d'alcool spécialement dénaturé, de glycol, d'alcool stéarylique, d'alcool cétéarylique, de menthol, de polyéthylène glycols (PEG)-400, d'alcool isopropylique, de diméthylaminoéthanol, de rétinol, de d-alpha-tocophérol, d'alcool SD, d'alcool SD 40, de c12-16, d'alcool laurylique, d'alcool myristylique, de butylène glycol et de propanediol.

14. Composition cosmétique comprenant un mélange d'un polynbonucléotide circulaire et d'un agent de pénétration cellulaire, dans laquelle l'agent de pénétration cellulaire constitue :
(i) 0,3 % v/v à 75 % v/v du mélange ; ou
(ii) 0,3 % v/v à 70 % v/v, 0,3 % v/v à 60 % v/v, 0,3 % v/v à 50 % v/v, 0,3 % v/v à 40 % v/ v, 30 % v/v à 20 % v/v, 0,3 % v/v à 15 % v/v, 0,3 % v/v à 10 % v/v, 0,3 % v/v à 5 % v/v, 0,3 % v/v à 1 % v/v, ou 0,3 % v/v à 0,5 % v/v du mélange ; ou
(iii) 0,5 % v/v à 75 % v/v, 1 % v/v à 75 % v/v, 5 % v/v à 75 % v/v, 10 % v/v à 75 % v/ v, 15 % v/v à 75 % v/v, 20 % v/v à 75 % v/v, 30 % v/v à 75 % v/v, 40 % v/v à 75 % v/v, 50 % v/v à 75 % v/v, 60 % v/v à 75 % v/v ou 70 % v/v à 75 % v/v du mélange ;
préférablement dans lequel l'agent pénétrant dans les cellules est un alcool ; et
plus préférablement dans lequel l'alcool est choisi dans le groupe constitué de : méthanol, d'éthanol, d'isopropanol, de butanol, de pentanol, d'alcool cétylique, d'éthylène glycol, de propylène glycol, d'alcool dénaturé, d'alcool benzylique, d'alcool spécialement dénaturé, de glycol, d'alcool stéarylique, d'alcool cétéarylique, de menthol, de polyéthylène glycols (PEG)-400, d'alcool isopropylique, de diméthylaminoéthanol, de rétinol, de d-alpha-tocophérol, d'alcool SD, d'alcool SD 40, de c12-16, d'alcool laurylique, d'alcool myristylique, de butylène glycol et de propanediol.

15. Composition cosmétique selon la revendication 14, dans laquelle l'agent de pénétration cellulaire est : (i) soluble dans les solvants polaires ; ou (ii) insoluble dans les solvants polaires.
